# EUROPEAN PATENT APPLICATION

(11) **EP 4 043 097 A1**
(43) Date of publication of application: **17.08.2022**
(21) Application number: 20869235.0
(22) Date of filing: 25.09.2020
(51) Int. Cl.: B01F 7/16, B01F 7/24, C12M 1/00, C12M 1/02, C12M 3/00, C12N 5/078, C12N 1/00

(54) **STIRRING BODY AND STIRRING DEVICE PROVIDED WITH SAME**

(30) Priority: 27.09.2019 JP 2019176476; 27.09.2019 JP 2019176477; 27.09.2019 JP 2019176478
(71) Applicant: Katsume, Shoji, Kanagawa 222-0022 (JP)
(72) Inventor: Katsume, Shoji, Kanagawa 222-0022 (JP)
(74) Representative: dompatent von Kreisler Selting Werner - Partnerschaft von Patent- und Rechtsanwälten mbB
(86) International application number: PCT/JP2020/036488
(87) International publication number: WO 2021/060550

(57) **Abstract**

Provided is a stirring body that can be easily manufactured with low shear and that can generate a plurality of flows including a circulating flow and a turbulent flow to stir a fluid medium in a complex and unsteady manner, and adjust the flow of a stirring flow according to the purpose. A stirring body includes a flat plate in which a plurality of spiral blade portions extending from a central portion is formed by a spiral cut that does not contact an outer peripheral portion; and an operating member that supports the flat plate, wherein either one of the operating member or the outer peripheral portion of the flat plate is movable in a direction of separating and approaching in relation to each other, and the blade portion rises while being separated from an adjacent blade portion in conjunction with the movement in the direction of separating in relation to each other so that the flat plate is extended to form a three-dimensional shape.

## Description

### TECHINCAL FIELD

The present invention relates to a stirring blade and a stirring device including the stirring blade.

### BACKGROUND ART

Conventionally, as a culture method of producing platelets in the medical field, there is static culture in a culture dish. However, in the static culture-based production method, it has been difficult to produce a large amount of inactive platelets required for blood transfusion. Therefore, attention is being paid to the production of platelets in vitro using human iPS cells. The key to this platelet production is unsteady stirring that generates turbulent flow, and there is a strong demand for the development of a three-dimensional suspension stirring culture device that produces a large amount of high-quality inactive platelets, aiming to realize a new production system for donor-independent platelet preparations and blood preparations.

Regarding the method of producing platelet preparations and blood preparations, as described in Patent Literature 1, there is a stirring culture device in which a stirring blade having the shape of a disk, an ellipse, and a rectangular shape reciprocates vertically to stir a culture solution in an unsteady manner. In this production method, megakaryocyte cells, which are the largest cells existing in the bone marrow in the living body, are three-dimensionally suspension-cultured in a culture solution while generating turbulent flow with vertical movement of a stirring blade and stirring the culture solution in an unsteady manner. In this way, the production efficiency and physiological activity of platelets released by inducing differentiation from megakaryocyte cells derived from human iPS cells can be increased, the degradation of platelets can be suppressed to a low level, and the number of abnormal platelets can be reduced.

### PRIOR ART

Patent Document 1: WO2017/077964

### DISCLOSURE OF THE INVENTION

### Problem to be Solved by the Invention

As described above, the reciprocating stirring culture device used for conventional platelet production performs unsteady stirring with a stirring blade that generates a simple flow. However, there is no reciprocating stirring culture device that generates a plurality of flows and performs complex unsteady stirring by the stirring blades dynamically behaving while forming a deformed form by simply causing the stirring blade to reciprocate in a vertical direction.

In addition to moving the stirring blade of the stirring device vertically in the normal direction to realize stirring, in order for the stirring blade to perform stirring in a plurality of operating directions accompanied by forward and reverse rotational movement, it is necessary, for example, to provide a plurality of driving devices for driving the stirring blade in different operating directions or a complex mechanism for realizing a plurality of operating directions with one driving device. Thus, there is a problem in terms of durability and cost of the driving devices.

Furthermore, in the conventional reciprocating stirring culture device, it is possible to adjust the flow rate of the stirring flow by controlling the reciprocating speed of the stirring blade. However, in order to change the flow pattern of the stirring flow, it is necessary to replace the stirring blades with different shapes each time. There is no stirring blade that dynamically behaves by elastic deformation to appropriately form a plurality of deformed forms to adjust the flow of stirring flow by simply controlling a reciprocating distance of one stirring blade.

Therefore, in order to solve the problem existing in the prior art, an object of the present invention is to provide a stirring body that can generate complex circulating flow and turbulent flow accompanied by vortex flow with a plurality of operating directions to stir a fluid medium in an extremely complex and unsteady manner by a stirring blade dynamically behaving by elastic deformation or plastic deformation to form a plurality of different deformed forms by simply causing the stirring blade to reciprocate and can adjust a flow of stirring flow easily according to the purpose by controlling a reciprocating distance of the stirring blade to cause the stirring blade to form a plurality of different deformed forms, and that can be manufactured easily with low shear and to provide a stirring device having the stirring body.

### Means for Solving the Problem

A stirring body of the present invention includes a flat plate in which a plurality of spiral blade portions extending from a central portion is formed by a spiral cut that does not contact an outer peripheral portion; and an operating member that supports the flat plate, wherein either one of the operating member or an outer peripheral portion of the flat plate being movable in a direction of separating and approaching in relation to the other, and the blade portion rising while being separated from an adjacent blade portion in conjunction with the movement in a direction of separating in relation to each other, thereby the flat plate being extended to form a three-dimensional shape.

In the stirring body of the present invention, the operating member supports the flat plate in a state of being fixed.

Moreover, the operating member supports the flat plate in a state of being rotatable.

Moreover, the operating member or the flat plate is rotatable in forward and reverse directions during the movement.

Moreover, one or more neck portions are formed in the three-dimensional shape in which the flat plate is extended.

Moreover, the blade portion is formed in a plurality of wavy shapes of which the front and back surfaces are exposed on condition that seen from one direction in the three-dimensional shape in which the flat plate is extended.

Moreover, the cut is formed by connecting circular arcs, straight lines, or combinations thereof in series.

A stirring device of the present invention includes the above-mentioned stirring body and a driving device that moves the operating member or the flat version.

In the stirring device of the present invention, the driving device rotates the operating member or the flat plate in addition to the movement.

### Advantages of the Invention

According to the present invention, it is possible to provide a stirring body that can generate a plurality of flows including circulating flow, turbulent flow, and the like to stir a fluid medium in a complex and unsteady manner and adjust the flow of stirring flow according to the purpose and can be manufactured easily with low shear and to provide a stirring device including the stirring body.

### BRIEF DESCRIPTION OF THE DRAWING

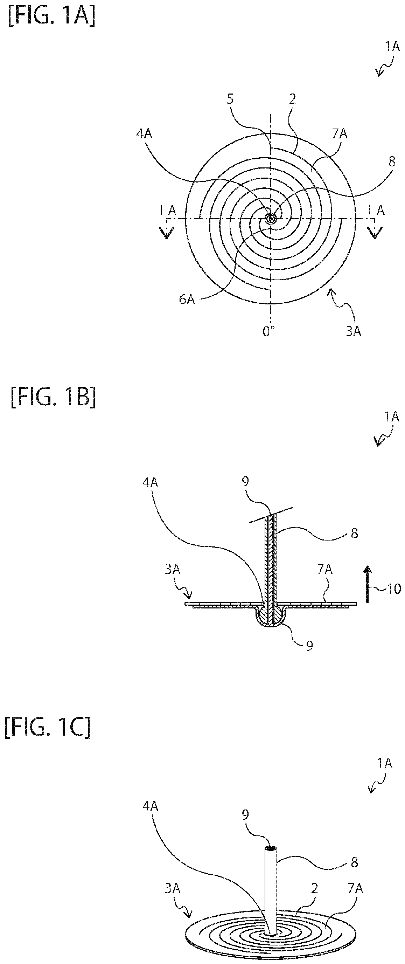

FIG. 1 shows a first embodiment of a stirring body according to the present invention, in which FIG. 1(A) is a plan view showing the stirring body having a two-dimensional shape, FIG. 1(B) is a cross-sectional view showing the stirring body along the line 1A-1A of FIG. 1(A), and FIG. 1(C) is a perspective view showing the stirring body having the two-dimensional shape of FIG. 1(A).

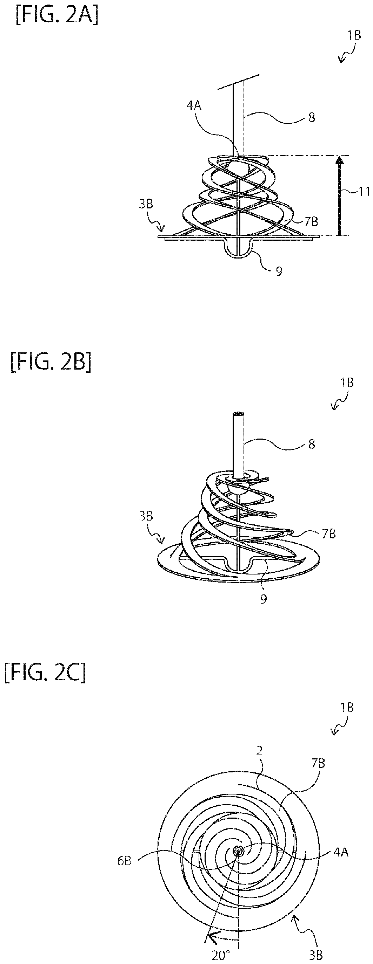

FIG. 2(A) is a side view of the stirring body extended from the two-dimensional shape shown in FIG. 1 to a three-dimensional shape, FIG. 2(B) is a perspective view of the stirring body having the three-dimensional shape of FIG. 2(A), and FIG. 2(C) is a top view of the stirring body having the three-dimensional shape of FIG. 2(A).

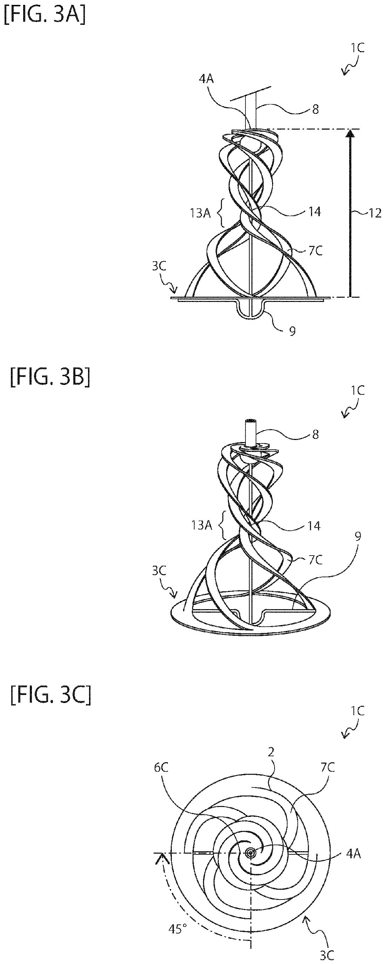

FIG. 3(A) is a side view showing a case in which the stirring body shown in FIG. 2 is further extended, FIG. 3(B) is a perspective view of the stirring body having the three-dimensional shape of FIG. 3(A), and FIG. 3(C) is a top view of the stirring body having the three-dimensional shape of FIG. 3(A).

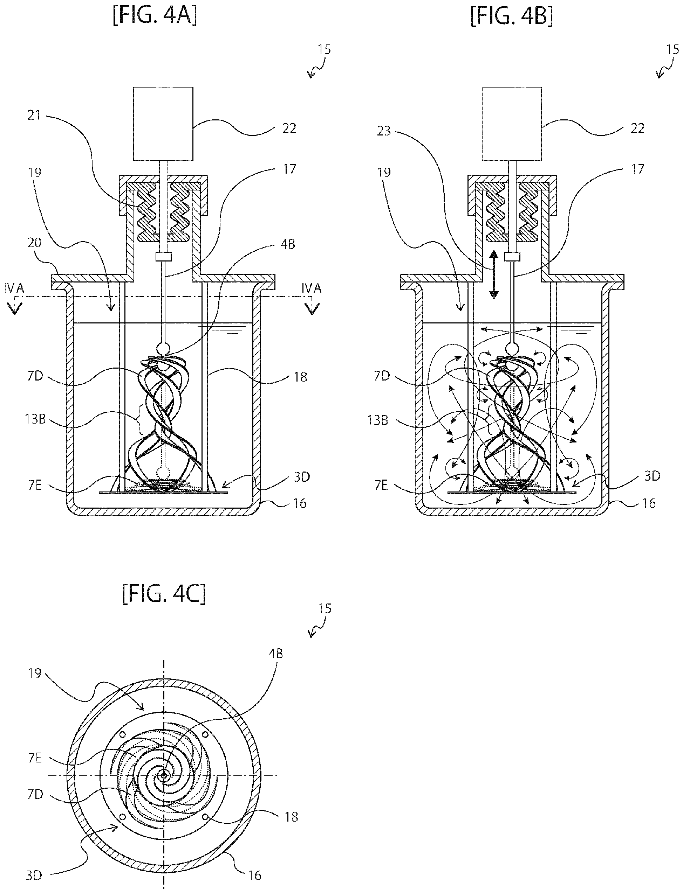

FIG. 4 shows a first embodiment of a stirring device including the stirring body according to the present invention, in which FIG. 4(A) is a side view of the stirring device including the stirring body, FIG. 4(B) is a schematic view showing the movement of a fluid by the stirring device of FIG. 4(A), and FIG. 4(C) is a cross-sectional view showing the stirring device along the line IVA-IVA of FIG. 4(A).

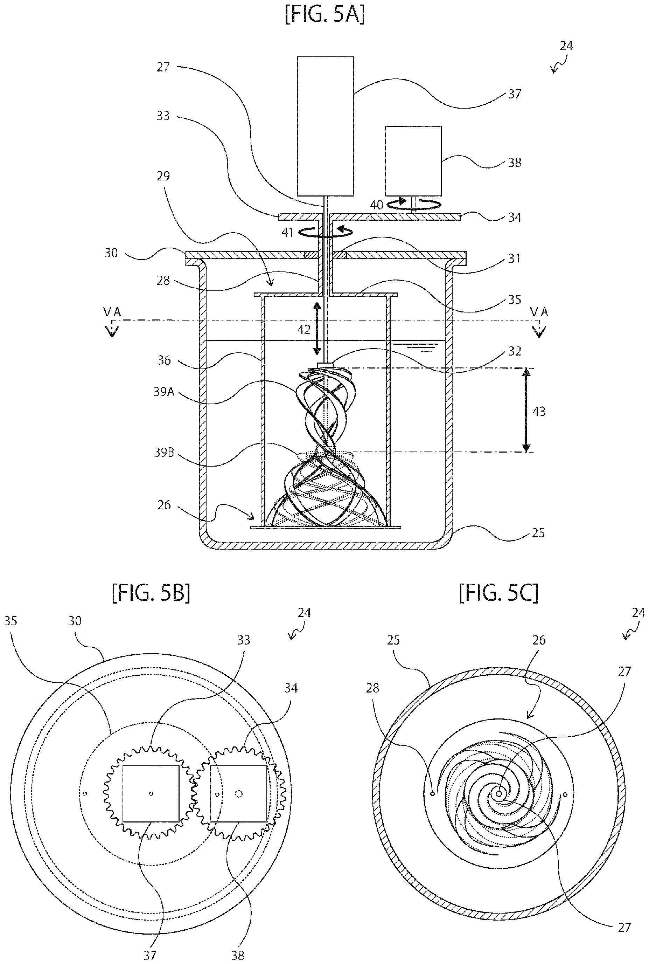

FIG. 5 shows a second embodiment of the stirring device including the stirring body according to the present invention, in which FIG. 5(A) is a side view showing the stirring device including the stirring body according to the present invention, FIG. 5(B) is a top view showing the stirring device of FIG. 5(A), and FIG. 5(C) is a cross-sectional view showing the stirring device along the broken-line VA-VA of FIG. 5(A).

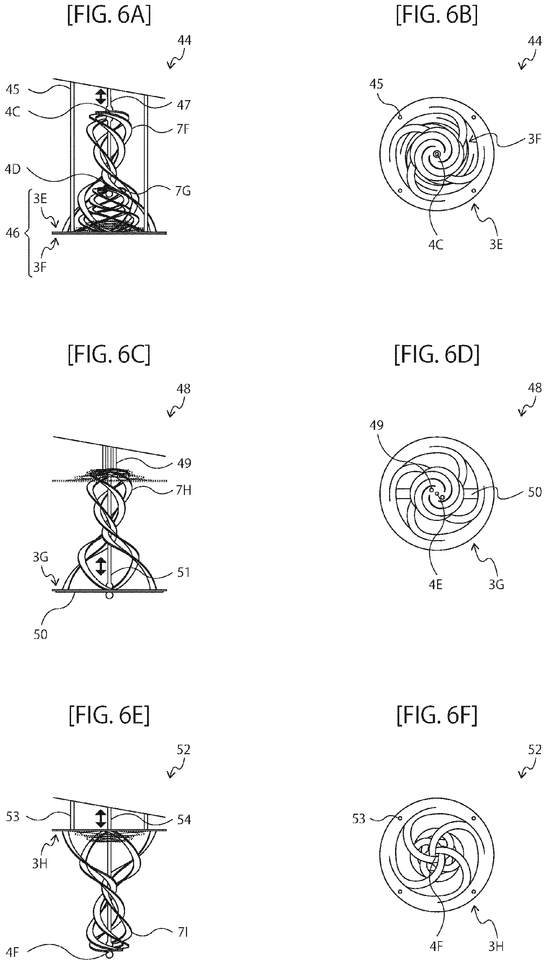

FIG. 6 illustrates embodiments of various configurations of a flat plate of the stirring body according to the present invention, in which FIG. 6(A) is a side view illustrating a stirring body including another set of different flat plates inside one set of flat plates, FIG. 6(B) is a top view of the stirring body shown in FIG. 6(A), FIG. 6(C) is a side view illustrating a stirring body in which a support member is connected to the central portion of a flat plate, FIG. 6(D) is a top view of the stirring body shown in FIG. 6(C), FIG. 6(E) is a side view illustrating a stirring body in which a support member is connected to the outer peripheral portion of a flat plate, and FIG. 6(F) is a top view of the stirring body shown in FIG. 6(E).

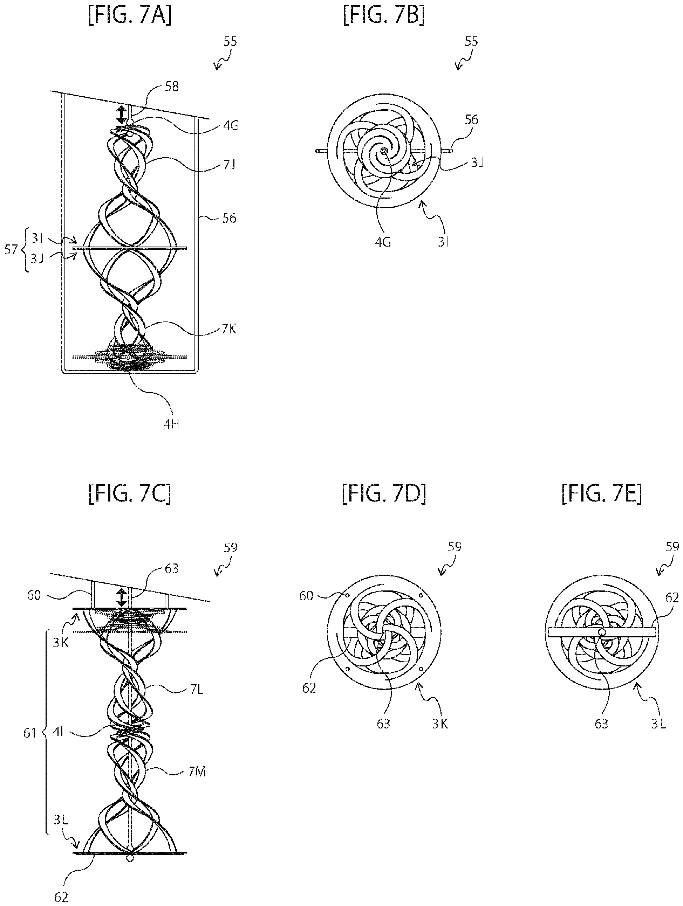

FIG. 7(A) is a side view showing a stirring body in which two flat plates are bonded to each other at the outer peripheral portions thereof, FIG. 7(B) is a top view of the stirring body shown in FIG. 7(A), FIG. 7(C) is a side view showing a stirring body in which two flat plates are bonded to each other at the central portions thereof, FIG. 7(D) is a top view of the stirring body shown in FIG. 7(C), and FIG. 7(E) is a bottom view of the stirring body shown in FIG. 7(C).

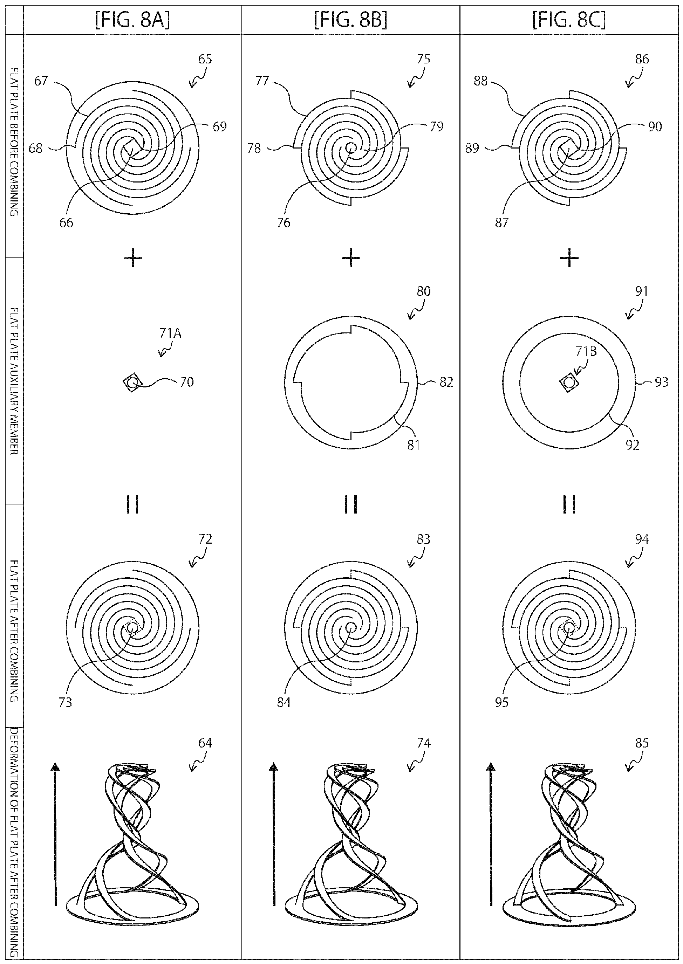

FIG. 8 illustrates embodiments of various forming methods of a flat plate of the stirring body according to the present invention, in which FIG. 8(A) is a top view and a perspective view illustrating a flat plate in which a flat plate auxiliary member is bonded to the central portion side, FIG. 8(B) is a top view and a perspective view illustrating a flat plate in which a flat plate auxiliary member is bonded to the outer peripheral portion side, and FIG. 8(C) is a top view and a perspective view illustrating a flat plate in which a flat plate auxiliary member is bonded to the central portion side and the outer peripheral portion side.

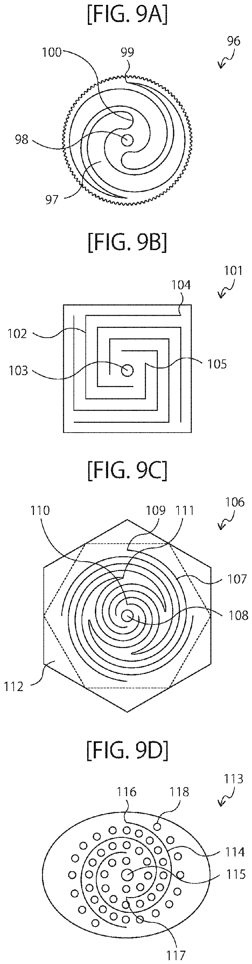

FIG. 9 illustrates embodiment of various cuts of a flat plate of the stirring body according to the present invention, in which FIG. 9(A) is a plan view illustrating a circular flat plate having a wavy shape having a spiral cutout FIG. 9(B) is a plan view illustrating a flat plate having a cut along the outer shape of a square, FIG. 9(C) is a plan view illustrating a regular hexagonal flat plate having a spiral cut and a flap extending in the left-right direction, and FIG. 9(D) is a plan view illustrating an elliptical flat plate having a plurality of through-holes.

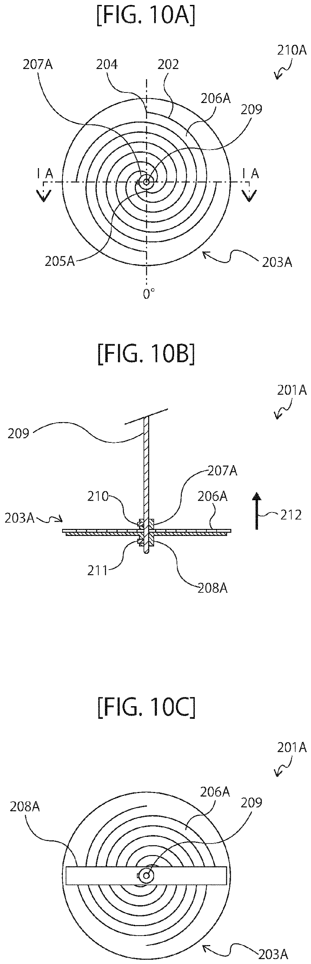

FIG. 10 shows a second embodiment of a stirring body according to the present invention, in which FIG. 10(A) is a top view showing the stirring body having a two-dimensional shape, FIG. 10(B) is a cross-sectional view showing the stirring body along the broken-line IA-IA of FIG. 10(A), and FIG. 10(C) is a bottom view showing the stirring body having the two-dimensional shape of FIG. 10(A).

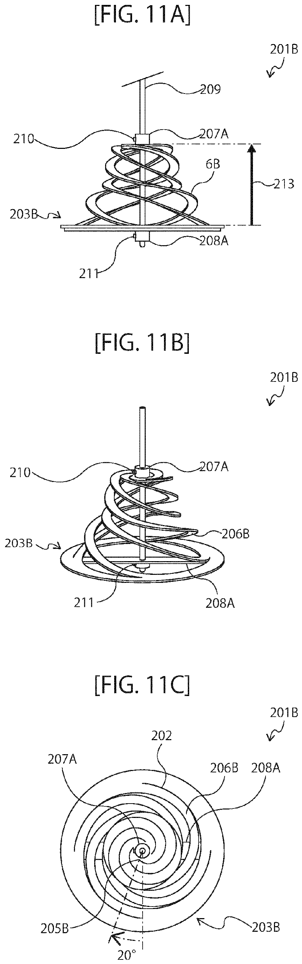

FIG. 11(A) is a side view of the stirring body extended from the two-dimensional shape shown in FIG. 10 to a three-dimensional shape, FIG. 11(B) is a perspective view of the stirring body having the three-dimensional shape of FIG. 11(A), and FIG. 11(C) is a top view of the stirring body having the three-dimensional shape of FIG. 11(A).

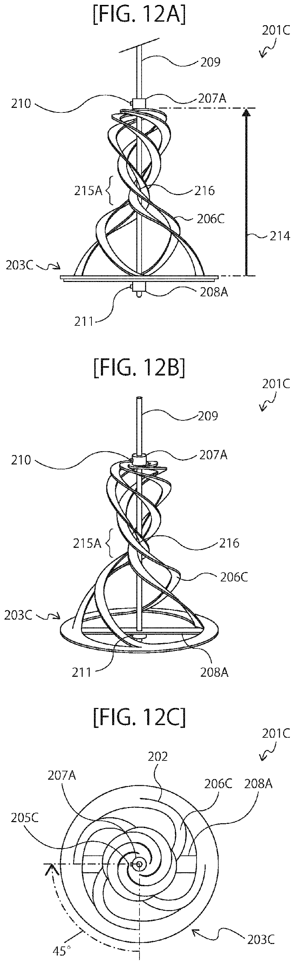

FIG. 12(A) is a side view showing a case in which the stirring body shown in FIG. 11 is further extended, FIG. 12(B) is a perspective view of the stirring body having the three-dimensional shape of FIG. 12(A), and FIG. 12(C) is a top view of the stirring body having the three-dimensional shape of FIG. 12(A).

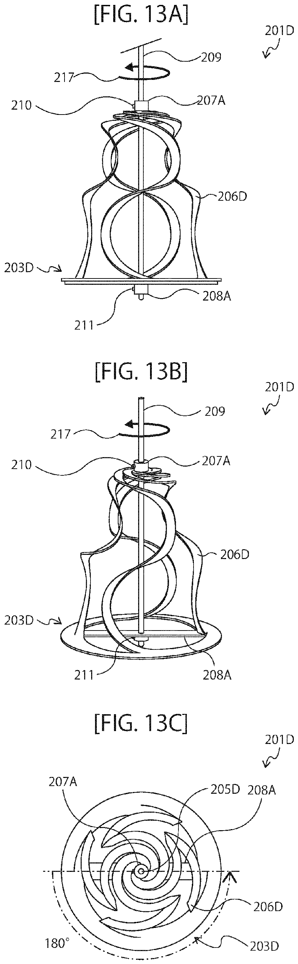

FIG. 13(A) is a side view when the central portion is twisted from the stirring body shown in FIG. 12, FIG. 13(B) is a perspective view of the stirring body having the three-dimensional shape of FIG. 13(A), and FIG. 13(C) is a top view of the stirring body having the three-dimensional shape of FIG. 13(A).

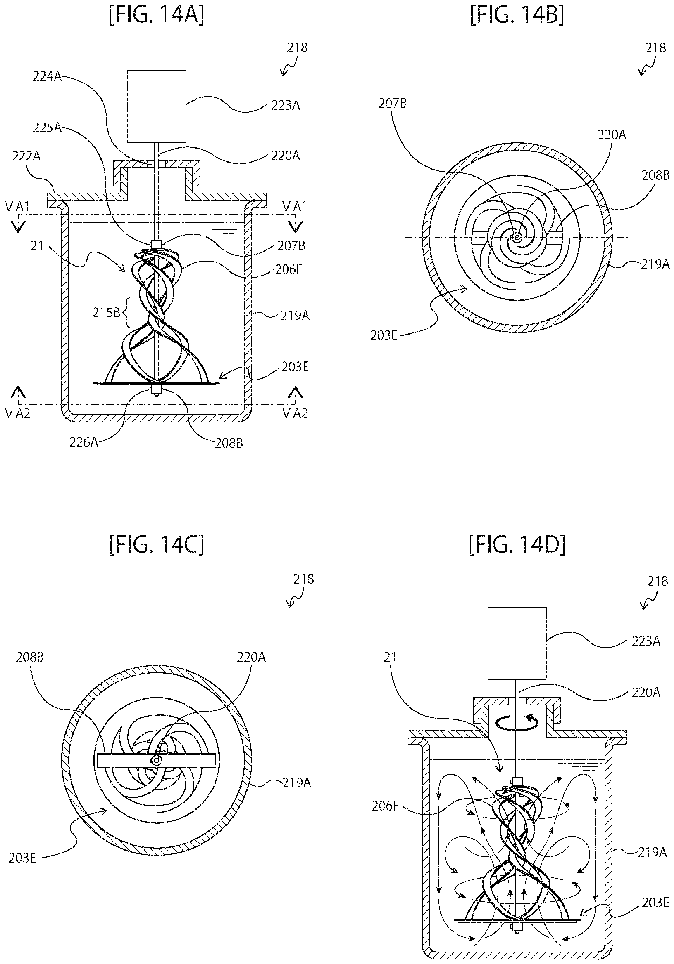

FIG. 14 shows a third embodiment of a stirring device including the stirring body according to the present invention, in which FIG. 14(A) is a side view of the stirring device including the stirring body, FIG. 14(B) is a cross-sectional view showing the stirring device along the broken-line VA1-VA1 of FIG. 14(A), FIG. 14(C) is a cross-sectional view showing the stirring device along the broken-line VA2-VA2 of FIG. 14(A), and FIG. 14(D) is a schematic view showing the movement of a fluid by the stirring device of FIG. 14(A).

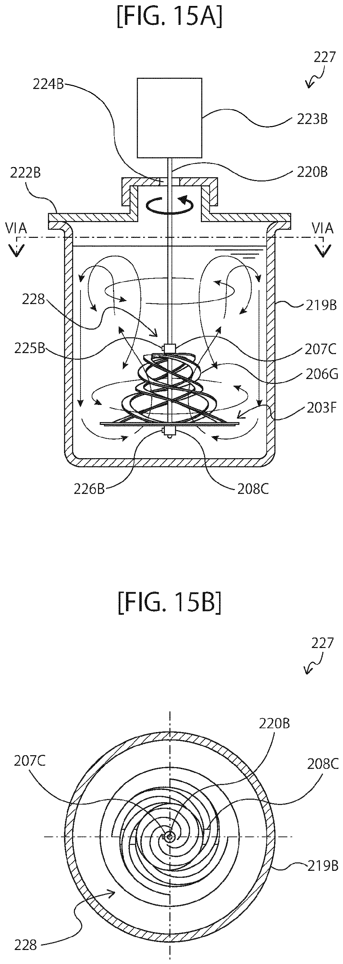

FIG. 15 shows a fourth embodiment of the stirring device including the stirring body according to the present invention, in which FIG. 15(A) is a schematic view showing the movement of a fluid by the stirring device including the stirring body, and FIG. 15(B) is a cross-sectional view showing the stirring device along the broken-line VIA-VIA of FIG. 15(A).

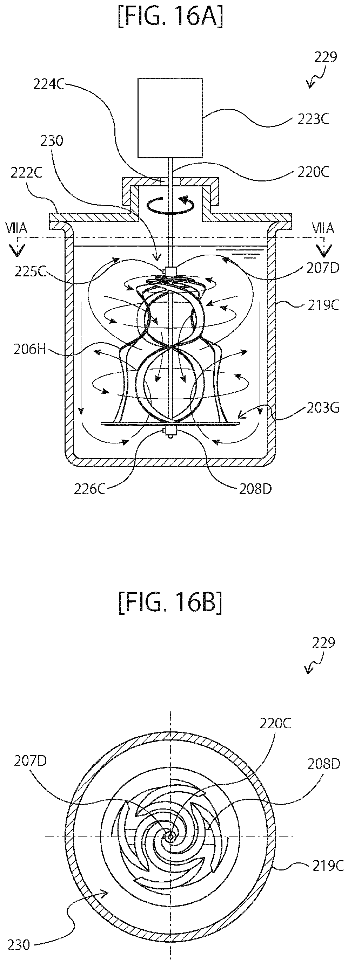

FIG. 16(A) is a schematic view showing the movement of a fluid by the stirring device including the stirring body, and FIG. 16(B) is a cross-sectional view showing the stirring body along the broken-line VIIA-VIIA of FIG. 16(A).

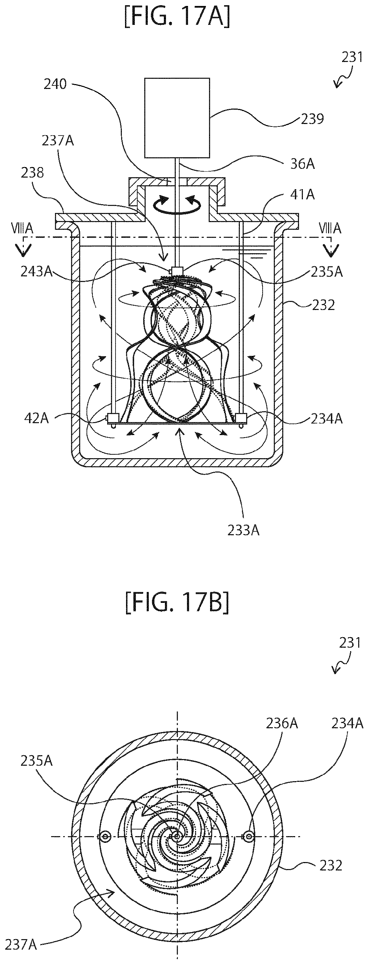

FIG. 17 illustrates a fifth embodiment of a stirring device including the stirring body according to the present invention, in which FIG. 17(A) is a schematic view illustrating the movement of a fluid by the stirring device including the stirring body, and FIG. 17(B) is a cross-sectional view illustrating the stirring device along the broken-line VIIIA-VIIIA of FIG. 17(A).

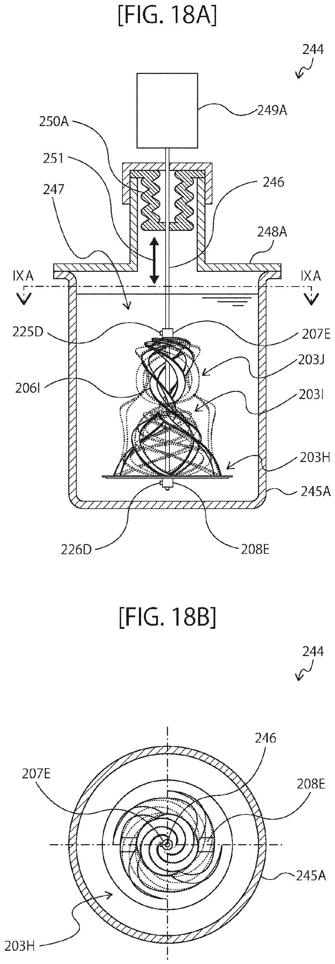

FIG. 18 illustrates a sixth embodiment of a stirring device including the stirring body according to the present invention, in which FIG. 18(A) is a side view illustrating a stirring device including the stirring body, and FIG. 18(B) is a cross-sectional view illustrating the stirring device along the broken-line IXA-IXA of FIG. 18(A).

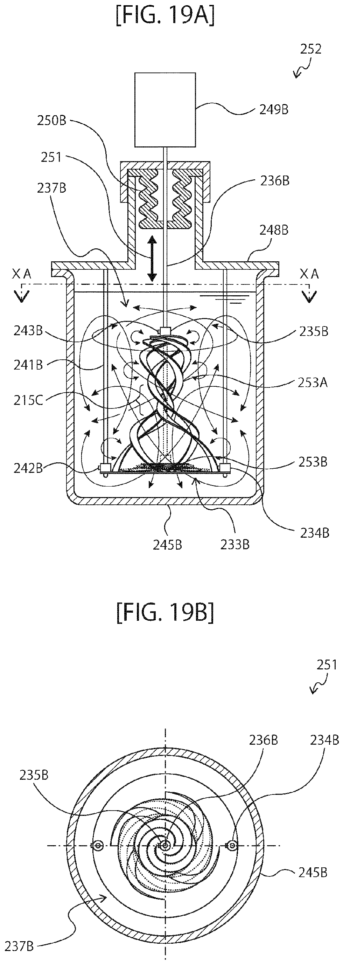

FIG. 19 illustrates a seventh embodiment of a stirring device including the stirring body according to the present invention, in which FIG. 19(A) is a schematic view illustrating the movement of a fluid by the stirring device including the stirring body, and FIG. 19(B) is a cross-sectional view illustrating the stirring device along the broken-line XA-XA of FIG. 19(A).

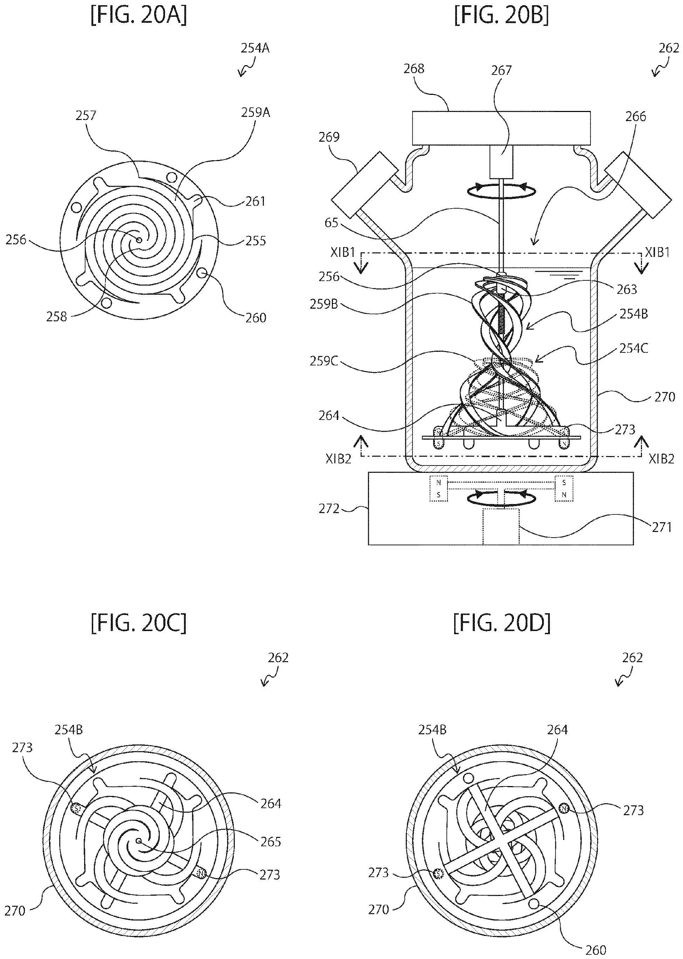

FIG. 20 illustrates an eighth embodiment of a stirring device including the stirring body according to the present invention, in which FIG. 20(A) is a plan view illustrating a flat plate of the stirring body according to the present invention, FIG. 20(B) is a side view illustrating the stirring device including the stirring body of FIG. 20(A), FIG. 20(C) is a cross-sectional view illustrating the stirring device along the broken-line XIB1-XIB1 of FIG. 20(B), and FIG. 20(D) is a cross-sectional view illustrating the stirring device along XIB2-XIB2 of FIG. 20(B).

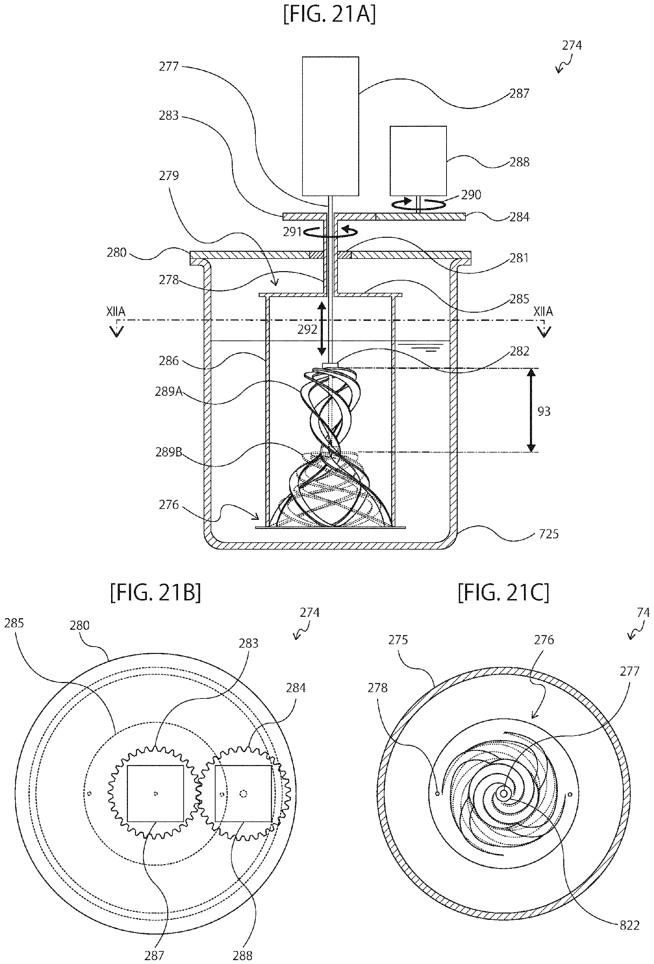

FIG. 21 illustrates a ninth embodiment of a stirring device including the stirring body according to the present invention, in which FIG. 21(A) is a side view illustrating a stirring device including the stirring body according to the present invention, FIG. 21(B) is a top view illustrating the stirring device of FIG. 21(A), and FIG. 21(C) is a cross-sectional view illustrating the stirring device along the broken-line XIIA-XIIA of FIG. 21(A).

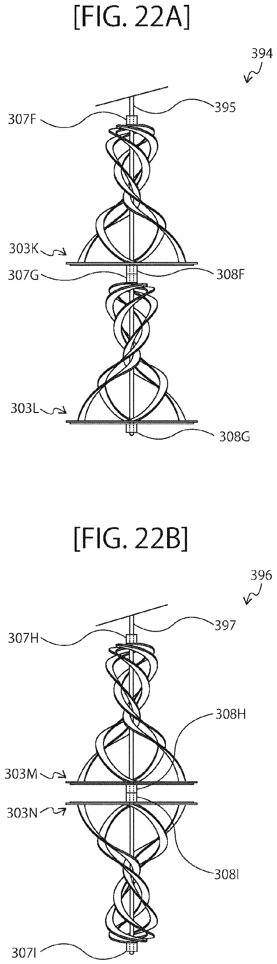

FIG. 22 illustrates embodiments of various configurations of the flat plate of the stirring body according to the present invention, in which FIG. 22(A) is a side view illustrating a stirring body in which two flat plates are arranged in a column, and FIG. 22(B) is a side view illustrating a stirring body in which two flat plates are arranged in a column with their outer peripheral portions adjacent to each other.

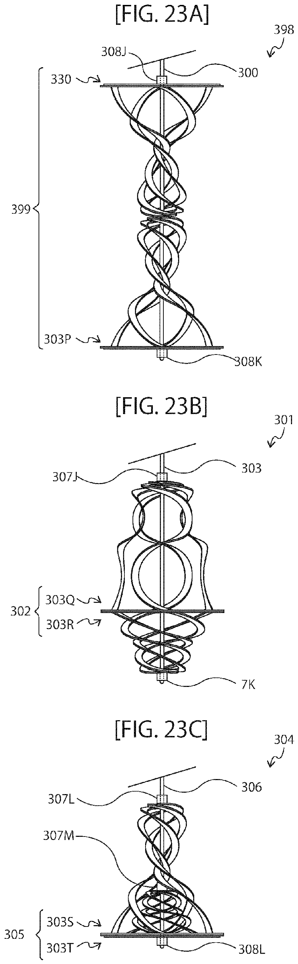

FIG. 23(A) is a side view illustrating a stirring body in which two flat plates are bonded to each other at the central portions thereof, FIG. 23(B) is a side view illustrating a stirring body in which two different flat plates are bonded to each other at the outer peripheral portions thereof, and FIG. 23(C) is a side view illustrating a stirring body including another set of flat plates inside one set of flat plates.

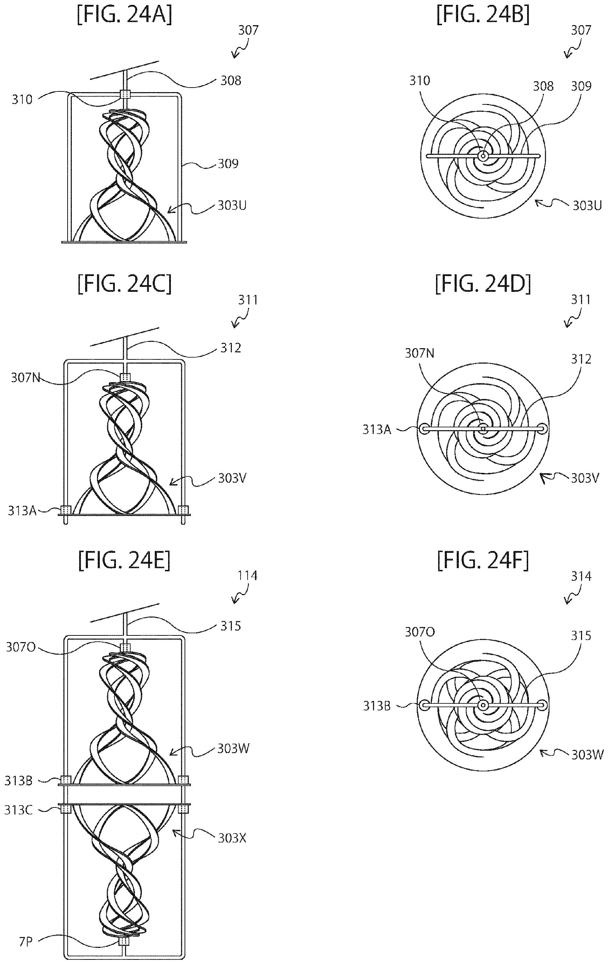

FIG. 24(A) is a side view illustrating a stirring body in which a movable member having a U-shape is bonded to an outer peripheral portion of a flat plate, FIG. 24(B) is a top view of the stirring body shown in FIG. 24(A), FIG. 24(C) is a side view illustrating a stirring body in which a flat plate is fixed to a guide member having three branch portions, FIG. 24(D) is a top view of the stirring body shown in FIG. 24(C), FIG. 24(E) is a side view illustrating a stirring body in which two flat plates having adjacent outer peripheral portions are fixed to a guide member having a rectangular frame, and FIG. 24(F) is a top view of the stirring body shown in FIG. 24(E).

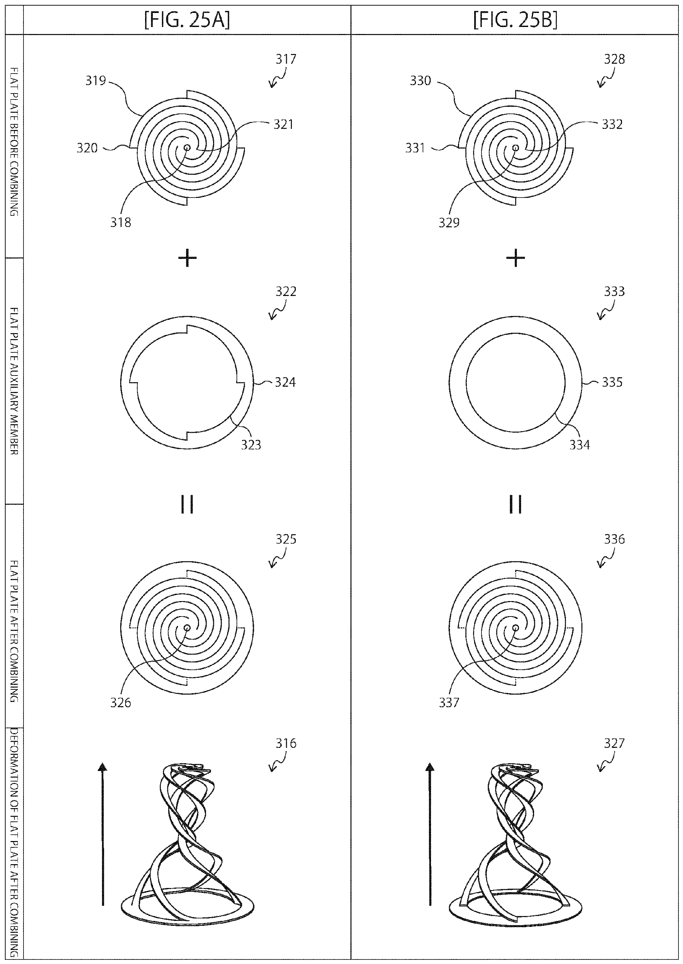

FIG. 25 illustrates embodiments of various forming methods of the flat plate of the stirring body according to the present invention, in which FIG. 25(A) illustrates a top view and a perspective view illustrating a flat plate in which a flat plate auxiliary member is bonded to the outer peripheral portion side, and FIG. 25(B) is a top view and a perspective view illustrating a flat plate in which a ring-shaped flat plate auxiliary member is bonded to the outer peripheral portion side.

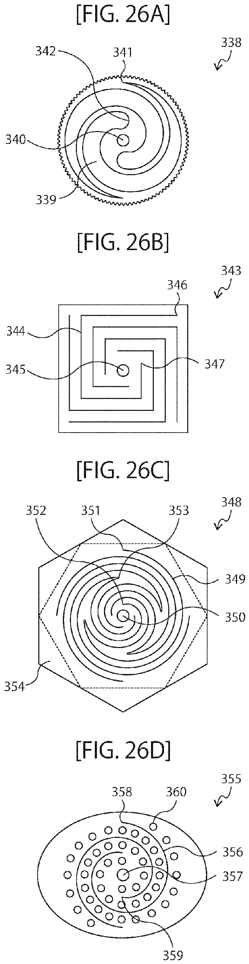

FIG. 26 illustrates embodiments of various cuts of a flat plate of the stirring body according to the present invention, in which FIG. 26(A) illustrates a circular flat plate having a wavy shape having a spiral cutout, FIG. 26(B) is a plan view illustrating a flat plate having a cut along the outer shape of a square, FIG. 26(C) is a plan view illustrating a regular hexagonal flat plate having a spiral cut and a flap extending in the left-right direction, and FIG. 26(D) is a plan view illustrating an elliptical flat plate having a plurality of through-holes.

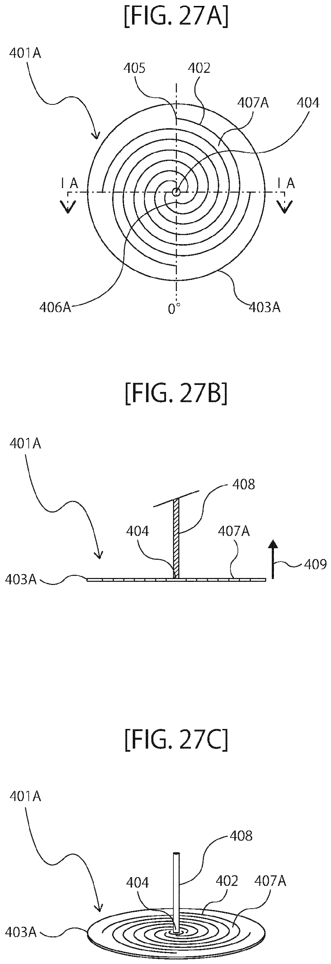

FIG. 27 shows a third embodiment of the stirring body according to the present invention, in which FIG. 27(A) is a plan view of the stirring body having a two-dimensional shape, FIG. 27(B) is a cross-sectional view showing the stirring body along the broken-line IA-IA of FIG. 27(A), and FIG. 27(C) is a perspective view of the stirring body having the two-dimensional shape of FIG. 27(A).

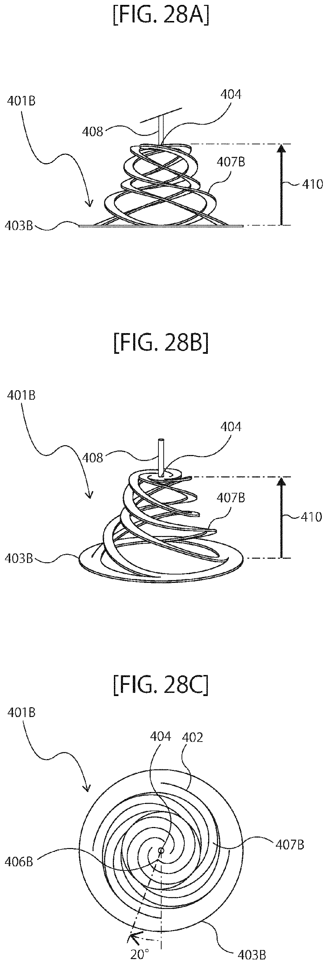

FIG. 28(A) is a side view of the stirring body extended from the two-dimensional shape shown in FIG. 27 to a three-dimensional shape, FIG. 28(B) is a perspective view of the stirring body having the three-dimensional shape of FIG. 28(A), and FIG. 28(C) is a top view of the stirring body having the three-dimensional shape of FIG. 28(A).

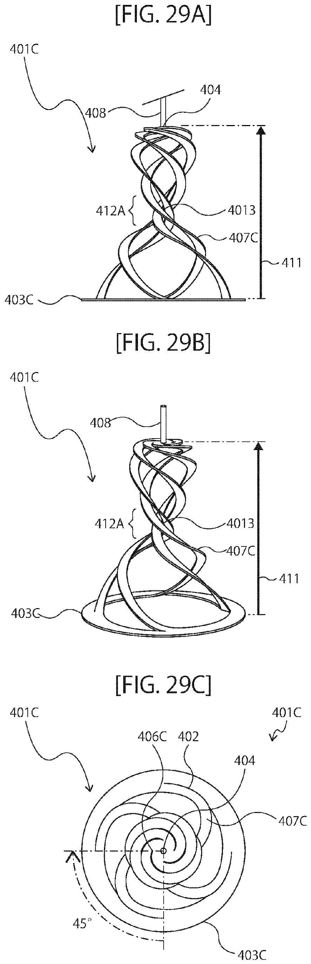

FIG. 29(A) is a side view showing a case where the stirring body shown in FIG. 28 is further extended, FIG. 29(B) is a perspective view of the stirring body having the three-dimensional shape of FIG. 29(A), and FIG. 29(C) is a top view of the stirring body having the three-dimensional shape of FIG. 29(A).

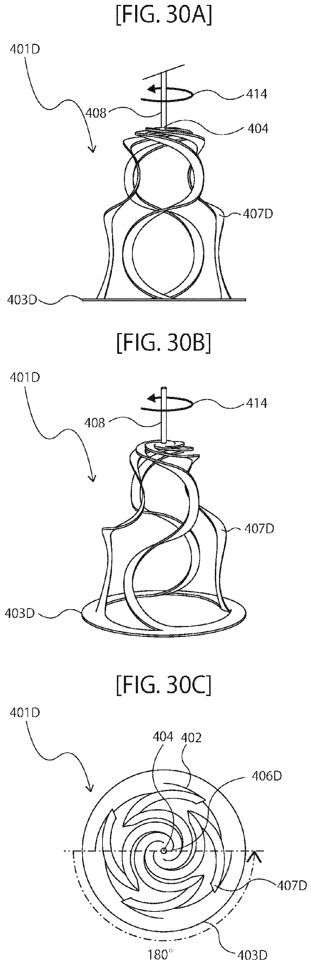

FIG. 30(A) is a side view when the central portion is twisted from the stirring body shown in FIG. 29, FIG. 30(B) is a perspective view of the stirring body having the three-dimensional shape of FIG. 30(A), and FIG. 30(C) is a top view of the stirring body having the three-dimensional shape of FIG. 30(A).

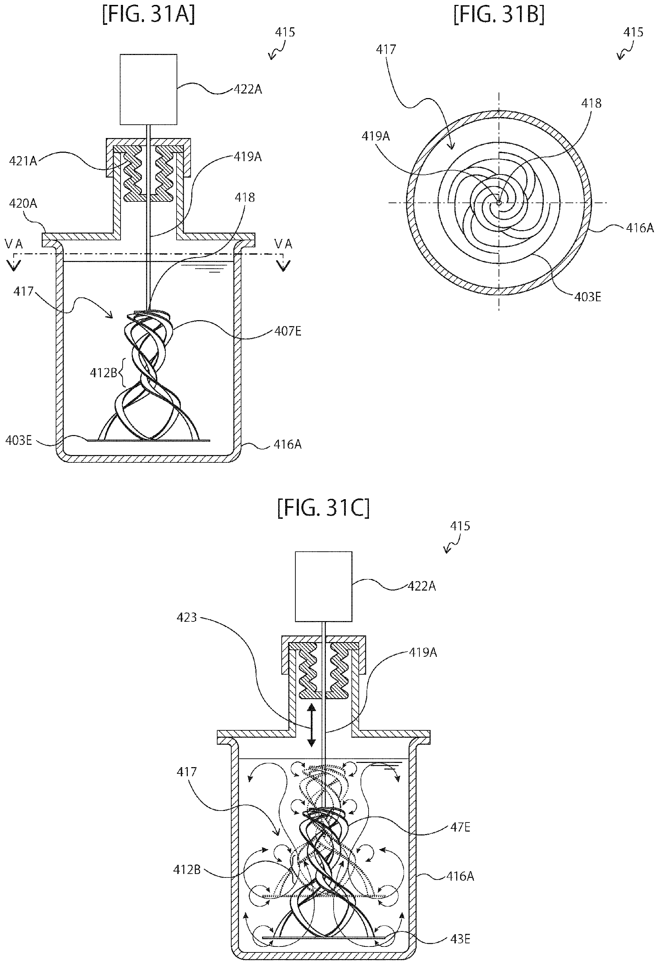

FIG. 31 shows a tenth embodiment of a stirring device including the stirring body according to the present invention, FIG. 31(A) is a side view showing the stirring device including the stirring body, FIG. 31(B) is a cross-sectional view showing the stirring device along the broken-line VA-VA of FIG. 31(A), and FIG. 31(C) is a schematic view showing the movement of a fluid by the stirring device of FIG. 31(A).

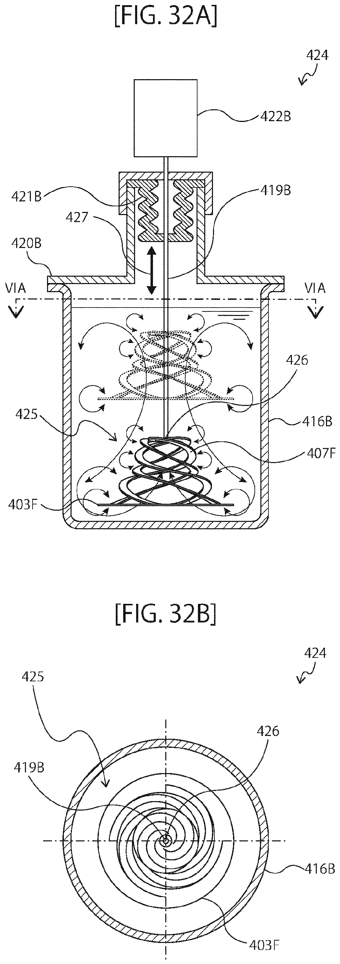

FIG. 32 shows an eleventh embodiment of a stirring device including the stirring body according to the present invention, in which FIG. 32(A) is a schematic view showing the movement of a fluid by another stirring device including the stirring body, and FIG. 32(B) is a cross-sectional view showing the stirring device along the broken-line VIA-VIA of FIG. 32(A).

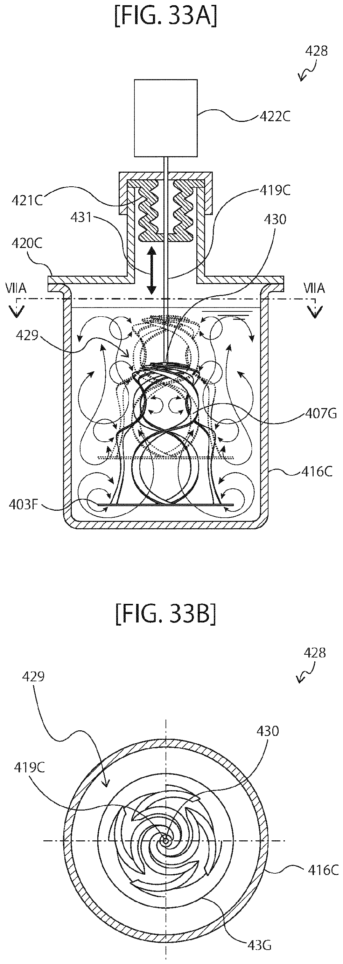

FIG. 33(A) is a schematic view showing the movement of a fluid by the stirring device including the stirring body, and FIG. 33(B) is a cross-sectional view showing the stirring device along the broken-line VIIA-VIIA of FIG. 33(A).

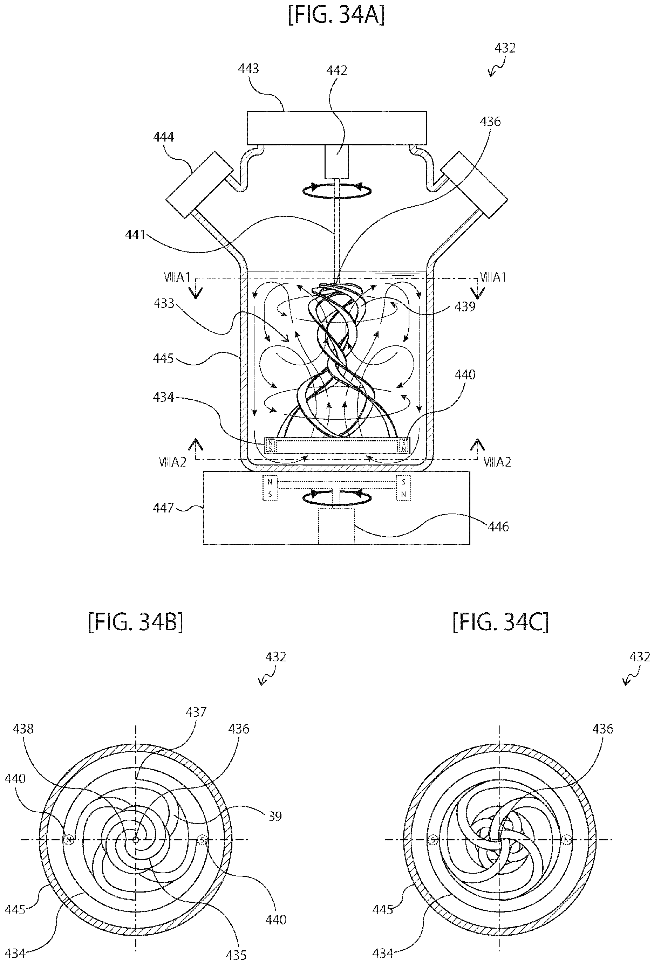

FIG. 34 illustrates a twelfth embodiment of a stirring device including the stirring body according to the present invention, in which FIG. 34(A) is a schematic view illustrating the movement of a fluid by the stirring device including the stirring body, FIG. 34(B) is a cross-sectional view illustrating the stirring device along the broken-line VIIIA1-VIIIA1, and FIG. 34(C) is a cross-sectional view illustrating the stirring device along the broken-line VIIIA2-VIIIA2 of FIG. 34(A).

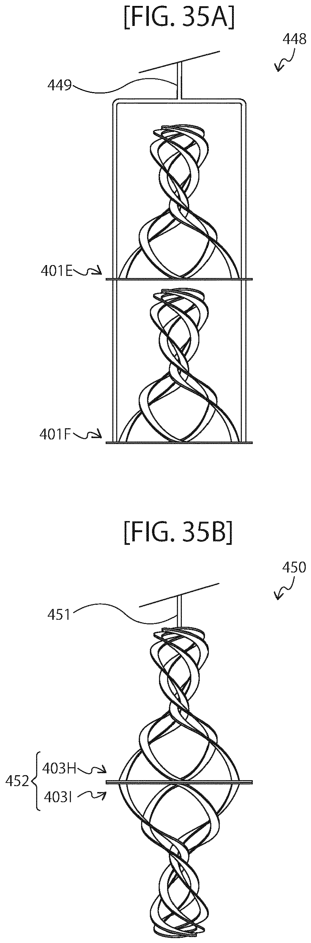

FIG. 35 illustrates an embodiment of a stirring blade provided in the fourth embodiment of the stirring body according to the present invention, in which FIG. 35(A) is a side view illustrating a stirring blade in which two stirring bodies are arranged in a column, and FIG. 35(B) is a side view illustrating a stirring blade including a stirring body in which two flat plates are bonded to each other at the outer peripheral portions thereof.

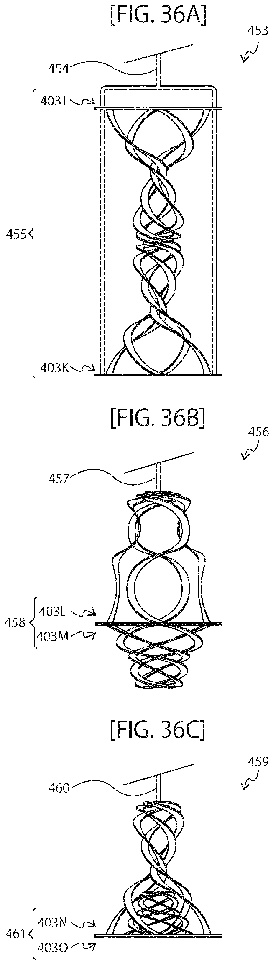

FIG. 36(A) is a side view illustrating a stirring blade including a stirring body in which two flat plates are bonded to each other at the central portions thereof, FIG. 36(B) is a side view illustrating a stirring blade including a stirring body in which two different flat plates are bonded to each other at the outer peripheral portions thereof, and FIG. 36(C) is a side view illustrating a stirring blade including a stirring body including another set of flat plates inside one set of flat plates.

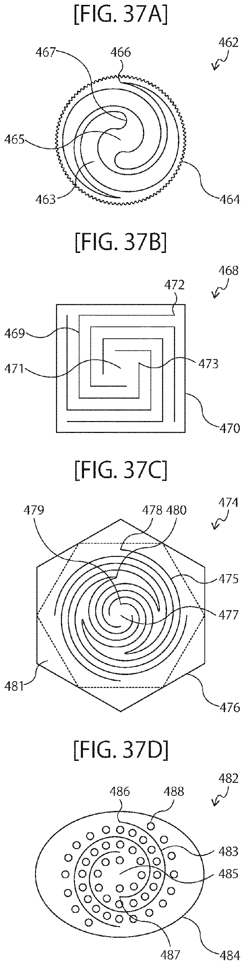

FIG. 37 illustrates embodiments of various cuts of a flat plate of the stirring body according to the present invention, in which FIG. 37(A) is a plan view illustrating a flat plate of a circular stirring body having a wavy shape having a spiral cutout, FIG. 37(B) is a plan view illustrating a flat plate of a stirring body having a cut along the outer shape of a square, FIG. 37(C) is a plan view illustrating a flat plate of a regular hexagonal stirring body having a spiral cut and a flap extending in the left-right direction, and FIG. 37(D) is a plan view illustrating a flat plate of an elliptical stirring body having a plurality of through-holes.

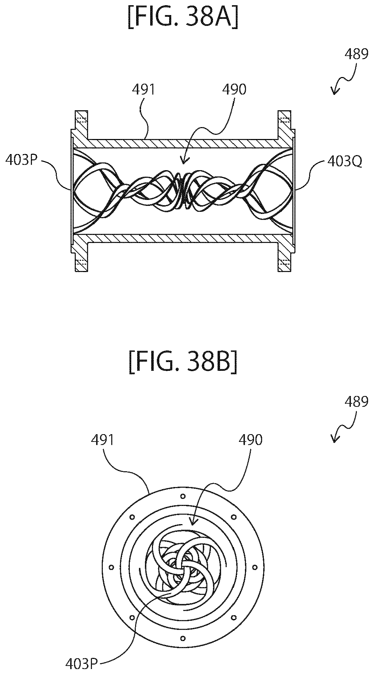

FIG. 38 illustrates a thirteenth embodiment of a stirring device including the stirring body according to the present invention, in which FIG. 38(A) is a conceptual cross-sectional view illustrating a mixer including a stirring body in which two flat plates are bonded to each other at the central portions thereof, and FIG. 38(B) is a front view of the mixer of FIG. 38(A).

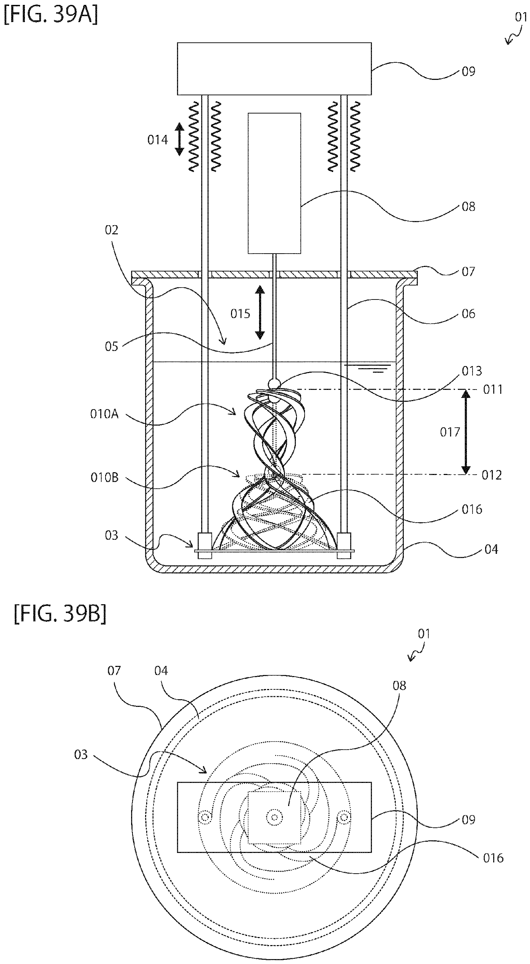

FIG. 39 illustrates a fourteenth embodiment of a stirring device including the stirring body according to the present invention, in which FIG. 39(A) is a cross-sectional view of the stirring device, and FIG. 39(B) is a plan view.

### MODE FOR CARRYING OUT THE INVENTION

A stirring body of the present invention includes a flat plate in which a plurality of spiral blades extending from a central portion is formed by a spiral cut that does not contact an outer peripheral portion; and an operating member that is movable in a direction of separating from the flat plate in a state of supporting the central portion of the flat plate, wherein the blade portion rises while being separated from an adjacent blade portion in conjunction with the movement of the operating member in the direction of separating from the flat plate so that the flat plate is extended to form a three-dimensional shape.

In the first embodiment described later, the operating member of the stirring body corresponds to a support member that supports one of the outer peripheral portion and the central portion of the flat plate, and a movable member that supports the other of the outer peripheral portion and the central portion and can reciprocate in relation to the support member in a direction in which the outer peripheral portion and the central portion approach and separate from each other.

Further, in the second embodiment described later, the operating member corresponds to a guide member and a movable member connected to one of the central portion and the outer peripheral portion of the flat plate so as to be rotatable and movable along the guide member.

Further, in the third and fourth embodiments described later, the operating member corresponds to a shaft member.

Hereinafter, embodiments of the stirring body and the stirring device including the stirring body according to the present invention will be described with reference to the drawings.

### (First Embodiment of Stirring Device)

In stirring bodies 1A, 1B, and 1C of the embodiment according to the present invention, a plurality of Archimedean spiral (that is, spiral) cuts 2 extending outward from the vicinity of a central portion 4A so as not to contact the outer peripheral edge of a flat plate 3A, 3B, or 3C made of an elastically deformable material are formed at equal angular intervals around the central portion 4A, so that a plurality of spiral blade portions 7A, 7B, and 7C formed by the cuts 2 are arranged at predetermined angular intervals in the circumferential direction around the central portion 4A. The flat plates 3B and 3C of the stirring bodies 1B and 1C shown in FIGS. 2 and 3 show two different deformed forms having a multiple-spiral structure formed by elastically deforming the flat plate 3A of the stirring body 1A shown in FIG. 1. The stirring body 1A of the present embodiment shown in FIG. 1 is the same as the stirring bodies 1B and 1C of the present embodiment shown in FIGS. 2 and 3, except for the flat plates 3A, 3B, and 3C that form different deformed forms by elastic deformation.

As shown in FIG. 1(A), in the stirring body 1A, four cuts 2 are formed so as to extend in a spiral form about 540 degrees around the central portion 4A from the outer-peripheral-side ends 5 located inside the outer peripheral edge of the circular flat plate 3A and provided around the central portion 4A at intervals of 90 degrees toward the inner-peripheral-side ends 6A located near the central portion 4A and provided around the central portion 4A at intervals of 90 degrees. That is, four blade portions 7A are arranged at positions rotated by 90 degrees in the circumferential direction around the central portion 4A with respect to the adjacent blade portions 7A, respectively, and are formed so as to extend in a spiral form about 540 degrees around the central portion 4A. The outer-peripheral-side end 5 of the cut 2 is preferably formed so as to be separated from the outer peripheral edge toward the central portion 4A by a distance of 5 to 10% of the diameter of the flat plate 3A. The inner-peripheral-side end 6A of the cut 2 is preferably formed so as to be separated from the center of the flat plate 3A toward the outer peripheral edge by a distance of 5 to 10% of the diameter of the flat plate 3A.

Further, as shown in FIGS. 1(B) and 1(C), the stirring body 1A has a convex spherical portion at the tip of a movable member 8 having a pipe-type structure, the central portion 4A of the flat plate 3A is rotatably supported through a circular through-hole provided in the central portion 4A of the flat plate 3A, and a branch portion provided in the support member 9 arranged in the manner which can reciprocate in the tube of the movable member 8 branches halfway into two parts so as to be orthogonal to the movable member 8, curves along the spherical portion provided in the movable member 8, terminates in the vicinity of the outer peripheral edge of the flat plate 3A, and is bonded to the outer peripheral portion on the bottom surface side of the flat plate 3A. Thus, the movable member 8 can reciprocate in relation to the support member 9 in direction in which the outer peripheral portion and the central portion 4A of the flat plate 3A approach and separate from each other. In this way, when the movable member 8 is moved (that is, lowered) below the flat plate 3A in the normal direction of the flat plate 3A, the blade portion 7A formed by the four cuts 2 is closed and the flat plate 3A having a two-dimensional shape is formed.

As shown in FIGS. 2(A) and 2(B), in the stirring body 1B, the movable member 8 shown in FIG. 1(B) is moved (that is, raised) to an extension position 11 shown in FIG. 2(A) in the direction of the arrow 10 and the central portion 4A of the flat plate 3A shown in FIGS. 1(B) and 1(C) is moved (that is, raised), whereby the blade portion 7A is extended and elastically deformed. In this way, a three-dimensional flat plate 3B having a truncated cone-shaped blade portion 7B as shown in FIGS. 2(A) and 2(B) is formed.

Further, as shown in FIG. 2(C), in the stirring body 1B, the movable member 8 shown in FIG. 1(B) is moved (that is, raised) to the extension position 11 shown in FIG. 2(A) in the direction of the arrow 10, and the blade portion 7A shown in FIG. 1(A) is extended and elastically deformed while being twisted so as to rotate in the direction of the cut 2 facing the central portion 4A, that is, in the right-handed winding direction. In this way, the inner-peripheral-side end 6A shown in FIG. 1(A) is rotated about 20 degrees in the right-handed winding direction with respect to the central portion 4A as the base axis and is rotationally moved to the position of the inner-peripheral-side end 6B shown in FIG. 2(C). In this way, a three-dimensional flat plate 3B having a truncated cone-shaped blade portion 7B as shown in FIGS. 2(A) and 2(B) is formed.

As shown in FIGS. 3(A) and 3(B), in the stirring body 1C, the movable member 8 shown in FIG. 2(A) is further raised from the extension position 11 to an extension position 12 shown in FIG. 3(A), the central portion 4A of the flat plate 3B shown in FIG. 2(A) is further raised, and the blade portion 7B is elastically deformed while being twisted accompanied by extension movement, outward spreading (hereinafter referred to as "spreading movement"), and convergence toward the center of the flat plate 3B (hereinafter referred to as "converging movement"). In this way, as shown in FIGS. 3(A) and 3(B), a three-dimensional flat plate 3C having a blade portion 7C that forms a neck portion 13A having a neck shape and creates a singular point 14 at which a plurality of blade portions 7C converges according to the strength of pressure is formed.

Further, as shown in FIG. 3(C), in the stirring body 1C, the movable member 8 shown in FIG. 2(A) is further raised from the extension position 11 to the extension position 12 shown in FIG. 3(A), and the blade portion 7B shown in FIG. 2(C) is elastically deformed while being twisted so as to further rotate in the right-handed winding direction accompanied by extension movement, spreading movement, and converging movement. In this way, the inner-peripheral-side end 6A shown in FIG. 1(A) is rotated about 45 degrees in the right-handed winding direction around the central portion 4A as the base axis and is rotationally moved to the position of the inner-peripheral-side end 6C shown in FIG. 3(C). In this way, a three-dimensional flat plate 3C having a blade portion 7C that forms a neck portion 13A is formed as shown in FIGS. 3(A) and 3(B).

According to the aforementioned stirring bodies 1A, 1B, and 1C, the movable member 8 can reciprocate in relation to the support member 9 in a direction in which the outer peripheral portion of the flat plate 3A and the central portion 4A approach and separate from each other. When the movable member 8 is moved in the direction of the arrow 10 shown in FIG. 1(B), that is, the normal direction of the flat plate 3A by a predetermined physical means, the flat plates 3A, 3B, and 3C made of an elastically deformable material having four blade portions 7A, 7B, and 7C formed by the four cuts 2 are elastically deformed while being twisted accompanied by a plurality of operation movements including extension movement. In this way, the flat plate can dynamically behave by forming a deformed form reversibly from the two-dimensional flat plate 3A having the blade portion 7A in which four spiral cuts 2 are closed to the three-dimensional flat plate 3B having the truncated cone-shaped blade portion 7B. Moreover, the flat plate can dynamically behave by forming a deformed form reversibly from the state of the flat plate 3B to the three-dimensional flat plate 3C having the blade portion 7C that forms the neck portion 13A by the flat plate 3B elastically deforming while the blade portion 7B being twisted, accompanied by the extension movement, the spreading movement, and the converging movement toward the center and creates the singular point 14 according to the strength of pressure. Further, since the spiral cut 2 is formed in the flat plates 3A, 3B, and 3C, when the central portion 4A of the flat plate 3A is raised, the blade portion 7A is rotated around the central portion 4A as the base axis in the direction of the cut 2 facing the central portion 4A, that is, in the right-handed winding direction, and the inner-peripheral-side end 6A of the flat plate 3A is rotationally moved in the right-handed winding direction with the central portion 4A as the base axis and is elastically deformed while being twisted accompanied by a plurality of operation movements including extension movement. In this way, the flat plate can dynamically behave by forming a deformed form reversibly from the two-dimensional flat plate 3A to the three-dimensional flat plates 3B and 3C. In this way, the cut 2 functions as an expansion/contraction movement structure and a rotation structure of the blade portions 7A, 7B, and 7C and a spreading/converging movement structure of the blade portions 7A, 7B, and 7C accompanied by the spreading movement and the converging movement. Moreover, the cut 2 functions as a displacement amount adjustment structure that adjusts, an expansion/contraction movement width, a rotational movement width, and a spreading/convergence movement width of the blade portions 7A, 7B, and 7C according to the length of the cut 2 extending around the central portion 4A, that is, the angle extending around the central portion 4A (hereinafter referred to as "circumferential angle"). Further, the movable member 8 functions as a shape deformation adjustment structure of the flat plates 3A, 3B, and 3C forming the deformed form. Since the flat plates 3A, 3B, and 3C have a circular outer shape without a tip wing, the flat plate functions as a container damage protection structure that prevents the inside of a container such as a stirring tank for stirring a stirring substance from being damaged. Therefore, for example, when an elastically deformable material is used as the material of the flat plates 3A, 3B, and 3C of the aforementioned stirring bodies 1A, 1B, and 1C of the present embodiment and the movable member 8 is appropriately reciprocated in relation to the support member 9 by a predetermined physical means in a direction in which the outer peripheral portion and the central portion 4A of the flat plate 3A approach and separate from each other, the blade portions 7A, 7B, and 7C is elastically deformed while being twisted accompanied by expansion/contraction movement in the vertical direction, rotational movement in forward and reverse rotation directions, and spreading movement and spreading/converging movement and the flat plates 3A, 3B, and 3C dynamically behave so as to repeatedly form different deformed forms reversibly. Thus, it is possible to provide a stirring blade that can stir a fluid medium in an extremely complex and unsteady manner with low shear, accompanied by a plurality of flows in a liquid, such as a vortex flow, a separation flow, a suction flow, an extrusion flow, a swirling flow, a vertical convection flow, a circulating flow, and a turbulent flow, and can adjust the flow of stirring flow by controlling a reciprocating distance of the movable member and the flat plate appropriately and reversibly forming deformed forms with different displacement amounts by elastic deformation and that can be manufactured easily by preventing damage in the stirring tank.

Further, the position of the movable member 8 of the aforementioned stirring bodies 1A, 1B, and 1C is not limited to the central portion 4A of the flat plates 3A, 3B, and 3C. For example, although not shown in the drawings, a movement auxiliary member or the like may be arranged between the flat plates 3A, 3B, and 3C and the movable member 8 so as to be rotatable in relation to the outer peripheral portion. The position of the movable member 8 of the aforementioned stirring bodies 1A, 1B, and 1C is not particularly limited.

### (First Embodiment of Stirring Device)

A first embodiment of the stirring body according to the present invention includes a flat plate in which a plurality of spiral blade portions extending from a central portion is formed by a spiral cut that does not contact an outer peripheral portion and which is made of an elastically deformable material, a support member that supports one of the outer peripheral portion and the central portion of the flat plate, and a movable member that supports the other of the outer peripheral portion and the central portion. The movable member is reciprocated in relation to the support member in a direction in which the outer peripheral portion and the central portion approach and separate from each other. The movable member can be used as a stirring blade of a stirring device capable of stirring a fluid medium in an unsteady manner.

Hereinafter, the first embodiment of the stirring device including the stirring body according to the present invention will be described with reference to FIG. 4. A flat plate 3D of a stirring body 19 provided in a stirring device 15 of the present embodiment shown in FIG. 4 is made of an elastically deformable material, and has exactly the same shape as the flat plates 3A, 3B, and 3C shown in FIGS. 1 to 3, that is, the same one is used. The stirring body 19 shown in FIG. 4 has the same configuration as the stirring bodies 1A, 1B, and 1C shown in FIGS. 1 to 3 except that four support members 18 are provided around a central portion 4B of the flat plate 3D at intervals of 90 degrees and arranged at positions of the outer peripheral portion and bonded to the flat plate 3D and a top plate portion 20. Thus, the detailed description thereof will be omitted here.

As shown in FIG. 4(A), the stirring device 15 includes a stirring tank 16 containing a liquid and having a volume of 300 mL, the stirring body 19 including the circular flat plate 3D, the movable member 17 and the support member 18, a top plate portion 20, a rubber seal 21 having a bellows structure, and a driving device 22 in which a brushless motor is provided and which reciprocates in the vertical direction. The driving shaft of the driving device 22 vertically suspended in the stirring tank 16 and the movable member 17 are bonded to each other. The movable member 17 passes through a circular through-hole provided in the central portion 4B of the flat plate 3D to rotatably support the central portion 4B side of the flat plate 3D between two adjacent convex spherical portions provided at the tip of the movable member 17. The driving shaft of the driving device 22 passes through a center-side opening of the top plate portion 20 and is connected to the seal 21 so as to follow the reciprocation. Thus, the inside of the stirring tank 16 is airtightly provided. The blade portion 7D shows a deformed form of the flat plate 3D having a neck portion 13B at a substantially uppermost position of the movable range in which the movable member 17 reciprocates, and a broken-line blade portion 7E shows a deformed form of the flat plate 3D at a substantially lowermost position of the movable range in which the movable member 17 reciprocates.

As shown in FIG. 4(B), in the stirring device 15, the driving device 22 is driven by a predetermined physical means to cause the movable member 17 to reciprocate in the direction of the arrow 23, and the flat plate 3D is elastically deformed. In this way, the flat plate 3D behaves dynamically by forming a deformed form reversibly while the blade portions 7D and 7E being twisted, accompanied by a plurality of operation movements such as an expansion/contraction movement in the vertical direction, a rotational movement in the forward and reverse rotation directions, a spreading movement, and a converging movement. Thus, a plurality of flows such as a vortex flow, a separation flow, a suction flow, an extrusion flow, a swirling flow, a vertical convection flow, a circulating flow, and a turbulent flow is generated in the liquid in the stirring tank 16. That is, FIG. 4(B) schematically shows the movement of a stirring flow in the stirring tank 16 by the stirring device 15 shown in FIG. 4(A).

As shown in FIG. 4(C), in the stirring device 15, four supporting members 18 vertically suspended in the stirring tank 16 are provided around the central portion 4B of the flat plate 3D at intervals of 90 degrees and are arranged in the outer peripheral portion of the flat plate 3D and bonded to the flat plate 3D and the top plate portion 20 as shown in FIG. 4(A). Thus, the outer peripheral portion side of the flat plate 3D is fixed. In this way, when the driving device 22 shown in FIG. 4(B) is driven by a predetermined physical means to cause the movable member 17 to reciprocate in the direction of the arrow 23, the flat plate 3D behaves dynamically so as to repeatedly form different deformed forms reversibly. Thus, the liquid in the stirring tank 16 can be stirred in an unsteady manner.

According to the stirring device 15 including the aforementioned stirring body 19, when the driving device 22 provided with the brushless motor is driven by a predetermined physical means, the movable member 17 reciprocates in relation to the support member 18 in a direction in which the outer peripheral portion and the central portion 4B of the flat plate 3D approach and separate from each other, that is, in the direction of the arrow 23. The blade portions 7D and 7E of the flat plate 3D made of an elastically deformable material are elastically deformed accompanied by an expansion/contraction movement in the vertical direction, a rotational movement in the forward and reverse rotation directions, the spreading movement, and the converging movement. In this way, the flat plate can dynamically behave so as to repeatedly form different deformed forms reversibly from the two-dimensional flat plate 3D to the truncated cone-shaped three-dimensional flat plate 3D. Moreover, the flat plate 3D is elastically deformed while the blade portion 7E being twisted accompanied by an extension movement, a spreading movement, and a converging movement toward the center. In this way, the flat plate can dynamically behave so as to repeatedly form different deformed forms reversibly from the truncated cone-shaped three-dimensional flat plate 3D to a three-dimensional flat plate 3D having the blade portion 7D forming the neck portion 13B. In this way, the spiral blade portions 7D and 7E reciprocate in the vertical direction in the liquid in the stirring tank 16, and the blade portions 7D and 7E generate a plurality of flows accompanied by a vortex flow around the adjacent blade portions 7D and 7E of the blade portions 7D and 7E and the gaps therebetween. Moreover, the central portion 4B of the flat plate 3D moves vertically to generate a vertical convection flow. Further, the neck portion 13B formed by the vertical movement of the central portion 4B of the flat plate 3D generates a vortex flow based on a separation flow. The blade portions 7D and 7E rotationally move in forward and reverse directions with the central portion 4B of the flat plate 3D as the base axis and generate a swirling flow. The central portion 4B of the flat plate 3D is raised and generates a suction flow rising due to a suction force from the bottom side of the stirring tank 16 toward the central portion of the flat plate 3D. The suction flow hits the neck portion 13B and the blade portions 7D and 7E that converge each other and generates a vortex flow and a vertical convection flow. The central portion 4B of the flat plate 3D is lowered and the blade portions 7D and 7E and the neck portion 13B spread toward the bottom side in the stirring tank 16 to generate an extrusion flow that descend and a vertical convection flow. In this way, the stirring device functions as a stirring device having a stirring blade capable of generating a plurality of flows in the liquid, such as a vortex flow, a separation flow, a suction flow, an extrusion flow, a swirling flow, a vertical convection flow, a circulating flow, and a turbulent flow and stirring a fluid medium in an extremely complex and unsteady manner with low shear. Therefore, for example, when a physicochemical environment suitable for proliferation and metabolism of cultured cells, differentiation and maturation of megakaryocytes, polynuclearization of megakaryocytes, production of platelets, and maintenance of bioactivity of platelets is established in the aforementioned stirring device 15 and a culture solution is stirred, it is possible to provide a stirring blade capable of stirring the culture solution in a more complex and unsteady manner with low shear accompanied by a plurality of flows as compared with conventional unsteady stirring required for stirring culture of culturing megakaryocyte cells derived from human iPS cells to produce a large amount of platelets. Moreover, it is possible to provide a stirring blade that can be manufactured easily, without the need to provide a plurality of driving devices for driving the stirring blade in different operating directions or a complex mechanism for realizing a plurality of operating directions with one driving device, which was required in a stirring device having a plurality of operating directions such that a stirring blade reciprocates in the vertical direction and the forward and reverse rotation directions and to provide a stirring device including this stirring blade.

The material of the seal 21 of the stirring device 15 including the aforementioned stirring body 19 may be, for example, silicon, resin, metal, polytetrafluoroethylene (registered trademark name: Teflon) or the like, in addition to rubber, and the material of the seal 21 of the stirring device 15 is not particularly limited.

Further, the structure of the seal 21 of the stirring device 15 including the aforementioned stirring body 19 may be, for example, a flexible membrane or a diaphragm, in addition to the bellows structure, and the structure of the seal 21 of the aforementioned stirring device 15 is not particularly limited.

### (Second Embodiment of Stirring Device)

The first embodiment of the stirring body according to the present invention includes a flat plate in which a plurality of spiral blade portions extending from a central portion is formed by a spiral cut that does not contact an outer peripheral portion and which is made of an elastically deformable material, a support member that supports one of the outer peripheral portion and the central portion of the flat plate, and a movable member that supports the other of the outer peripheral portion and the central portion. The movable member is reciprocated in relation to the support member in a direction in which the outer peripheral portion and the central portion approach and separate from each other. The stirring body can be used as a stirring blade of a stirring device capable of stirring a fluid medium in an unsteady manner, generating a complex vertical reflux to stir a fluid medium satisfactorily without any mixing unevenness by rotating the support member, and adjust the flow of a stirring flow. Hereinafter, a second embodiment of a stirring device including the stirring body according to the present invention will be described with reference to FIG. 5. A flat plate 26 of a stirring body 29 provided in a stirring device stirring device 24 of the present embodiment shown in FIG. 5 is the same as the flat plate 3D of the stirring body 19 shown in FIG. 4. Therefore, the detailed description thereof will be omitted here.

As shown in FIG. 5, the stirring device 24 includes a stirring tank 25 containing a liquid and having a volume of 300 mL, the stirring body 29 including a circular flat plate 26 made of an elastically deformable material, a movable member 27, and a support member 28, a top plate portion 30, ceramic bearings 31 and 32, gears 33 and 34, a protective portion 35, a branch portion 36, a driving device 37 in which a brushless motor is provided and which has a controller capable of adjusting a reciprocating distance in the vertical direction, and a driving device 38 that rotates. The movable member 27 reciprocates and the support member 28 rotates according to the driving devices 37 and 38. In this way, the flat plate 26 rotates accompanied with dynamic behavior so as to repeatedly form different deformed forms reversibly by elastic deformation. The three-dimensional flat plate 26 forming a blade portion 39A having a neck portion has exactly the same shape as the flat plate 3C shown in FIG. 3. A three-dimensional flat plate 26 having a broken-line truncated cone-shaped blade portion 39B has exactly the same shape as the flat plate 3B shown in FIG. 2. The flat plate 26 having the blade portion 39A shows a deformed form at a substantially uppermost position of a movable range in which the movable member 27 reciprocates. The flat plate 26 having the blade portion 39B shows a deformed form at a substantially lowermost position of a movable range in which the movable member 27 reciprocates. However, the material of the bearings 31 and 32 may be stainless steel, resin or the like in addition to ceramics, and the material of the bearings 31 and 32 is not particularly limited.

As shown in FIGS. 5(A) to 5(C), in the stirring device 24, the support member 28 having a pipe-type structure as shown in FIG. 5(A) includes the gear 33 provided on the upper part of the central shaft thereof and the circular protective portion 35 provided in the lower part thereof so as to prevent liquid from adhering to the bearing 31. The support member 28 is connected to the bearing 31 provided on the top plate portion 30, and two branch portions 36 are provided around the central portion of the flat plate 26 at intervals of 180 degrees and bonded to the protective portion 35 and the outer peripheral portion of the flat plate 26. The gear 33 and the gear 34 provided on the driving shaft of the driving device 38 are rotatably combined. Further, the movable member 27, which is the driving shaft of the driving device 37, is vertically suspended in the stirring tank 25 and connected to the bearing 33 provided in the central portion of the flat plate 26, so that the central portion side of the flat plate 26 is rotatably supported in relation to the movable member 27. In this way, when the driving shaft of the driving device 38 rotates in the direction of the arrow 40, the support member 28 having the gear 33 connected to the gear 34 is rotated in the direction of the arrow 41, and the flat plate 26 is rotated. Moreover, the movable member 27, which is the driving shaft of the driving device 37, is reciprocated in the direction of the arrow 42, and the central portion side of the flat plate 26 is reciprocated in the vertical direction. As shown in FIG. 5(A) the flat plate 26 is elastically deformed to the flat plate 26 forming the blade portion 39A having a neck portion and the flat plate 26 having the truncated cone-shaped blade portion 39B and the flat plate 26 is rotated accompanied by dynamic behavior so as to repeatedly form different deformed forms reversibly. The driving device 37 includes a controller for adjusting the relative moving distance between the movable member 27 and the support member 28, and can appropriately reciprocate within a distance of the movable range 43.

According to the stirring device 24 including the aforementioned stirring body 29, when the driving devices 37 and 38 are driven by predetermined physical means so that the movable member 27 and the support member 28 appropriately reciprocate and rotate, the flat plate 26 made of an elastically deformable material is elastically deformed. Thus, the flat plate 26 can dynamically behave so as to repeatedly form different deformed forms reversibly from the three-dimensional flat plate 26 having the truncated cone-shaped blade portion 39B to the three-dimensional flat plate 26 forming the blade portion 39A having a neck portion, and the support member 28 can rotate around the central portion of the flat plate 26 as the base axis. In this way, the movable member 27 reciprocates in the vertical direction in the liquid in the stirring tank 25, and the blade portions 39A and 39B generate a plurality of flows accompanied by a vortex flow around the adjacent blade portions 39A and 39B of the blade portions 39A and 39B and the gaps therebetween. Moreover, the central portion of the flat plate 26 moves vertically to generate a vertical convection flow. Further, the neck portion formed by the vertical movement of the central portion of the flat plate 26 generates a vortex flow based on a separation flow. The central portion of the flat plate 26 is raised and generates a suction flow rising due to a suction force from the bottom side of the stirring tank 25 toward the central portion of the flat plate 26. The suction flow hits the neck portion and the blade portions 39A and 39B that converge each other and generates a vortex flow and a vertical convection flow. The central portion of the flat plate 26 is lowered and the blade portions 39A and 39B and the neck portion spread and move toward the bottom side in the stirring tank 25 to generate an extrusion flow and a vertical convection flow that descend. In this way, the blade portions 39A and 39B can generate a plurality of flows such as a vortex flow, a separation flow, a suction flow, an extrusion flow, a vertical convection flow, a circulating flow, and a turbulent flow and stir a fluid medium in an extremely complex and unsteady manner with low shear. Further, the support member 28 rotates around the central portion of the three-dimensional flat plate 26 having the broken-line truncated cone-shaped blade portion 39B as the base axis. Moreover, a complex vertical reflux is generated accompanied by a plurality of flows such as a swirling flow, a vertical convection flow, and a suction flow due to a suction force generated from the bottom side near the outer peripheral portion of the flat plate 26 toward the central portion of the flat plate 26. In this way, it is possible to satisfactorily stir a fluid medium without any mixing unevenness. Further, the support member 28 rotates around the central portion of the three-dimensional flat plate 26 forming the blade portion 39A having the neck portion as the base axis. Moreover, a complex vertical reflux is generated accompanied by a plurality of flows such as a suction flow due to a suction force generated from the bottom side near the outer peripheral portion of the flat plate 26 toward the central portion of the flat plate 26, a suction flow due to a suction force generated from the vicinity of the neck portion toward the central portion of the flat plate 26, a swirling flow, and a vertical convection flow. In this way, it is possible to satisfactorily stir a fluid medium without any mixing unevenness. Furthermore, since the controller provided in the driving device 37 adjusts the relative moving distance between the movable member 27 and the support member 28, the flat plate 26 can function as a displacement amount adjustment mechanism, that is, a shape deformation adjustment mechanism capable of appropriately forming a plurality of deformed forms with different displacement amounts by elastic deformation. Moreover, since the driving devices 37 and 38 adjust the operating direction based on reciprocating movement, rotational movement, or a combination thereof, the controller can function as a mechanism for adjusting the operation of the flat plate 26. Therefore, for example, when an elastically deformable material is used for the material of the flat plate 26 of the aforementioned stirring body 29 of the present embodiment and the driving devices 37 and 38 are driven by a predetermined physical means to cause the movable member 27 and the support member 28 to appropriately reciprocate and rotate, the movable member 27 reciprocates in relation to the support member 28 in a direction in which the outer peripheral portion and the central portion of the flat plate 26 approach and separate from each other. The blade portions 39A and 39B are elastically deformed while being twisted accompanied by an expansion/contraction movement in the vertical direction, a rotational movement in forward and reverse directions, a spreading movement, and a spreading/converging movement and the flat plate 26 behaves dynamically so as to repeatedly form different deformed forms reversibly. In this way, it is possible to stir a fluid medium in the liquid in an extremely complex and unsteady manner with low shear accompanied by a plurality of flows such as a vortex flow, a separation flow, a suction flow, an extrusion flow, a swirling flow, a vertical convection flow, a circulating flow, and a turbulent flow. Moreover, when the support member 28 rotates around the central portion of the flat plate 26 as the base axis, the flat plate 26 is elastically deformed to behave dynamically so as to repeatedly and reversibly form different deformed forms including the three-dimensional flat plate 26 having the truncated cone-shaped blade portion 39B and the three-dimensional flat plate 26 forming the blade portion 39A having the neck portion. In this way, it is possible to generate a complex vertical reflux accompanied by a plurality of flows such as a suction flow, a swirling flow, and a vertical convection flow and satisfactorily stir a fluid medium without any mixing unevenness. Moreover, the controller provided in the driving device 37 controls the reciprocating distance of the movable member 27 and the flat plate 26 is elastically deformed to appropriately form deformed forms with different displacement amounts reversibly. In this way, it is possible to provide a stirring blade that can automatically adjust the flow of a stirring flow and that can be easily manufactured. However, the configuration of the aforementioned stirring device 24 that is driven in the plurality of operating directions is not limited to a configuration in which the gears 33 and 34 are combined and the driving devices 37 and 38 are arranged on the upper side of the stirring tank 25. For example, any one of the driving devices 37 and 38 may be arranged on the upper side of the stirring tank 25 and the other may be arranged on the bottom side of the stirring tank 25 so that the flat plate 26 can reciprocate and rotate. The configuration of the aforementioned stirring device 24 driven in the plurality of operating directions is not particularly limited.

### (Configuration Example of Flat Plate in First Embodiment of Stirring body)

As shown in FIG. 4, in the stirring body 19, one flat plate 3D is rotatably supported with respect to the movable member 17. However, the number of flat plates of the stirring body according to the present invention is not limited to one, and for example, two or more flat plates may be connected to each other at the outer peripheral portions or the central portions thereof. The number and configuration of the flat plates of the stirring body according to the present invention are not particularly limited. FIGS. 6 and 7 illustrate embodiments of various configurations of the flat plate of the stirring body according to the present invention. The circular flat plates 3E, 3F, 3G, 3H, 31, 3J, 3K, and 3L included in the stirring bodies 44, 48, 52, 55, and 59 shown in FIGS. 6 and 7 are made of an elastically deformable material and have exactly the same shape as any one of the flat plates 3A, 3B, and 3C shown in FIGS. 1 to 3, that is, are the same. Thus, the detailed description thereof will be omitted here.

As shown in FIGS. 6(A) and 6(B), the stirring body 44 includes four support members 45, a connecting flat plate 46 in which the flat plate 3E is superimposed on the flat plate 3F so that the outer peripheral portions thereof are bonded together, and a movable member 47. The support member 45 is provided around the central portion 4C of the circular connecting flat plate 46 at intervals of 90 degrees and is bonded to the outer peripheral portion of the connecting flat plate 46 so that the outer peripheral portion side of the connecting flat plate 46 is fixed to the support member 45. The movable member 47 passes through a through-hole provided in the central portions 4C and 4D of the flat plates 3E and 3F to rotatably support the central portions 4C and 4D side of the flat plates 3E and 3F between two adjacent convex spherical portions provided at the tip and the intermediate portion of the movable member 47. The movable member 47 reciprocates in the direction of the arrow shown in the drawing and the connecting flat plate 46 having the blade portions 7F and 7G shown in the drawing is elastically deformed reversibly. The flat plate 3E has exactly the same shape as the flat plate 3C having the three-dimensional shape shown in FIG. 3(A), the flat plate 3F has exactly the same shape as the flat plate 3B having the three-dimensional shape shown in FIG. 2(A) of which the dimensions are reduced by 30% at a magnification of 1:1, and the broken-line flat plate 3F shows a deformed form when the movable member 47 is moved the lowermost position.

As shown in FIGS. 6(C) and 6(D), the stirring body 48 includes two support members 49, a circular flat plate 3G, a rectangular movement auxiliary member 50 bonded to the outer peripheral portion of the flat plate 3G, and a movable member 51. The support member 49 is provided around the central portion 4E of the flat plate 3G at intervals of 180 degrees and is bonded to the central portion 4E of the flat plate 3G so that the central portion 4E side of the flat plate 3G is fixed to the support member 49. The movable member 51 passes through the through-holes provided in the central portion 4E of the flat plate 3G and the central portion of the movement auxiliary member 50 to rotatably support the movement auxiliary member 50 between two adjacent convex spherical portions provided at the tip of the movable member 51. In this way, the movable member 51 reciprocates in the direction of the arrow shown in the drawing and the flat plate 3G having the blade portion 7H shown in the drawing is elastically deformed reversibly. The flat plate 3G has exactly the same shape as the flat plate 3C having the three-dimensional shape shown in FIG. 3(A), and the broken-line flat plate 3G shows a deformed form when the movable member 51 is moved to the uppermost position.

As shown in FIGS. 6(E) and 6(F), the stirring body 52 includes four support members 53, a circular flat plate 3H, and a movable member 54. The support member 53 is provided around the central portion 4F of the flat plate 3H at intervals of 90 degrees and bonded to the outer peripheral portion of the flat plate 3H so that the outer peripheral portion side of the flat plate 3H is fixed to the support member 53. The movable member 54 passes through the through-hole provided in the central portion 4F of the flat plate 3H to rotatably support the central portion 4F side of the flat plate 3H between two adjacent convex spherical portions provided in the tip of the movable member 54. In this way, the movable member 54 reciprocates in the direction of the arrow shown in the drawing and the flat plate 3H having the blade portion 71 shown in the drawing is elastically deformed reversibly. The flat plate 3H has exactly the same shape as the flat plate 3C having the three-dimensional shape shown in FIG. 3(A), and the broken-line flat plate 3H shows a deformed form when the movable member 54 is moved to the uppermost position.

As shown in FIGS. 7(A) and 7(B), the stirring body 55 includes a support member 56 having a U-shape, a circular connecting flat plate 57 in which the flat plates 3I and 3J are bonded at the outer peripheral portions thereof, and a movable member 58. The central portion 4H of the movable member 58 and the intermediate portion of the support member 56 are bonded so that the central portion 4H side of the connecting flat plate 57 is fixed to the support member 56. The movable member 58 passes through the through-hole provided in the central portion 4G of the flat plate 31 to rotatably support the central portion 4G side of the flat plate 31 between two adjacent convex spherical portions provided in the tip of the movable member 58. In this way, the movable member 58 reciprocates in the direction of the arrow shown in the drawing and the connecting flat plate 57 having the blade portions 7J and 7K shown in the drawing is elastically deformed reversibly. The flat plates 3I and 3J have exactly the same shape as the flat plate 3C having the three-dimensional shape shown in FIG. 3(A), and the broken-line connecting flat plate 57 shows a deformed form when the movable member 58 is moved to the lowermost position.

As shown in FIGS. 7(C) to 7(E), the stirring body 59 includes four support members 60, a circular connecting flat plate 61 in which the flat plates 3K and 3L are bonded at the central portions thereof, a rectangular movement auxiliary member 62 bonded to the outer peripheral portion of the flat plate 3L, and a movable member 63. The four support members 60 are provided around the central portion 41 of the flat plate 3K at intervals of 90 degrees and bonded to the outer peripheral portion of the flat plate 3K so that the outer peripheral portion side of the flat plate 3K is fixed to the support member 60. The movable member 63 passes through the through-hole provided in the central portion of the movement auxiliary member 62 to rotatably support the movement auxiliary member 62 between two adjacent convex spherical portions provided in the movable member 63. In this way, the movable member 63 reciprocates in the direction of the arrow shown in the drawing and the connecting flat plate 61 having the blade portions 7L and 7M shown in the drawing is elastically deformed reversibly. The flat plates 3K and 3L have exactly the same shape as the flat plate 3C having the three-dimensional shape shown in FIG. 3(A), and the broken-line connecting flat plate 61 shows a deformed form when the movable member 63 is moved to the uppermost position.

In any of the aforementioned flat plates 3E, 3F, 3G, 3H, 31, 3J, 3K, and 3L, the cut is formed so as not to contact the outer peripheral edges of the flat plates 3E, 3F, 3G, 3H, 31, 3J, 3K, and 3L.

According to the aforementioned stirring bodies 44, 48, 52, 55, 59, when the flat plates 3E, 3F, 3G, 3H, 31, 3J, 3K, and 3L made of an elastically deformable material are appropriately combined and the movable members 47, 51, 54, 58, and 63 are moved in the normal direction of the flat plates 3E, 3F, 3G, 3H, 31, 3J, 3K, and 3L, the flat plates 3E, 3F, 3G, 3H, 31, 3J, 3K, and 3L can reversibly form a plurality of deformed forms with different displacement amounts by elastic deformation. Therefore, for example, when the stirring body 44, 48, 52, 55, or 59 of the present embodiment is used in place of the stirring body 19 of the stirring device 15 shown in FIG. 4, the blade portions 7F, 7G, 7H, 71, 7J, 7K, 7L, and 7M perform expansion/contraction movement in the vertical direction, rotational movement in the forward and reverse directions, a spreading movement, and a converging movement and the flat plates 3G and 3H and the connecting flat plates 46, 57, and 61 are elastically deformed. As a result, the flat plates 3G and 3H and the connecting flat plates 46, 57, and 61 behave dynamically so as to repeatedly form different deformed forms reversibly. In this way, it is possible to stir a fluid medium in an extremely complex and unsteady manner with low shear accompanied by a plurality of flows such as a vortex flow, a separation flow, a suction flow, an extrusion flow, a swirling flow, a vertical convection flow, a circulating flow, and a turbulent flow. Moreover, the relative moving distance between the movable members 47, 51, 54, 58, and 63 and the support members 45, 49, 53, 56, and 60 are adjusted, and the flat plates 3G and 3H and the connecting flat plates 46, 57, and 61 are elastically deformed to appropriately form deformed forms with different displacement amounts reversibly. In this way, it is possible to provide a stirring blade that can adjust the flow of a stirring flow and that can be manufactured easily and to provide a stirring device including the stirring blade.

### (Example of Forming Flat Plate in First Embodiment of Stirring body)

As shown in FIG. 1, in the flat plate 3A of the stirring body 1A, the four cuts 2 are formed so as to extend in a spiral form about 540 degrees around the central portion 4A from the outer-peripheral-side ends 5 located inside the outer peripheral edge of the circular flat plate 3A and provided around the central portion 4A of the flat plate 3A at intervals of 90 degrees toward the inner-peripheral-side ends 6A located near the central portion 4A and provided around the central portion 4A at intervals of 90 degrees. However, the method of forming the flat plate of the stirring body according to the present invention is not limited to a method in which both ends of the cut 2 as shown in FIG. 1 are terminated near the inner side of the outer peripheral edge and the central portion 4A of the flat plate 3A. For example, the flat plate and the flat plate auxiliary member in which the cut is penetrated to the outer peripheral edge may be appropriately combined to form a shape the same as or similar to that of the flat plates 3A, 3B, and 3C of the stirring bodies 1A, 1B, and 1C as shown in FIGS. 1 to 3 to obtain the same features and effects as the flat plates 3A, 3B, and 3C. The method of forming the flat plate of the stirring body according to the present invention is not particularly limited. Hereinafter, embodiments of various forming methods of the flat plate of the stirring body according to the present invention will be described with reference to FIG. 8. The three-dimensional flat plates 64, 74, and 85 shown in FIG. 8 are made of an elastically deformable material.

As shown in FIG. 8(A), in the three-dimensional flat plate 64 forming the neck portion, a square through-hole is provided in the central portion 66 of the circular flat plate 65, and four cuts 67 are formed so as to extend in a spiral form about 540 degrees around the central portion 66 from the outer-peripheral-side ends 68 provided around the central portion 66 of the flat plate 65 at intervals of 90 degrees toward the inner-peripheral-side ends 69 provided at square corners of the through-hole edges of the central portion 66. Further, the outer shape of the flat plate auxiliary member 71A has the same shape as the through-hole in the central portion 66 of the flat plate 65, and a circular through-hole is provided in the central portion 70 of the flat plate auxiliary member 71A. In this way, when the central portion 73 of the flat plate 72 in which the flat plate 65 and the flat plate auxiliary member 71A are bonded at the broken-line portion shown in the drawing is elastically deformed so as to appropriately extend in the direction of the arrow shown in the drawing, that is, in the normal direction of the flat plate 72, it is possible to form the three-dimensional flat plate 64 forming the neck portion from the two-dimensional flat plate 72. The flat plate 64 has exactly the same shape as the flat plate 3C of the stirring body 1C shown in FIG. 3(B), that is, the same one, and has the same features and effects as the flat plate 3C of the stirring body 1C.

In the three-dimensional flat plate 74 forming the neck portion as shown in FIG. 8(B), a circular through-hole is provided in the central portion 76 of the flat plate 75, and the four cuts 77 are formed so as to extend in a spiral form about 540 degrees around the central portion 76 from the outer-peripheral-side ends 78 located at the outer peripheral edges of the flat plate 75 provided around the central portion 76 of the flat plate 75 at intervals of 90 degrees toward the inner-peripheral-side ends 79 located near the central portion 76 and provided around the central portion 76 at intervals of 90 degrees. Further, in the flat plate auxiliary member 80 having a ring structure, an inner shape 81 has the same as the outer shape of the flat plate 75 and an outer shape 82 has a circular shape. In this way, when the central portion 84 of the flat plate 83 in which the flat plate 75 and the flat plate auxiliary member 80 are bonded at the broken-line portion shown in the drawing is elastically deformed so as to appropriately extend in the direction of the arrow shown in the drawing, that is, in the normal direction of the flat plate 83, it is possible to form the three-dimensional flat plate 74 forming the neck portion from the two-dimensional flat plate 83. The flat plate 74 has exactly the same shape as the flat plate 3C of the stirring body 1C shown in FIG. 3(B), that is, the same one, and has the same features and effects as the flat plate 3C of the stirring body 1C.

In the three-dimensional flat plate 85 forming the neck portion as shown in FIG. 8(C), a square through-hole is provided in the central portion 87 of the flat plate 86, and four cuts 88 are formed so as to extend in a spiral form about 540 degrees around the central portion 87 from the outer-peripheral-side ends 89 located at the outer peripheral edges of the flat plate 86 provided around the central portion 87 of the flat plate 86 at intervals of 90 degrees toward the inner-peripheral-side ends 90 located near the central portion 87 and provided around the central portion 87 at intervals of 90 degrees. Further, the flat plate auxiliary member 71B has the same outer shape as the through-hole in the central portion 87 of the flat plate 86, a circular through-hole is provided in the central portion of the flat plate auxiliary member 71B, and the flat plate auxiliary member 91 having a ring structure has a circular inner shape 92 and a circular outer shape 93. In this way, when the central portion 95 of the flat plate 94 in which the flat plate 86, the flat plate auxiliary member 71B, and the flat plate auxiliary member 91 are bonded at the broken-line portion shown in the drawing is elastically deformed so as to appropriately extend in the direction of the arrow shown in the drawing, that is, in the normal direction of the flat plate 94, it is possible to form the three-dimensional flat plate 85 forming the neck portion from the two-dimensional flat plate 94. The flat plate auxiliary member 71B is the same as the flat plate auxiliary member 71A shown in FIG. 8(A), the flat plate 85 is exactly the same as the flat plate 3C of the stirring body 1C shown in FIG. 3(B) except for the outer peripheral portion, and the flat plate 85 has substantially the same features and effects as the flat plate 3C of the stirring body 1C.

According to the aforementioned flat plates 64, 74, and 85 of the stirring body, when the flat plates 65, 75, and 86 and the flat plate auxiliary members 71A, 71B, 80, and 91 are appropriately combined and elastically deformed by a predetermined physical means to form a deformed form appropriately, it is possible to form a deformed form the same as or similar to that of the flat plates 3A, 3B, and 3C as shown in FIGS. 1 to 3. Thus, the flat plates 64, 74, and 85 can provide features and effects the same as or similar to those of the flat plates 3A, 3B, and 3C of the stirring bodies 1A, 1B, and 1C shown in FIGS. 1 to 3.

### (Example of Cut in Flat Plate in First Embodiment of Stirring body)

In the flat plate 3A as shown in FIG. 1, four spiral cuts 2 are formed in the flat plate 3A, but the number and shape of the cuts 2 are not particularly limited. FIG. 9 illustrates embodiments of various cuts of the flat plate of the stirring body according to the present invention. The flat plates 96, 101, 106, and 113 shown in FIG. 9 are made of an elastically deformable material.

In the flat plate 96 shown in FIG. 9(A), the spiral cut is modified to two slit-shaped cutouts 97. The two cutouts 97 are formed so as to extend in a spiral form about 360 degrees around the central portion 98 from two outer-peripheral-side ends 99 located inside the outer peripheral edges of a circular flat plate 96 having a wavy shape formed by connecting combinations of circular arcs and straight lines in series and provided around the central portion 98 at intervals of 180 degrees toward two inner-peripheral-side ends 100 located near the central portion 98 having a circular through-hole and provided around the central portion 98 at intervals of 180 degrees.

In the flat plate 101 shown in FIG. 9(B), four cuts 102 formed by connecting combinations of straight lines in series are formed so as to extend in a rectangular spiral form about 360 degrees around the central portion 103 from four outer-peripheral-side ends 104 located inside the vicinity of the corners of the outer peripheral edges of the square flat plate 101 and provided around the central portion 103 at intervals of 90 degrees toward four inner-peripheral-side ends 105 located near the central portion 103 having a circular through-hole and provided around the central portion 103 at intervals of 90 degrees.

In the flat plate 106 shown in FIG. 9(C), four cuts 107 formed by connecting combinations of circular arcs in series are formed so as to extend to the intermediate portion 111 in a spiral form about 360 degrees around the central portion 108 from four outer-peripheral-side ends 109 located inside the vicinity of the outer peripheral edges of the regular hexagonal flat plate 106 toward four inner-peripheral-side ends 110 located near the central portion 108 having a circular through-hole and provided around the central portion 108 at intervals of 90 degrees and are formed so as to extend in a spiral form about 360 degrees around the central portion 108 from the intermediate portion 111 toward the inner-peripheral-side ends 110 in the direction opposite to the spiral direction of the cuts 107 extending from the outer-peripheral-side ends 108 to the intermediate portion 111. Further, the outer peripheral edge of the flat plate 106 is bent toward the central portion 108 side with the broken-line portion shown in the drawing as the base point, whereby six flaps 112 are formed on the outer peripheral portion of the flat plate 106.

In the flat plate 113 shown in FIG. 9(D), two cuts 114 formed by connecting combinations of circular arcs in series are formed so as to extend in a spiral form about 360 degrees around the central portion 115 from two outer-peripheral-side ends 116 located inside the vicinity of the outer peripheral edges of an elliptical flat plate 113 and provided around the central portion 115 at intervals of 180 degrees toward two inner-peripheral-side ends 117 located near the central portion 115 having a circular through-hole and provided around the central portion 115 at intervals of 180 degrees, and a plurality of circular through-holes 118 are arranged.

According to the aforementioned flat plates 96, 101, 106, and 113 of the stirring body, when the spiral cuts formed in the flat plate 96 are modified as two slit-shaped cutouts 97, the cutouts function as a flat plate displacement amount adjustment structure that adjusts a blade width of the blade portion by elastic deformation, an expansion/contraction movement width, a rotational movement width, and a spreading/converging movement width according to the shape of the cutout 97 to form a plurality of deformed forms with different displacement amounts reversibly. The flat plate 96 having a wavy shape can generate a plurality of flows accompanied by a plurality of small vortex flows. Since the cuts 102 formed in the square flat plate 101 are formed by connecting combinations of straight lines in series, it is possible to form a spiral cut shape that follows the shape of a polygonal flat plate. Due to the flaps 112 and the cuts 107 formed by being connected in series by a combination of right-handed winding and left-handed winding provided in the flat plate 106, it is possible to generate a plurality of flows accompanied by a plurality of large vortex flows. Furthermore, due to the plurality of through-holes 118 arranged in the elliptical flat plate 113, it is possible to generate a plurality of flows accompanied by a plurality of large and small vortex flows and generate a circulating flow in a highly viscous liquid. In this way, for example, when the flat plates 96, 101, 106, and 113 are used in place of the flat plate 3D of the stirring device 15 shown in FIG. 4, since a plurality of different deformed forms can be formed reversibly by elastic deformation to create dynamic behavior, it is possible to generate a circulating flow in a fluid medium having high and low viscosity. Moreover, it is possible to stir a fluid medium in the liquid in an extremely complex and unsteady manner with low shear accompanied by various flows such as a vortex flow, a separation flow, a suction flow, an extrusion flow, a swirling flow, a vertical convection flow, a circulating flow, and a turbulent flow. Thus, it is possible to provide a stirring blade that can adjust the displacement amounts of the flat plates 96, 101, 106, and 113 by elastic deformation to appropriately form deformed forms reversibly to adjust the flow of a stirring flow and that can be manufactured easily and to provide a stirring device including this stirring blade.

### (Second Embodiment of Stirring body)

In the stirring bodies 201A, 201B, 201C, and 201D of the second embodiment shown in FIG. 10, the movable members that support the central portion and/or the outer peripheral portion of each of the flat plates 203A, 203B, 203C, and 203D made of an elastically deformable material are arranged in a guide member 209 so as to be separated from each other. A plurality of Archimedean spiral (that is, spiral) cuts 2 extending outward from the vicinity of the central portions of the flat plates 203A, 203B, 203C, and 203D so as not to contact the outer peripheral edges of the flat plates 203A, 203B, 203C, and 203D are formed at equal angular intervals around the central portions of the flat plates 203A, 203B, 203C, and 203D. Thus, a plurality of spiral blade portions 206A, 206B, 206C, and 206D formed by the cuts 202 are arranged at predetermined angular intervals in the circumferential direction around the central portions of the flat plates 203A, 203B, 203C, and 203D. The flat plates 203B, 203C, and 203D of the stirring bodies 201B, 201C, and 201D shown in FIGS. 11 to 13 show three different deformed forms having a multiple-spiral structure formed reversibly by elastically deforming the flat plate 203A of the stirring body 201A shown in FIG. 10. The stirring bodies 201B, 201C, and 201D of the present embodiment shown in FIGS. 11 to 13 are the same as the stirring body 201A of the present embodiment shown in FIG. 10 except for the flat plates 203B, 203C, and 203D that are elastically deformed so as to form different deformed forms.

As shown in FIG. 10(A), in the stirring body 201A, four cuts 2 are formed so as to extend in a spiral form about 540 degrees around the central portion of the flat plate 203A from the outer-peripheral-side ends 204 located inside the outer peripheral edges of the circular flat plate 203A and provided around the central portion of the flat plate 203A at intervals of 90 degrees toward the inner-peripheral-side ends 205A located near the central portion of the flat plate 203A and provided around the central portion at intervals of 90 degrees. That is, the four blade portions 206A are arranged at positions rotated by 90 degrees in the circumferential direction around the central portion of the flat plate 203A with respect to the adjacent blade portions 206A, and are formed so as to extend in a spiral form about 540 degrees around the central portion of the flat plate 203A. The outer-peripheral-side end 204 of the cut 202 is preferably formed so as to be separated from the outer peripheral edge toward the central portion of the flat plate 203A by a range of 5 to 10% of the diameter of the flat plate 203A. Moreover, the inner-peripheral-side end 5A is preferably formed so as to be separated from the central portion of the flat plate 203A toward the outer peripheral edge by a range of 5 to 10% of the diameter of the flat plate 203A.

As shown in FIGS. 10(B) and 10(C), in the stirring body 201A, the movable member 207A is bonded to the central portion on the upper surface side of the flat plate 203A, the rectangular movable member 208A terminating in the vicinity of the outer peripheral edge on the bottom surface side of the flat plate 203A is bonded to the outer peripheral portion of the flat plate 203A, and the movable members 207A and 208A are separated from each other and slidably and rotatably attached to the guide member 209 passing through a through-hole provided in the central portion of the flat plate 203A. The movable members 207A and 208A are locked to the guide member 209 by lock mechanisms 210 and 211 such as hexagon socket set screws provided in the movable members 207A and 208A so as not to be rotatable and movable in the axial direction. In this way, the blade portion 206A formed by the four cuts 2 is closed to form the two-dimensional flat plate 203A.

As shown in FIGS. 11(A) and 11(B), in the stirring body 201B, the central portion side of the flat plate 203A shown in FIG. 10(B) is moved (that is, raised) to the extension position 213 shown in FIG. 11(A) in the direction of the arrow 212, that is, in the normal direction of the flat plate 203A, and the central portion of the flat plate 203A shown in FIGS. 10(A) and 10(B) is moved (that is, raised) so that the blade portion 206A is elastically deformed so as to extend while being twisted. The movable members 207A and 208A are separated from each other and locked to the guide member 209 by the lock mechanisms 210 and 211 provided in the movable members 207A and 208A so as not to be rotatable and movable in the axial direction. In this way, the three-dimensional flat plate 203B having the truncated cone-shaped blade portion 206B as shown in FIGS. 11(A) and 11(B) is formed.

Further, as shown in FIG. 11(C), in the stirring body 201B, the central portion side of the flat plate 203A shown in FIG. 10(B) is raised to the extension position 213 shown in FIG. 11(A) in the direction of the arrow 212, and the blade portion 206A shown in FIG. 10(A) is elastically deformed so as to extend while being twisted so as to rotate in the direction of the cut 202 facing the central portion of the flat plate 203A, that is, in the right-handed winding direction. In this way, the inner-peripheral-side end 205A shown in FIG. 10(A) rotates around the central portion of the flat plate 203A about 20 degrees in the right-handed winding direction and rotationally moves to the position of the inner-peripheral-side end 205B shown in FIG. 11(C). In this way, the three-dimensional flat plate 203B having the truncated cone-shaped blade portion 206B is formed as shown in FIGS. 11(A) and 11(B).

As shown in FIGS. 12(A) and 12(B), in the stirring body 201C, the central portion side of the flat plate 203B shown in FIG. 11(A) is further raised from the extension position 213 to the extension position 214 shown in FIG. 12(A), the central portion of the flat plate 203B shown in FIGS. 11(A) and 11(B) is further raised, and the blade portion 206B is elastically deformed while being twisted accompanied by extension movement, outward spreading (hereinafter referred to as "spreading movement"), and convergence toward the center of the flat plate 203B (hereinafter referred to as "converging movement"). Moreover, the movable members 207A and 208A are locked by the lock mechanisms 210 and 211 provided in the movable members 207A and 208A so as not to be rotatable and movable in the axial direction. In this way, as shown in FIGS. 12(A) and 12(B), a three-dimensional flat plate 203C having a blade portion 206C that forms a neck portion 215A having a neck shape and creates a singular point 216 at which the plurality of blade portions 206C converges according to the strength of pressure is formed.

Further, as shown in FIG. 12(C), in the stirring body 201C, the central portion side of the flat plate 203B shown in FIG. 11(A) is further raised from the extension position 213 to the extension position 214 shown in FIG. 12(A), and the blade portion 206B shown in FIG. 11(C) is elastically deformed while being twisted so as to further rotate in the right-handed winding direction accompanied by extension movement, spreading movement, and converging movement. In this way, the inner-peripheral-side end 205A shown in FIG. 10(A) is rotated about 45 degrees in the right-handed winding direction around the central portion of the flat plate 203A as the base axis and is rotationally moved to the position of the inner-peripheral-side end 205C shown in FIG. 12(C). In this way, a three-dimensional flat plate 203C having a blade portion 206C forming a neck portion 215Ais formed as shown in FIGS. 12(A) and 12(B).

Further, as shown in FIGS. 13(A) and 13(B), in the stirring body 201D, the central portion side of the flat plate 203C shown in FIG. 12(A) is rotated about 180 degrees around the central portion of the flat plate 203C as the base axis in the twisting direction 217 shown in FIG. 13(A) from the extension position 214, and the blade portion 206C is rotationally moved while being twisted and the blade portion 206C around the neck portion 215A is elastically deformed accompanied by reverse movement. Moreover, the movable members 207A and 208A are locked to the guide member 9 by the lock mechanisms 210 and 211 provided in the movable members 207A and 208A so as not to be rotatable and movable in the axial direction. In this way, a three-dimensional flat plate 203D having a plurality of wavy blade portions 206D of which the front and back surfaces are exposed when seen from one direction is formed as shown in FIGS. 13(A) and 13(B).

As shown in FIG. 13(C), in the stirring body 201D, the central portion side of the flat plate 203C shown in FIG. 12(A) is twisted (that is, rotated) about 180 degrees around the central portion of the flat plate 203C as the base axis in the direction opposite to the direction of the cut 202 facing the central portion of the flat plate 203C, that is, the left-handed twisting direction 217 shown in FIG. 13(A). Moreover, the blade portion 206C shown in FIG. 12(C) is elastically deformed while being twisted in the left-handed winding direction accompanied by rotational movement and reverse movement. In this way, the inner-peripheral-side end 205C shown in FIG. 12(C) is rotated about 180 degrees around the central portion of the flat plate 203C as the base axis in the direction opposite to the direction of the cut 202 facing the central portion of the flat plate 203C, that is, in the left-handed winding direction and is rotationally moved to the position of the inner-peripheral-side end 205D shown in FIG. 13(C). In this way, a three-dimensional flat plate 203D having a plurality of wavy blade portions 206D of which the front and back surfaces are exposed when seen from one direction is formed as shown in FIGS. 13(A) and 13(B).

According to the aforementioned stirring bodies 201A, 201B, 201C, 201D, the movable member 207A is bonded to the central portion on the upper surface side of each of the flat plates 203A, 203B, 203C, 203D, the rectangular movable member 208A terminating in the vicinity of the outer peripheral edge on the bottom surface side of each of the flat plates 203A, 203B, 203C, and 203D is bonded to the outer peripheral portion of each of the flat plates 203A, 203B, 203C, and 203D, and the movable members 207A and 208A are separated from each other and slidably and rotatably attached to the guide member 209 passing through the through-hole provided in the central portion of each of the flat plates 203A, 203B, 203C, and 203D. The movable members 207A and 208A are locked to the guide member 209 by lock mechanisms 210 and 211 such as hexagon socket set screws provided in the movable members 207A and 208A so as not to be rotatable and movable in the axial direction. In this way, the flat plates 203A, 203B, 203C, and 203D made of an elastically deformable material can appropriately form a plurality of deformed forms with different displacement amounts reversibly by elastic deformation. The flat plates 203A, 203B, 203C, and 203D can form a deformed form from the two-dimensional flat plate 203A having the blade portion 206A closed by the four spiral cuts 2 to the three-dimensional flat plate 203B having the truncated cone-shaped blade portion 206B in which the blade portion 206A is elastically deformed while being twisted accompanied by a plurality of operation movements including extension movement. Moreover, the flat plate can form a deformed form from the state of the three-dimensional flat plate 203B to the three-dimensional flat plate 203C having the blade portion 206C that forms the neck portion 215A and creates the singular point 216 according to the strength of pressure in which the blade portion 206B is further elastically deformed while being twisted accompanied by a plurality of operation movements including extension movement, spreading movement, and converging movement toward the center. Furthermore, the flat plate can form a deformed form from the state of the three-dimensional flat plate 203C to the complex three-dimensional flat plate 203D having a plurality of wavy blade portions 206D of which the front and back surfaces are exposed when seen from one direction in which the central portion side of the flat plate 203C is rotated about 180 degrees in the twisting direction 217, and the blade portion 206C around the neck portion 215A is elastically deformed while being twisted accompanied by a plurality of operation movements including rotational movement and reverse movement. In this way, the cut 202 functions as an expansion/contraction movement structure, a rotational movement structure, a convergence movement structure, and a twist/reverse movement structure of the blade portions 206A, 206B, 206C, and 206D. The cut 202 also functions as a flat plate displacement amount adjustment structure that adjusts the expansion/contraction movement width and the rotational movement width of the blade portions 206A, 206B, 206C, and 206D according to the length of the cut 202 extending around the central portion of the flat plates 203A, 203B, 203C, and 203D, that is, the angle extending around the central portion of the flat plates 203A, 203B, 203C, and 203D (hereinafter referred to as a "circumferential angle") to reversibly form a plurality of deformed forms with different displacement amounts. The movable member 207A functions as a shape deformation structure of the flat plates 203A, 203B, 203C, and 203D forming the deformed form. Since the flat plates 203A, 203B, 203C, and 203D have a circular outer shape without a tip wing, the flat plate functions as a container damage protection structure that prevents the inside of a container such as a stirring tank for stirring a stirring substance from being damaged. Therefore, for example, when an elastically deformable material is used as the material of the flat plates 203A, 203B, 203C, and 203D of the aforementioned stirring bodies 201A, 201B, 201C, and 201D of the present embodiment, and the movable members 207A and 208A are separated from each other and locked to the guide member 208 so as not to be rotatable and movable in the axial direction by the lock mechanisms 210 and 211 provided in the movable members 207A and 208A bonded to the flat plates 203A, 203B, 203C, and 203D, the flat plates 203A, 203B, 203C, and 203D can appropriately form a plurality of deformed forms with different displacement amounts by elastic deformation. When the guide member 9 is rotated by a predetermined physical means, it is possible to generate a complex vertical reflux in the liquid accompanied by a plurality of flows such as a swirling flow, a vertical convection flow, and a suction flow and to satisfactorily stir a fluid medium without any mixing unevenness. When the guide member 209 is reciprocated in the vertical direction by a predetermined physical means, it is possible to stir a fluid medium in an extremely complex and unsteady manner in the liquid accompanied by a plurality of flows such as a vortex flow, a separation flow, a vertical convection flow, a circulating flow, and a turbulent flow. In this way, it is further possible to provide a low-cost stirring blade that has a simple configuration, and prevents damages in the stirring tank, and that can be manufactured easily, the stirring blade being capable of adjusting the flow of a stirring flow by adjusting the arrangement positions of the movable members 207A and 208A in relation to the guide member 209, adjusting the displacement amounts of the flat plates 203A, 203B, 203C, and 203D by elastic deformation, and appropriately and reversibly forming a plurality of deformed forms with different displacement amounts by elastic deformation of the flat plate.

The position of the guide member 209 that supports the flat plates 203A, 203B, 203C, and 203D of the aforementioned stirring bodies 201A, 201B, 201C, and 201D is not limited to the central portion of each of the flat plates 203A, 203B, 203C, and 203D. Although not shown in the drawing, the position of the guide member 209 may be the outer peripheral portion or the central portion and the outer peripheral portion of each of the flat plates 203A, 203B, 203C, and 203D. The position of the guide member 209 of each of the aforementioned stirring bodies 201A, 201B, 201C, and 201D is not particularly limited.

### (Third Embodiment of Stirring body)

The second embodiment of the stirring body according to the present invention includes the flat plate in which a plurality of spiral blade portions extending from a central portion is formed by a spiral cut that does not contact an outer peripheral portion and which is made of an elastically deformable material, the guide member, and the movable member connected to one of the central portion and the outer peripheral portion of the flat plate. The movable member is rotatable and movable along the guide member and causes the guide member to rotate. Thus, the stirring body can be used as a stirring blade of a stirring device capable of satisfactorily stirring a fluid medium without any mixing unevenness accompanied by a complex vertical reflux. Hereinafter, a third embodiment of the stirring device including the stirring body according to the present invention will be described with reference to FIG. 14. A flat plate 203E of a stirring body 221 provided in the stirring device 218 of the present embodiment shown in FIG. 14 is made of an elastically deformable material and has exactly the same shape as the flat plate 203C shown in FIG. 12, that is, the same one is used. The stirring body 221 shown in FIG. 14 has the same configuration as the stirring body 201C shown in FIG. 12. Thus, the detailed description thereof will be omitted here.

As shown in FIG. 14, the stirring device 218 includes a stirring tank 219A containing a liquid and having a volume of 300 mL, a stirring body 221 including a circular flat plate 203E, a guide member 220A, and movable members 207B and 208B, a top plate portion 222A, a rotating driving device 223A provided with a brushless motor, and a bearing 224A. The guide member 220A serving as the driving shaft of the driving device 233A vertically suspended in the stirring tank 219A is rotatably connected to the bearing 224A arranged in the center-side opening of the touch operation 222A. Thus, the inside of the stirring tank 219A is airtightly provided.

As shown in FIGS. 14(A) to 14(C), in the stirring device 218, the movable member 27B is bonded to the central portion on the upper surface side of the flat plate 23E, and the rectangular movable member 28B is bonded to the outer peripheral portion on the bottom surface side of the flat plate 23E. As shown in FIG. 14(A), the movable members 207B and 208B are separated from each other and slidably and rotatably attached to the guide member 220A passing through the through-hole provided in the central portion of the flat plate 203E. The movable members 207B and 208B are locked to the guide member 220A so as not to be rotatable and movable in the axial direction by the lock mechanisms 225A and 226A such as hexagon socket set screws provided in the movable members 207B and 208B. In this way, a three-dimensional flat plate 203E having four blade portions 206F forming the neck portion 215B is formed.

As shown in FIG. 14(D), in the stirring device 218, the driving device 223A is driven by a predetermined physical means, so that the guide member 220A is rotated in the direction of the arrow shown in the drawing and the three-dimensional flat plate 203E rotates. Thus, a complex vertical reflux is generated in the liquid accompanied by a plurality of flows such as a swirling flow, a vertical convection flow, ad a suction flow. That is, FIG. 14(D) schematically shows the movement of the stirring flow in the stirring tank 291A by the stirring device 218 shown in FIG. 14(A).

According to the stirring device 218 including the aforementioned stirring body 221 described above, the movable members 207B and 208B bonded to the circular flat plate 203E made of an elastically deformable material are separated from each other and slidably and rotatably attached to the guide member 220A. The movable members 207B and 208B are locked to the guide member 220A so as not to be rotatable and movable in the axial direction by the lock mechanisms 225A and 226A such as hexagon socket set screws provided in the movable members 207B and 208B. When the driving device 223A provided with a brushless motor is driven by a predetermined physical means to cause the guide member 220A to rotate in the direction of the arrow shown in FIG. 14(D), the three-dimensional flat plate 203E having the blade portion 206F forming the neck portion 215B by elastic deformation can be rotated in the stirring tank 219A. In this way, it is possible to generate a complex vertical reflux in the liquid in the stirring tank 219A accompanied by a suction flow due to a suction force generated from the bottom surface side near the outer peripheral portion of the flat plate 203E toward the central portion of the flat plate 203E, a suction flow due to a suction force generated from the vicinity of the neck portion 215B toward the central portion of the flat plate 203E, a swirling flow, and a vertical convection flow, and satisfactorily stir a fluid medium without any mixing unevenness.

### (Fourth Embodiment of Stirring Device)

The second embodiment of the stirring body according to the present invention includes the flat plate in which a plurality of spiral blade portions extending from a central portion is formed by a spiral cut that does not contact an outer peripheral portion and which is made of an elastically deformable material, the guide member, and the movable member connected to one of the central portion and the outer peripheral portion of the flat plate. The movable member is rotatable and movable along the guide member and causes the guide member to rotate. Thus, the stirring body can be used as a stirring blade of a stirring device capable of satisfactorily stirring a fluid medium without any mixing unevenness accompanied by a complex vertical reflux and adjusting the flow of a stirring flow. Hereinafter, a fourth embodiment of the stirring device including the stirring body according to the present invention will be described with reference to FIGS. 15 and 16. The flat plates 3F and 3G of the stirring bodies 228 and 230 provided in the stirring devices 227 and 229 of the present embodiment of FIGS. 15 and 16 are made of an elastically deformable material, and are two different deformed forms formed by the flat plate 203E of the stirring body 221 provided in the stirring device 218 shown in FIG. 14. The stirring devices 227 and 229 shown in FIGS. 15 and 16 are exactly the same as the stirring device 218 shown in FIG. 14 except for the flat plates 203F and 203G, that is, the same ones are used. Further, the stirring body 228 shown in FIG. 15 has exactly the same shape as the stirring body 201B shown in FIG. 11, and the stirring body 230 shown in FIG. 16 has exactly the same shape as the stirring body 1D shown in FIG. 13. The stirring bodies 228 and 230 shown in FIGS. 15 and 16 are the same as the stirring bodies 201B and 201D shown in FIGS. 11 and 13. Thus, the detailed description thereof will be omitted here.

As shown in FIG. 15, the stirring device 227 includes a stirring tank 219B containing a liquid and having a volume of 300 mL, a stirring body 228 including a circular flat plate 203F, a guide member 220B, and movable members 207C and 208C, a top plate portion 222B, a rotating driving device 223B provided with a brushless motor, and a bearing 224B. The guide member 220B vertically suspended in the stirring tank 219B is rotatably connected to the bearing 224B disposed in the center-side opening of the top plate portion 222B. Thus, the inside of the stirring tank 219B is airtightly provided.

As shown in FIGS. 15(A) and 15(B), in the stirring device 227, the movable member 207C is bonded to the central portion on the upper surface side of the flat plate 203F, and the rectangular movable member 208C is bonded to the outer peripheral portion on the bottom surface side of the flat plate 203F. As shown in FIG. 15(A), the movable members 207C and 208C are separated from each other and slidably and rotatably attached to the guide member 220B passing through the through-hole provided in the central portion of the flat plate 203F. The movable members 207C and 208C are locked to the guide member 220B so as not to be rotatable and movable in the axial direction by the lock mechanisms 225B and 226B such as hexagon socket set screws provided in the movable members 207C and 208C. In this way, a three-dimensional flat plate 203F having the truncated cone-shaped blade portion 206G is formed. FIG. 15(A) shows a state in which the driving device 223B is driven by a predetermined physical means, so that the guide member 220B is rotated in the direction of the arrow shown in the drawing and the three-dimensional flat plate 203F is rotated whereby a complex vertical reflux is generated in the liquid accompanied by a plurality of flows such as a swirling flow, a vertical convection flow, and a suction flow. That is, FIG. 15(A) schematically shows the movement of the stirring flow in the stirring tank 219B by the stirring device 227.

According to the stirring device 227 including the stirring body 228 described above, the movable members 207C and 208C bonded to the circular flat plate 203F made of an elastically deformable material are separated from each other and slidably and rotatably attached to the guide member 220B. The movable members 207C and 208C are locked to the guide member 220B so as not to be rotatable and movable in the axial direction by the lock mechanism 225B and 226B such as hexagon socket set screws provided in the movable members 207C and 208C. When the driving device 223B provided with a brushless motor is driven by a predetermined physical means so that the guide member 220B is rotated in the direction of the arrow shown in FIG. 15(A), the three-dimensional flat plate 203F having the truncated cone-shaped blade portion 206G can be rotated in the stirring tank 219B by elastic deformation. In this way, it is possible to generate a complex vertical reflux in the liquid in the stirring tank 219B accompanied by a plurality of flows such as a suction flow based on a suction force generated from the bottom surface side near the outer peripheral portion of the flat plate 203F toward the central portion of the flat plate 203F, a swirling flow, and a vertical convection flow and to satisfactorily stir a fluid medium without any mixing unevenness.

As shown in FIG. 16, the stirring device 229 includes a stirring tank 219C containing a liquid and having a volume of 300 mL, a stirring body 230 including a circular flat plate 203G, a guide member 220C, and movable members 207D and 208D, a top plate portion 222C, a rotating driving device 223C provided with a brushless motor, and a bearing 224C. The guide member 220C vertically suspended in the stirring tank 219C is rotatably connected to the bearing 224C disposed in the center-side opening of the top plate portion 222C. Thus, the inside of the stirring tank 219C is airtightly provided.

As shown in FIGS. 16(A) and 16(B), in the stirring device 229, the movable member 207D is bonded to the central portion on the upper surface side of the flat plate 203G, and the rectangular movable member 208D is bonded to the outer peripheral portion on the bottom surface side of the flat plate 203G. As shown in FIG. 16(A), the movable members 207D and 208D are separated from each other and slidably and rotatably attached to the guide member 220C passing through the through-hole provided in the central portion of the flat plate 203G. The movable members 207D and 208D are locked to the guide member 220C so as not to be rotatable and movable in the axial direction by the lock mechanisms 225C and 226C such as hexagon socket set screws provided in the movable members 207D and 208D. In this way, a three-dimensional flat plate 203G having a plurality of wavy blade portions 206H of which the front and back surfaces are exposed when seen from one direction is formed. FIG. 16(A) shows a state in which the driving device 223C is driven by a predetermined physical means, so that the guide member 220C is rotated in the direction of the arrow shown in the drawing and the three-dimensional flat plate 203G is rotated whereby a complex vertical reflux is generated in the liquid accompanied by a plurality of flows such as a swirling flow, a vertical convection flow, and a suction flow. That is, FIG. 16(A) schematically shows the movement of the stirring flow in the stirring tank 219C by the stirring device 229.

According to the stirring device 29 including the aforementioned stirring body 230, the movable members 207D and 208D bonded to the circular flat plate 203G made of an elastically deformable material are separated from each other and slidably and rotatably attached to the guide member 220C. The movable members 720D and 208D are appropriately locked to the guide member 220C so as not to be rotatable and movable in the axial direction by the lock mechanism 225C and 226C such as hexagon socket set screws provided in the movable members 207D and 208D. When the driving device 223C provided with a brushless motor is driven by a predetermined physical means so that the guide member 220C is rotated in the direction of the arrow shown in FIG. 16(A), the three-dimensional flat plate 203G having the plurality of wavy blade portions 206H of which the front and back surfaces are exposed when seen from one direction can be rotated in the stirring tank 219C by elastic deformation. In this way, it is possible to generate a complex vertical reflux in the liquid in the stirring tank 219B accompanied by a plurality of flows such as a suction flow based on a suction force generated from the bottom surface side near the outer peripheral portion of the flat plate 203G toward the central portion of the flat plate 203G, a suction flow based on a suction force generated from the periphery of a twisted portion in which the front and back surfaces of the blade portion 206H are inverted and exposed toward the central portion of the flat plate 203G, a swirling flow, and a vertical convection flow and to satisfactorily stir a fluid medium without any mixing unevenness.

Therefore, according to the stirring devices 218, 227, and 229 of the embodiment including the stirring bodies 221, 228, and 230 shown in FIGS. 14 to 16, the movable members 207B, 207C, 207D, 208B, 208C, and 208D bonded to the flat plates 203E, 203F, and 203G are separated from each other and slidably and rotatably attached to the guide members 220A, 220B, and 220C. The movable members 207B, 207C, 207D, 208B, 208C, and 208D are locked to the guide members 220A, 220B, and 220C so as not to be rotatable and movable in the axial direction by the lock mechanisms 225A, 225B, 225C, 226A, 226B, and 226C such as hexagon socket set screws provided in the movable members 207B, 207C, 207D, 208B, 208C, and 208D. When the driving devices 223A, 223B, and 223C provided with a brushless motor are driven by a predetermined physical means so that the guide members 220A, 220B, and 220C are rotated, the flat plates 203E, 203F, and 203G are elastically deformed. In this way, it is possible to appropriately and reversibly form a plurality of different deformed forms including the three-dimensional flat plate 203F having the truncated cone-shaped blade portion 206G, the three-dimensional flat plate 203E having the blade portion 206F forming the neck portion 215B, and the three-dimensional flat plate 203G having a plurality of wavy blade portions 206H of which the front and back surfaces are exposed when seen from one direction so that the flat plate can rotate in the stirring tanks 219A, 219B, and 219C. In this way, it is possible to generate a complex vertical reflux in the liquid in the stirring tanks 219A, 219B, and 219C accompanied by a plurality of flows such as a swirling flow, a vertical convection flow, and a suction flow and to satisfactorily stir a fluid medium without any mixing unevenness. In this way, the stirring device functions as a stirring device including a stirring blade capable of adjusting the flow of a stirring flow by adjusting the arrangement positions of the movable members 207B, 207C, 207D, 208B, 208C, and 208D in relation to the guide members 220A, 220B, and 220C to appropriately and reversibly forming a plurality of deformed forms with different displacement amounts by elastic deformation of the flat plates 203E, 203F, and 203G. Therefore, for example, when a fluid medium is stirred using the stirring devices 218, 227, and 229 including the stirring bodies 221, 228 and 230, it is possible to generate a complex vertical reflux in the liquid accompanied by a swirling flow, a vertical convection flow, and a suction flow as compared with the conventional stirring device that generates a vertical reflux and to satisfactorily stir a fluid medium without any mixing unevenness. In this way, it is possible to provide a stirring device including a low-cost stirring blade that has a simple configuration and can be manufactured easily, the stirring blade being capable of adjusting the flow of a stirring flow by adjusting the arrangement positions of the movable member in relation to the guide member to appropriately and reversibly form a plurality of deformed forms with different displacement amounts by elastic deformation of the flat plate.

### (Fifth Embodiment of Stirring Device)

The second embodiment of the stirring body according to the present invention includes the flat plate in which a plurality of spiral blade portions extending from a central portion is formed by a spiral cut that does not contact an outer peripheral portion and which is made of an elastically deformable material, the guide member, and the movable member connected to one of the central portion and the outer peripheral portion of the flat plate. The movable member is rotatable and movable along the guide member and causes the guide member to reciprocate and rotate. Thus, the stirring body can be used as a stirring blade of a stirring device capable of stirring a fluid medium in an extremely complex and unsteady manner and adjusting the flow of a stirring flow. Hereinafter, a fifth embodiment of a stirring device including the stirring body according to the present invention will be described with reference to FIG. 17. A flat plate 233A of a stirring body 237A provided in the stirring body 231 of the present embodiment shown in FIG. 17 is made of an elastically deformable material.

As shown in FIG. 17, the stirring device 231 includes a stirring tank 232 containing a liquid and having a volume of 300 mL, a stirring body 237A including a circular flat plate 233A, movable members 234A and 235A, and a guide member 236A, a top plate portion 238, a driving device 239 provided with a brushless motor and having a controller capable of adjusting the reciprocating rotation distance which reciprocates and rotates, a bearing 240, and an auxiliary member 241A. The guide member 236A which is the driving shaft of the driving device 239 vertically suspended in the tank 232, is rotatably connected to the bearing 240 arranged in the center-side opening of the top plate portion 238. Thus, the inside of the stirring tank 232 is airtightly provided. The three-dimensional flat plate 233A having a plurality of wavy blade portions of which the front and back surfaces are exposed when seen from one direction has exactly the same shape as the flat plate 203D having the three-dimensional shape shown in FIG. 13. The three-dimensional flat plate 233A forming the broken-line neck portion has exactly the same shape as the flat plate 203C having the three-dimensional shape shown in FIG. 12. When the driving device 239 of the stirring device 231 of the present embodiment reciprocates and rotates in the left-right direction, the flat plate 233A of the stirring body 237A provided in the stirring device 231 is elastically deformed to exactly the same shape as the flat plates 203C and 203D shown in FIGS. 12 and 13 to dynamically behave so as to repeatedly form different deformed forms reversibly.

As shown in FIGS. 17(A) and 17(B), in the stirring body 231, two movable members 234A are provided around the central portion of the flat plate 233A at intervals of 180 degrees and bonded to the outer peripheral portion of the flat plate 233A. The movable member 235A is bonded to the central portion of the flat plate 233A and the top plate portion 238 and the auxiliary member 241A are bonded together. As shown in FIG. 17(A), the movable members 234A and 235A are slidably and rotatably attached to the auxiliary member 241A and the guide member 236A, respectively. The movable members 234A and 235A are locked to the auxiliary member 241A and the guide member 236A, respectively, so as not to be rotatable and movable in the axial direction by the lock mechanisms 242A and 243A such as hexagon socket set screws provided in the movable members 234A and 235A, and the outer peripheral portion side of the flat plate 233A is fixed. When the guide member 236A, which is the driving shaft of the driving device 239, is reciprocated and rotated by a predetermined physical means within the range of 180 degrees in the left-right direction, the three-dimensional flat plate 233A is elastically deformed so that the central portion side of the flat plate 233A is rotated about 180 degrees in the direction of the cut facing the central portion, that is, in the right-handed winding direction. Moreover, the three-dimensional flat plate 233A is elastically deformed to the three-dimensional flat plate 233A having a plurality of wavy blade portions of which the front and back surfaces are exposed when seen from one direction and the three-dimensional flat plate 233A forming the broken-line neck portion shown in the drawing, accompanied by a plurality of operations including a rotational movement and reverse movement while the blade portion being twisted in the right-handed winding direction. In this way, the flat plate behaves dynamically so as to repeatedly form different deformed forms reversibly. FIGS. 17(A) and 17(B) show the state in which a plurality of flows including a vortex flow, a suction flow, an extrusion flow, a swirling flow, a vertical convection flow, a circulating flow, and a turbulent flow is generated in the liquid in the stirring tank 232. That is, FIGS. 17(A) and 17(B) schematically show the movement of the stirring flow in the stirring tank 232.

According to the stirring body 231 including the stirring body 237A described above, when the guide member 236A, which is the driving shaft of the driving device 239 provided with the brushless motor, is reciprocated and rotated by a predetermined physical means within a driving range of 180 degrees in the left-right direction, the central portion side of the circular flat plate 233A made of an elastically deformable material reciprocates and rotates in the left-right direction. Moreover, the flat plate 233A is elastically deformed to the three-dimensional flat plate 233A having the plurality of wavy blade portions of which the front and back surfaces are exposed when seen from one direction and the three-dimensional flat plate 233A forming the neck portion, accompanied by a plurality of operations including rotational movement and reverse movement while the blade portion of the flat plate 233A being twisted. In this way, the flat plate 233A can behave dynamically so as to repeatedly form different deformed forms reversibly. In this way, a plurality of wavy blade portions of which the front and back surfaces are exposed when seen from one direction is formed in the liquid in the stirring tank 232 accompanied by rotational movement and reverse movement while the blade portion around the neck portion of the flat plate 233A being twisted. Moreover, an extrusion flow from the center direction of the three-dimensional flat plate 233A toward the side surface of the stirring tank 32 and a swirling flow toward the left side are generated. Moreover, the plurality of wavy blade portions converges toward the center of the flat plate 233A while being twisted and generates an extrusion flow. Furthermore, the three-dimensional flat plate 233A having the neck portion is formed, and an extrusion flow from the side surface of the stirring tank 232 toward the center of the flat plate 233A and a swirling flow toward the right side are generated. In this way, it is possible to stir a fluid medium in an extremely complex and unsteady manner accompanied by a plurality of flows including a vortex flow, a suction flow, an extrusion flow, a swirling flow, a vertical convection flow, a circulating flow, and a turbulent flow. That is, the stirring device functions as a stirring device including a stirring blade capable of adjusting the flow of a stirring flow by adjusting the arrangement positions of the movable members 234A and 235A in relation to the guide member 236A to appropriately form a plurality of displacement amounts reversibly by elastic deformation of the flat plate 233A. However, the driving range in which the guide member 236A, which is the driving shaft of the driving device 239 provided in the aforementioned stirring device 231 is reciprocated and rotated in the left-right direction is not limited to 180 degrees. For example, the driving range may be 90 degrees, 270 degrees, 360 degrees, 450 degrees, 540 degrees, 630 degrees, 720 degrees, or the like. That is, the driving range in which the guide member 236A, which is the driving shaft of the driving device 239 provided in the aforementioned stirring device 231 is reciprocated and rotated in the left-right direction is not particularly limited.

### (Sixth Embodiment of Stirring Device)

The second embodiment of the stirring body according to the present invention includes the flat plate in which a plurality of spiral blade portions extending from a central portion is formed by a spiral cut that does not contact an outer peripheral portion and which is made of an elastically deformable material, the guide member, and the movable member connected to one of the central portion and the outer peripheral portion of the flat plate. The movable member is rotatable and movable along the guide member and causes the guide member to reciprocate. Thus, the stirring body can be used as a stirring blade of a stirring device capable of stirring a fluid medium in an unsteady manner and adjusting the flow of a stirring flow. Hereinafter, a sixth embodiment of the stirring device including the stirring body according to the present invention will be described with reference to FIG. 18. The flat plates 203H, 2031, and 203J of a stirring body 247 included in a stirring device 244 of the present embodiment shown in FIG. 18 are made of an elastically deformable material and have exactly the same shape as the flat plates 203B, 203C, and 203D of the stirring bodies 201B, 201C, and 201D shown in FIGS. 11 to 13, that is, the same ones are used. The stirring device 244 including the stirring body 247 shown in FIG. 18 has the same configuration as the stirring devices 218, 227, and 229 including the stirring bodies 221, 228, and 230 shown in FIGS. 14 to 16, except that the stirring device 244 includes a driving device 249A provided with a brushless motor and reciprocating in the vertical direction and a rubber seal 250A having a bellows structure. Thus, the detailed description thereof will be omitted here.

As shown in FIG. 18, in the stirring device 244 includes a stirring tank 245A containing a liquid and having a volume of 300 mL, a stirring body 247 including a flat plate 203H, movable members 207E and 208E, and a guide member 246, a top plate portion 248A, a driving device 249A provided with a brushless motor and reciprocating in the vertical direction, and a rubber seal 250A having a bellows structure. As shown in FIG. 18(A), in the stirring device 244, the guide member 246 vertically suspended in the stirring tank 245A is connected to the seal 250A disposed in the center-side opening of the top plate portion 248A so as to be able to follow reciprocation. Thus, the inside of the tank 245A is airtightly provided.

As shown in FIGS. 18(A) and 18(B), in the stirring device 244, the movable member 27E is bonded to the central portion on the upper surface side of the flat plate 203H, and the rectangular movable member 208E is bonded to the outer peripheral portion on the bottom surface side of the flat plate 203H. The movable members 207E and 208E are separated from each other and slidably and rotatably attached to the guide member 246 passing through the through-hole provided in the central portion of the flat plate 203H. The movable members 207E and 208E are locked to the guide member 246 serving as the driving shaft of the driving device 249A so as not to be rotatable and movable in the axial direction by the lock mechanisms 225D and 226D such as hexagon socket set screws provided in the movable members 207E and 208E. In this way, a three-dimensional flat plate 203H having the blade portion 261 forming the neck portion is formed. The broken-line three-dimensional flat plates 2031 and 203J are obtained by elastically deforming the flat plate 203H to form two different deformed forms reversibly. The flat plate 2031 shows the deformed form having the truncated cone-shaped blade portion 2061, and the flat plate 203J shows the deformed form having a plurality of wavy blade portions 2061 of which the front and back surfaces are exposed when seen from one direction. The flat plate 203H has exactly the same shape as the flat plate 203C of the stirring body 201C shown in FIG. 12, the flat plate 2031 has exactly the same shape as the flat plate 203B of the stirring body 201B shown in FIG. 11, and the flat plate 203J has exactly the same shape as the flat plate 203D of the stirring body 201D shown in FIG. 13.

According to the stirring device 244 provided with the stirring body 247 described above, the movable members 207E and 208E bonded to the circular flat plates 203H, 2031, and 203J made of an elastically deformable material are separated from each other and slidably and rotatably attached to the guide member 246. The movable members 207E and 208E are locked to the guide member 246 so as not to be rotatable and movable in the axial direction by the lock mechanism 225D and 226D such as hexagon socket set screws provided in the movable members 207E and 208E. When the driving device 249A provided with a brushless motor is driven by a predetermined physical means so that the guide member 246 reciprocates in the direction of the arrow 251 shown in FIG. 18(A), that is, in the normal direction of the flat plates 203H, 2031, and 203J, the flat plates 203H, 2031, and 203J can be reciprocated in the vertical direction. In this way, when the three-dimensional flat plate 203H forming the blade portion 2061 having the neck portion is reciprocated in the vertical direction in the liquid in the stirring tank 245A, the guide member 246 reciprocates in the vertical direction, the blade portions 2061 generate a plurality of vortex flows around the adjacent blade portions 2061 and the gaps therebetween, the central portion of the flat plate 203H moves vertically to generate a vertical convection flow, the blade portions 2061 having the neck portion move in the vertical direction to generate a vortex flow based on a separation flow around the neck portion, and the outer peripheral portion of the flat plate 203H moves vertically to generate a large vortex flow. When the three-dimensional flat plate 2031 having the truncated cone-shaped blade portion 2061 is reciprocated in the vertical direction, the guide member 246 reciprocates in the vertical direction, the blade portions 2061 generate a plurality of vortex flows around the adjacent blade portions 2061 and the gaps therebetween, the central portion of the flat plate 2031 moves vertically to generate a vertical convection flow, and the outer peripheral portion of the flat plate 2031 moves vertically to generate a large vortex flow. Furthermore, when the complex three-dimensional flat plate 203J having the plurality of wavy blade portion 2061 of which the front and back surfaces are exposed when seen from one direction is reciprocated in the vertical direction, the guide member 246 reciprocates in the vertical direction, the blade portions 2061 generate a plurality of vortex flows around the adjacent blade portions 2061 and the gaps therebetween, the central portion of the flat plate 203J moves vertically to generate a vertical convection flow, and the plurality of wavy blade portions 2061 of which the front and back surfaces are exposed when seen from one direction moves vertically to generate a large vortex flow around a curved portion of the blade portion 2061. In this way, it is possible to generate a complex circulating flow and a complex turbulent flow in the liquid accompanied by a plurality of flows such as a large and small vortex flow, a separation flow, and a vertical convection flow to stir a fluid medium in an extremely complex and unsteady manner. That is, the stirring device functions as a stirring device including a stirring blade capable of adjusting the flow of a stirring flow by adjusting the arrangement positions of the movable members 207E and 208E in relation to the guide member 246 to appropriately and reversibly form a plurality of deformed forms with different displacement amounts by elastic deformation of the flat plates 203H, 2031, and 203J. Therefore, for example, when a physicochemical environment suitable for proliferation and metabolism of cultured cells, differentiation and maturation of megakaryocytes, polynuclearization of megakaryocytes, production of platelets, and maintenance of bioactivity of platelets is established in the aforementioned stirring device 44 of the present embodiment and a culture solution is stirred, it is possible to provide a stirring culture device including a stirring blade capable of stirring a culture solution in a more complex and unsteady manner as compared with conventional unsteady stirring required for stirring culture of culturing megakaryocyte cells derived from human iPS cells to produce a large amount of platelets.

### (Seventh Embodiment of Stirring Device)

The second embodiment of the stirring body according to the present invention includes the flat plate in which a plurality of spiral blade portions extending from a central portion is formed by a spiral cut that does not contact an outer peripheral portion and which is made of an elastically deformable material, the guide member, and the movable member connected to one of the central portion and the outer peripheral portion of the flat plate. The movable member is rotatable and movable along the guide member and causes the guide member to reciprocate. Thus, the stirring body can be used as a stirring blade of a stirring device capable of stirring a fluid medium in an unsteady manner and adjusting the flow of a stirring flow. Hereinafter, a seventh embodiment of a stirring device including the stirring body according to the present invention will be described with reference to FIG. 19. The stirring body 237B provided in the stirring device 252 of the present embodiment shown in FIG. 19 is the same as the stirring body 237A shown in FIG. 17. The stirring device 252 shown in FIG. 19 has the same configuration as the stirring device 244 including the stirring body 247 shown in FIG. 18 except that the stirring device 252 includes the stirring body 237B instead of the stirring body 247. Thus, the detailed description thereof will be omitted here.

As shown in FIG. 19, the stirring device 252 includes a stirring tank 245B containing a liquid and having a volume of 300 mL, a stirring body 237B including a circular flat plate 233B made of an elastically deformable material, movable members 234B and 235B, and a guide member 236B, a top plate portion 248B, a driving device 249B provided with a brushless motor and having a controller capable of adjusting the reciprocating distance which reciprocates in the vertical direction provided, a seal 250B, and an auxiliary member 241B. The guide member 236B which is the driving shaft of the driving device 249B vertically suspended in the stirring tank 245B is connected to the seal 250B disposed in the center-side opening of the top plate portion 248B so as to be able to follow reciprocation. Thus, the inside of the stirring tank 245B is airtightly provided. The three-dimensional flat plate 233B forming the blade portion 253A having the neck portion 215C has exactly the same shape as the flat plate 203C of the stirring body 201C shown in FIG. 12. The flat plate 233B having the blade portion 253A shows a deformed form at the substantially uppermost position in which the guide member 236B is at a substantially uppermost position of the movable range in which the guide member 236B reciprocates. The flat plate 233B having the broken-line blade portion 253B shows a deformed form at the substantially the lowermost position of the movable range in which the guide member 236B reciprocates.

As shown in FIGS. 19(A) and 19(B), in the stirring body 252, two movable members 234B are provided around the central portion of the flat plate 233B at intervals of 180 degrees and bonded to the outer peripheral portion of the flat plate 233B. The movable member 235B is bonded to the central portion of the flat plate 233B and the top plate portion 248B and the auxiliary member 241B are bonded together. As shown in FIG. 19(A), the movable members 234B and 235B are separated from each other and slidably and rotatably attached to the auxiliary member 241B and the guide member 236B, respectively. The movable members 234B and 235B are locked to the auxiliary member 241B and the guide member 236B, respectively, so as not to be rotatable and movable in the axial direction by the lock mechanisms 242B and 243B such as hexagon socket set screws provided in the movable members 234B and 235B, respectively, and the outer peripheral portion side of the flat plate 233B is fixed. In this way, a three-dimensional flat plate 233B forming the blade portion 253A having a neck portion 215C is formed. In this way, when the guide member 236B, which is the driving shaft of the driving device 249B, is reciprocated in the direction of the arrow 251 by a predetermined physical means, the flat plate 233B is elastically deformed, the blade portions 253A and 253B perform a plurality of operation movements such as an expansion/contraction movement in the vertical direction, a spreading movement, and a converging movement while being twisted, and the flat plate 233B dynamically behaves so as to repeatedly form different deformed forms reversibly. Thus, a plurality of flows such as a vortex flow, a separation flow, a suction flow, an extrusion flow, a vertical convection flow, a circulating flow, and a turbulent flow is generated in the liquid in the stirring tank 245B. That is, FIG. 19(A) schematically shows the movement of the stirring flow in the stirring tank 245B by the stirring device 252.

According to the stirring device 252 including the stirring body 237B described above, when the guide member 236B which is the driving shaft of the driving device 249B provided with a brushless motor is reciprocated by a predetermined physical means, the flat plate 233B made of an elastically deformable material is elastically deformed. In this way, the flat plate 233B can behave dynamically so as to repeatedly form different deformed forms reversibly from the two-dimensional flat plate 233B to the truncated cone-shaped three-dimensional flat plate 233B and the three-dimensional flat plate 233B forming the neck portion 215C. In this way, the guide member 236B reciprocates in the vertical direction in the liquid in the stirring tank 245B, the blade portions 253A and 253B generate a plurality of flows accompanied by a vortex flow around the adjacent blade portions 253A and 253B and the gaps therebetween. Moreover, the central portion of the flat plate 233B moves vertically to generate a vertical convection flow, and the neck portion 215C formed by the vertical movement of the central portion of the flat plate 233B generates a vortex flow based on a separation flow. Furthermore, the central portion of the flat plate 233B is raised and a suction flow rising due to a suction force from the bottom side of the stirring tank 245B toward the central portion of the flat plate 233B is generated. The suction flow hits the neck portion 215C and the blade portions 253A and 253B that converge each other and generates a vortex flow and a vertical convection flow. The central portion of the flat plate 233B is lowered and the blade portions 253A and 253B and the neck portion 215C spread toward the bottom side in the stirring tank 245B to generate an extrusion flow and a vertical convection flow that descend. In this way, the stirring device functions as a stirring device including a stirring blade capable of generating a plurality of flows such as a vortex flow, a separation flow, a suction flow, an extrusion flow, a vertical convection flow, a circulating flow, and a turbulent flow to stir a fluid medium in an extremely complex and unsteady manner with low shear. Moreover, the stirring device functions as a stirring device including a stirring blade capable of adjusting the flow of a stirring flow by adjusting the arrangement positions of the movable members 234B and 235B in relation to the guide member 236B, adjusting the displacement amount of the flat plate 233B by elastic deformation, and appropriately reversibly forming a plurality of deformed forms with different displacement amounts by elastic deformation. Therefore, for example, when a physicochemical environment suitable for proliferation and metabolism of cultured cells, differentiation and maturation of megakaryocytes, polynuclearization of megakaryocytes, production of platelets, and maintenance of bioactivity of platelets is established in the aforementioned stirring device 252 and a culture solution is stirred, it is possible to provide a stirring blade capable of stirring the culture solution in a more complex and unsteady manner with low shear accompanied by a plurality of flows as compared with conventional unsteady stirring required for stirring culture of culturing megakaryocyte cells derived from human iPS cells to produce a large amount of platelets. Moreover, it is possible to provide a stirring blade that can be manufactured easily, and that can adjust the flow of a stirring flow by adjusting the arrangement position of the movable member in relation to the guide member, adjusting the displacement amount of the flat plate by elastic deformation, and appropriately forming a deformed form without the need to provide a plurality of driving devices having different operating directions or a complex driving mechanism for the driving device, which was conventionally required for the stirring blade to reciprocate in the vertical direction and the forward and reverse directions.

### (Eighth Embodiment of Stirring Device)

The second embodiment of the stirring body according to the present invention includes the flat plate in which a plurality of spiral blade portions extending from a central portion is formed by a spiral cut that does not contact an outer peripheral portion and which is made of an elastically deformable material, the guide member, and the movable member connected to one of the central portion and the outer peripheral portion of the flat plate. The movable member is rotatable and movable along the guide member and causes the guide member to rotate. Thus, the stirring body can be used as a stirring blade of a stirring device capable of satisfactorily stirring a fluid medium without any mixing unevenness accompanied by a complex vertical reflux and adjusting the flow of a stirring flow. Hereinafter, the eighth embodiment by the stirring device including the stirring body according to the present invention will be described with reference to FIG. 20. The blade portion 259B of the flat plate 254B of the stirring body 266 provided in the stirring device 262 of the present embodiment shown in FIG. 20 has exactly the same shape as the blade portion 206C of the flat plate 203C shown in FIG. 21. The flat plate 254B shown in FIG. 20 is the same as the flat plate 203C shown in FIG. 21 except that a through-hole 260 and a cut 261 are provided in the outer peripheral portion of the flat plate 254B.

As shown in FIG. 20(A), in the flat plate 254A, four cuts 55 are formed so as to extend in a spiral form about 540 degrees around the central portion 256 from the outer-peripheral-side ends 257 located inside the outer peripheral edge of the circular flat plate 254A made of an elastically deformable material and provided around the central portion 256 of the flat plate 254A at intervals of 90 degrees toward the inner-peripheral-side ends 258 located near the central portion 256 and provided around the central portion 256 at intervals of 90 degrees. That is, four blade portions 259A are arranged at positions rotated by 90 degrees in the circumferential direction around the central portion 256 with respect to the adjacent blade portions 259A, respectively, and are formed so as to extend in a spiral shape about 540 degrees around the central portion 256. Further, the flat plate 254A has a circular through-hole in the central portion 256, four circular through-holes 260 are arranged in the outer peripheral portion of the flat plate 254A provided around the central portion 256 at intervals of 90 degrees, and the four slit-shaped cuts 261 are formed at the outer-peripheral-side end 257 of the cut 255. The outer-peripheral-side end 257 of the cut 255 is preferably formed so as to be separated from the outer peripheral edge toward the central portion 256 of the flat plate 254A by a distance of 5 to 10% of the diameter of the flat plate 254A. The inner-peripheral-side end 258 of the cut 255 is preferably formed so as to be separated from the central portion 256 of the flat plate 254A toward the outer peripheral edge by a distance of 5 to 10% of the diameter of the flat plate 254A.

As shown in FIGS. 20(B) to 20(D), the stirring device 262 includes a stirring body 266 including a flat plate 254B, movable members 263 and 264, and a guide member 265, a center cap 268 having a bearing 267, two side arm caps 269, a glass stirring body tank 270 with three openings, containing liquid and having a volume of 500 mL, and a magnetic stirring driving device 272 provided with a motor 271 having a bar magnet provided at the tips of a driving shaft having a T-shaped branch portion. As shown in FIG. 20(B), the movable member 263 is bonded to the bottom surface side of the central portion 256 of the flat plate 254B. As shown in FIGS. 20(B) to 20(D), the movable member 264 has four branch portions provided at intervals of 90 degrees with the guide member 265 as the base point. Two bar magnets 273 are arranged at the tip of the branch portion and sealed with polytetrafluoroethylene (trade name: Teflon). The movable member 264 is locked to the tip of the guide member 265 having a thread groove so as not to be rotatable and movable in the axial direction by a lock mechanism such as on fastening of screw members provided in the movable member 264. A cylindrical protrusion provided at the tip of the branch portion of the movable member 264 is received in the through-hole 260 of the flat plate 254B. In this way, the outer peripheral portion side of the flat plate 254B is fixed to the movable member 264, and the movable member 263 is slidably and rotatably attached to the guide member 265 so as to be separated from the movable member 264. The movable member 263 is locked to the guide member 265 rotatably connected to the bearing 267 so as not to be rotatable and movable in the axial direction by a lock mechanism such as fastening of two screw members provided in the movable member 263. In this way, a three-dimensional flat plate 254B having a blade portion 259B forming a neck portion is formed as shown in FIG. 20(B). Further, the stirring tank 270 is disposed on the magnetic stirring driving device 272 as shown in FIG. 20(B) such that the inside of the stirring tank 270 is airtightly provided by an O-ring provided in the center cap 268 and the two side arm caps 269. The three-dimensional flat plate 254B having the blade portion 259B forming the neck portion shows a deformed form at the uppermost position of the movable range of the axial movement of the movable member 263 attached to the guide member 265. The flat plate 254C having the broken-line blade portion 259C shows a deformed form at the lowermost position of the movable range in the axial direction of the movable member 263 attached to the guide member 265.

According to the stirring device 262 including the stirring body 266 described above, the movable member 264 having the bar magnet 273 is sealed with polytetrafluoroethylene (registered trademark: Teflon) and bonded to the guide member 265 rotatably connected to the bearing 267 having the center cap 268. The cylindrical protrusion provided at the tip of the branch portion of the movable member 264 is received in the through-hole 260 provided in the circular flat plate 254B made of an elastically deformable material so that the outer peripheral portion of the flat plate 254B is fixed. The arrangement position of the movable member 263 in relation to the guide member 265 is adjusted by the lock mechanism of the movable member 263 slidably and rotatably attached to the guide member 265 so that the movable member 263 is locked to the guide member 265 so as not to be rotatable and movable in the axial direction. In this way, when the magnetic stirring driving device 272 is driven by a predetermined physical means, the motor 271 having the driving shaft and the bar magnet provided in the magnetic stirring driving device 272 rotates. In this way, the flat plates 254B and 254C can be rotated in the stirring tank 270 by the magnetic force. In this way, in the liquid in the stirring tank 270, the three-dimensional flat plate 254B having the blade portion 259B forming the neck portion generates a complex vertical reflux accompanied by a plurality of flows such as a suction flow based on a suction force generated from the bottom surface side near the outer peripheral portion of the flat plate 254B toward the central portion of the flat plate 254B, a suction flow based on a suction force generated from the vicinity of the neck portion toward the central portion of the flat plate 254B, a swirling flow, and a vertical convection flow. Moreover, the three-dimensional flat plate 254C having the truncated cone-shaped blade portion 259C generates a complex vertical reflux accompanied by a plurality of flows such as a suction flow based on a suction force generated from the bottom surface side near the outer peripheral portion of the flat plate 254C toward the central portion of the flat plate 254C, a swirling flow, and a vertical convection flow to satisfactorily stir a fluid medium without any mixing unevenness. In this way, it is possible to provide a stirring device including a stirring blade capable of adjusting the flow of a stirring flow by adjusting the arrangement positions of the movable members 263 and 264 in relation to the guide member 265, adjusting the displacement amounts of the flat plates 254B and 254C by elastic deformation, and appropriately and reversibly forming a plurality of deformed forms with different displacement amounts by elastic deformation. Therefore, for example, when the flat plates 254A and 254B, the movable members 263 and 264, the guide member 265, the bearing 267, the center cap 268, the side arm cap 269, and the O-ring are processed with polytetrafluoroethylene (registered trademark: Teflon), and the motor 271 provided in the magnetic stirring driving device 272 (magnetic stirring body) is driven by a predetermined physical means, the flat plate 254B connected to the movable member 264 provided with the bar magnet 273 rotates. In this way, it is possible to generate a complex vertical reflux accompanied by a plurality of flows in the liquid in the stirring tank 270, such as a suction flow based on a suction force generated from the bottom surface side near the outer peripheral portion of the flat plate 254B toward the central portion of the flat plate 254B, a suction flow based on a suction force from the vicinity of the neck portion toward the central portion of the flat plate 254B, a swirling flow, and a vertical convection flow to satisfactorily stir a fluid medium without any mixing unevenness. Moreover, it is possible to adjust the flow of a stirring flow by adjusting the arrangement positions of the movable members 263 ad 264 in relation to the guide member 265, adjusting the displacement amounts of the flat plates 254A, 254B, and 254C, and appropriately and reversibly forming a plurality of deformed forms with different displacement amounts by elastic deformation. Moreover, since such a sealing portion as in a direct drive-type stirring device is not required, the stirring device can be used as a stirring blade and a magnetic stirring device or a spinner flask including the stirring blade, which has no contamination (bacterial contamination) and is easy to clean. However, the flat plate 254B, the movable members 263 and 264, the guide member 265, the bearing 267, the center cap 268, the side arm cap 269, and the O-ring are not limited to being processed by polytetrafluoroethylene (registered trademark: Teflon). For example, they may be sealed with glass coating, polycarbonate, or the like. That is, the materials and processing methods of the flat plate 254B, the movable members 263 and 264, the guide member 265, the bearing 267, the center cap 268, the side arm cap 269, and the O-ring are not particularly limited. Further, the means for generating magnetization of the magnetic stirring driving device 272 provided in the stirring device 262 is not limited to magnets and electromagnets that generate magnetization by electricity may be used. That is, the means for generating the magnetization of the magnetic stirring driving device of the stirring device including the stirring body according to the present invention is not particularly limited.

### (Ninth Embodiment of Stirring Device)

The second embodiment of the stirring body according to the present invention includes the flat plate in which a plurality of spiral blade portions extending from a central portion is formed by a spiral cut that does not contact an outer peripheral portion and which is made of an elastically deformable material, the guide member, and the movable member connected to one of the central portion and the outer peripheral portion of the flat plate. The movable member is rotatable and movable along the guide member and causes the guide member to rotate. Thus, the stirring body can be used as a stirring blade of a stirring device capable of stirring a fluid medium in an unsteady manner, rotating the guide member to generate a complex vertical reflux to satisfactorily stir a fluid medium without any mixing unevenness, and adjusting the flow of a stirring flow. Hereinafter, a ninth embodiment of the stirring device including the stirring body according to the present invention will be described with reference to FIG. 21. The flat plate 276 of the stirring body 279 provided in the stirring body 274 of the present embodiment shown in FIG. 21 is the same as the flat plate 233B of the stirring body 237B shown in FIG. 19. Therefore, the detailed description thereof will be omitted here.

As shown in FIG. 21, the stirring body 274 includes a stirring tank 275 containing a liquid and having a volume of 300 mL, a stirring body 279 including a circular flat plate 276 made of an elastically deformable material, a movable member 277, and a guide member 278, a top plate portion 280, ceramic bearings 281, 282, gears 283, 284, a protective portion 285, a branch portion 286, a driving device 287 provided with a brushless motor and having a controller capable of adjusting a reciprocating distance in the vertical direction, and a rotating driving device 288. The movable member 277 is reciprocated and the guide member 278 is rotated by the driving devices 287 and 288. In this way, the flat plate 276 rotates accompanied by dynamic behavior so as to repeatedly form different deformed forms reversibly by elastic deformation. The three-dimensional flat plate 276 forming the blade portion 289A having a neck portion has exactly the same shape as the flat plate 203C shown in FIG. 12. The three-dimensional flat plate 276 having the broken-line truncated cone-shaped blade portion 289B has exactly the same shape as the flat plate 203B shown in FIG. 11. The flat plate 276 having the blade portion 289A shows a deformed form at the substantially uppermost position of the movable range in which the movable member 277 reciprocates. The flat plate 276 having the blade portion 289B shows a deformed form at the substantially lowermost position of the movable range in which the movable member 277 reciprocates. However, the material of the bearings 281 and 282 is not limited to ceramics but may be stainless steel, resin or the like. That is, the material of the bearings 281 and 282 is not particularly limited.

As shown in FIGS. 21(A) to 21(C), in the stirring body 274, the guide member 278 having a pipe-type structure as shown in FIG. 21(A) includes the gear 283 on the upper part of the central shaft thereof and the circular protective portion 285 provided in the lower part thereof so as to prevent liquid from adhering to the bearing 281. The guide member 278 is connected to the bearing 281 provided on the top plate portion 280, and two branch portions 286 are provided around the central portion of the flat plate 276 at intervals of 180 degrees and bonded to the protective portion 285 and the outer peripheral portion of the flat plate 276. The gear 283 and the gear 284 provided on the driving shaft of the driving device 288 are rotatably combined. Further, the movable member 277, which is the driving shaft of the driving device 287, is vertically suspended in the stirring tank 275 and connected to the bearing 282 provided in the central portion of the flat plate 276, so that the central portion side of the flat plate 226 is rotatably supported in relation to the movable member 227. In this way, when the driving shaft of the driving device 288 rotates in the direction of the arrow 290, the guide member 278 having the gear 283 connected to the gear 284 is rotated in the direction of the arrow 291 and the flat plate 276 is rotated. Moreover, the movable member 277, which is the driving shaft of the driving device 287, is reciprocated in the direction of the arrow 292, and the central portion side of the flat plate 276 is reciprocated in the vertical direction. As shown in FIG. 21(A), the flat plate 276 is elastically deformed to the flat plate 276 forming the blade portion 289A having the neck portion and the flat plate 276 having the truncated cone-shaped blade portion 289B, and the flat plate 276 is rotated accompanied by dynamic behavior so as to repeatedly form different deformed forms reversibly. The driving device 287 includes a controller that adjusts the distance for moving the movable member 277 along the guide member 278, and can appropriately reciprocate within a distance of the movable range 293.

According to the stirring device 274 including the aforementioned stirring body 279, when the driving device 287 and 288 are driven by a predetermined physical means so that the movable member 277 and the guide member 278 appropriately reciprocate and rotate, the flat plate 276 made of an elastically deformable material is elastically deformed. Thus, the flat plate 276 can dynamically behave so as to repeatedly form different deformed forms reversibly from the three-dimensional flat plate 276 having the truncated cone-shaped blade portion 289B to the three-dimensional flat plate 276 forming the blade portion 289A having a neck portion, and the guide member 278 can rotate around the central portion of the flat plate 276 as the base axis. In this way, the movable member 277 reciprocates in the vertical direction in the liquid in the stirring tank 275, and the blade portions 289A and 289B generate a plurality of flows accompanied by a vortex flow around the adjacent blade portions 289A and 289B and the gaps therebetween. Moreover, the central portion of the flat plate 276 moves vertically to generate a vertical convection flow. Further, the neck portion formed by the vertical movement of the central portion of the flat plate 276 generates a vortex flow based on a separation flow. The central portion of the flat plate 276 is raised and generates a suction flow rising due to a suction force from the bottom side of the stirring tank 275 toward the central portion of the flat plate 276. The suction flow hits the neck portion and the blade portions 289A and 289B that converge each other and generates a vortex flow and a vertical convection flow. The central portion of the flat plate 276 is lowered and the blade portions 289A and 289B and the neck portion spread toward the bottom side in the stirring tank 275 to generate an extrusion flow and a vertical convection flow that descend. In this way, the blade portions 289A and 289B can generate a plurality of flows such as a vortex flow, a separation flow, a suction flow, an extrusion flow, a vertical convection flow, a circulating flow, and a turbulent flow and stir a fluid medium in an extremely complex and unsteady manner with low shear. Further, the guide member 278 rotates around the central portion of the three-dimensional flat plate 276 having the broken-line truncated cone-shaped blade portion 289B as the base axis. Moreover, a complex vertical reflux is generated accompanied by a plurality of flows such as a swirling flow, a vertical convection flow, and a suction flow due to a suction force generated from the bottom side near the outer peripheral portion of the flat plate 276 toward the central portion of the flat plate 276. In this way, it is possible to satisfactorily stir a fluid medium without any mixing unevenness. Further, the guide member 278 rotates around the central portion of the three-dimensional flat plate 276 forming the blade portion 289A having the neck portion as the base axis. Moreover, a complex vertical reflux is generated accompanied by a plurality of flows such as a suction flow due to a suction force generated from the bottom side near the outer peripheral portion of the flat plate 276 toward the central portion of the flat plate 76, a suction flow due to a suction force generated from the vicinity of the neck portion toward the central portion of the flat plate 276, a swirling flow, and a vertical convection flow. In this way, it is possible to satisfactorily stir a fluid medium without any mixing unevenness. Furthermore, when the controller provided in the driving device 87 adjusts the relative moving distance between the movable member 277 and the guide member 278, the flat plate 276 can function as a displacement amount adjustment mechanism, that is, a shape deformation adjustment mechanism capable of appropriately forming a plurality of deformed forms with different displacement amounts by elastic deformation. Moreover, since the driving devices 287 and 288 adjust the operating direction based on reciprocating movement, rotational movement, or a combination thereof, the controller can function as a mechanism for adjusting the operation of the flat plate 276. Therefore, for example, when an elastically deformable material is used for the material of the flat plate 276 of the aforementioned stirring body 279 of the present embodiment and the driving devices 287 and 288 are driven by a predetermined physical means to cause the movable member 277 and the guide member 278 to appropriately reciprocate and rotate, the movable member 277 reciprocates in relation to the guide member 278 in a direction in which the outer peripheral portion and the central portion of the flat plate 276 approach and separate from each other. The blade portions 289A and 289B are elastically deformed while being twisted accompanied by an expansion/contraction movement in the vertical direction, a rotational movement in forward and reverse directions, a spreading movement, and a spreading/converging movement and the flat plate 276 behaves dynamically so as to repeatedly form different deformed forms reversibly. In this way, it is possible to stir a fluid medium in the liquid in an extremely complex and unsteady manner with low shear accompanied by a plurality of flows such as a vortex flow, a separation flow, a suction flow, an extrusion flow, a swirling flow, a vertical convection flow, a circulating flow, and a turbulent flow. Moreover, when the guide member 278 rotates around the central portion of the flat plate 76 as the base axis, the flat plate 276 is elastically deformed to behave dynamically so as to repeatedly and reversibly form different deformed forms including the three-dimensional flat plate 276 having the truncated cone-shaped blade portion 289B and the three-dimensional flat plate 276 forming the blade portion 289A having the neck portion. In this way, it is possible to generate a complex vertical reflux accompanied by a plurality of flows such as a suction flow, a swirling flow, and a vertical convection flow and satisfactorily stir a fluid medium without any mixing unevenness. Moreover, the controller provided in the driving device 287 controls the reciprocating distance of the movable member 277 and the flat plate 76 is elastically deformed to appropriately form deformed forms with different displacement amounts reversibly. In this way, it is possible to provide a stirring blade that can automatically adjust the flow of a stirring flow and that can be easily manufactured. However, the configuration of the stirring device 274 that is driven in the aforementioned plurality of operating directions is not limited to a configuration in which the gears 283 and 284 are combined and the driving devices 287 and 288 are arranged on the upper side of the stirring tank 275. For example, any one of the driving devices 287 and 288 may be arranged on the upper side of the stirring tank 275 and the other may be arranged on the bottom side of the stirring tank 275 so that the flat plate 276 can reciprocate and rotate. The configuration of the stirring device 274 driven in the aforementioned plurality of operating directions is not particularly limited.

### (Configuration Example of Flat Plate in Second Embodiment of Stirring body)

As shown in FIG. 12, in the stirring body 201C, the movable members 207A and 208A supporting one flat plate 203C are arranged apart from each other with respect to the guide member 209. However, the number of flat plates of the stirring body according to the present invention is not limited to one, and for example, two or more flat plates may be connected to each other at the outer peripheral portions or the central portions thereof. The number and configuration of the flat plates of the stirring body according to the present invention are not particularly limited. FIGS. 22 to 24 illustrate embodiments of various configurations of the flat plate of the stirring body according to the present invention. The circular flat plates 303K, 303L, 303M, 303N, 303O, 303P, 303Q, 303R, 303S, 303T, 303U, 303V, 303W, and 303X included in the stirring bodies 294, 296, 298, 301, 304, 307, 311, and 314 shown in FIGS. 22 to 24 are made of an elastically deformable material and have exactly the same shape as any one of the flat plates 203B, 203C, and 203D shown in FIGS. 11 to 13, that is, are the same. Thus, the detailed description thereof will be omitted here.

As shown in FIG. 22(A), the stirring body 394 includes flat plates 303K and 303L, a guide member 395, and movable members 307F, 307G, 308F, and 308G. The rectangular movable members 308F and 308G terminating in the vicinity of the outer peripheral edge on the bottom surface side of the flat plates 303K and 303L are bonded to the outer peripheral portions of the flat plates 303K and 303L. The movable members 307F and 307G bonded to the central portions of the flat plates 303K and 303L are separated from each other and slidably and rotatably attached to the guide member 395 passing through a through-hole provided in the central portions of the flat plates 303K and 303L. The movable members 307F, 307G, 308F, and 308G are locked to the guide member 395 so as not to be rotatable and movable in the axial direction by lock mechanisms such as hexagon socket set screws provided in the movable members 307F, 307G, 308F, and 308G. As shown in the drawing, the flat plates 303K and 303L are arranged in a column with respect to the guide member 395. The flat plates 303K and 303L have exactly the same shape as the flat plate 203C having the three-dimensional shape shown in FIG. 12.

As shown in FIG. 22(B), the stirring body 396 includes flat plates 303M and 303N, a guide member 397, and movable members 307H, 3071, 308H, and 3081. The rectangular movable members 308H and 3081 terminating in the vicinity of the outer peripheral edges of the flat plates 303M and 303N are bonded to the outer peripheral portions of the flat plates 303M and 303N. The movable members 307H and 3071 bonded to the central portions of the flat plates 303M and 303N are separated from each other and slidably and rotatably attached to the guide member 397 having the through-hole provided in the central portions of the flat plates 303M and 303N. The movable members 307H, 3071, 308H, and 3081 are locked to the guide member 397 so as not to be rotatable and movable in the axial direction by the lock mechanisms such as hexagon socket set screws provided in the movable members 307H, 3071, 308H, and 3081. As shown in the drawing, the flat plates 303M and 303N are arranged in a column with respect to the guide member 397 so that the outer peripheral portions thereof are adjacent to each other. The flat plates 303M and 303N have exactly the same shape as the flat plate 203C having the three-dimensional shape shown in FIG. 12.

As shown in FIG. 23(A), the stirring body 398 includes a flat plate 399 in which the flat plates 303O and 303P are bonded to each other at the central portions thereof, a guide member 300, and movable members 308J and 308K. The rectangular movable members 308J and 308J terminating in the vicinity of the outer peripheral edges of the flat plates 303O and 303P are bonded to the outer peripheral portions of the flat plates 303O and 303P. The movable members 308J and 308J are separated from each other and slidably and rotatably attached to the guide member 300 passing through a through-hole provided in the central portions of the flat plates 303O and 303P. The movable members 308J and 308K are locked to the guide member 100 so as not to be rotatable and movable in the axial direction by lock mechanisms such as hexagon socket set screws provided in the movable members 308J and 308K. As shown in the drawing, the flat plate 399 is supported with respect to the guide member 300. The flat plates 303O and 303P have exactly the same shape as the flat plate 203C having the three-dimensional shape shown in FIG. 12.

As shown in FIG. 23(B), the stirring body 301 includes a flat plate 302 in which the flat plates 303Q and 303R are bonded to each other at the outer peripheral portions thereof, a guide member 33, and movable members 30 and 30. The movable members 30 and 30 bonded to the central portions of the flat plates 30 and 30 are separated from each other and slidably and rotatably attached to the guide member 33 passing through a through-hole provided in the central portions of the flat plates 30 and 30. The movable members 30 and 30 are locked to the guide member 303 so as not to be rotatable and movable in the axial direction by lock mechanisms such as hexagon socket set screws provided in the movable members 30 and 30. As shown in the drawing, the flat plate 32 is supported with respect to the guide member 33. The flat plate 303Q has exactly the same shape as the flat plate 203D having the three-dimensional shape shown in FIG. 13, and the flat plate 303R has exactly the same shape as the flat plate 203B having the three-dimensional shape shown in FIG. 11.

As shown in FIG. 23(C), the stirring body 304 includes a flat plate 305 in which the flat plate 303S is superposed on the flat plate 303T so that the outer peripheral portions thereof are bonded together, a guide member 306, movable members 307L, 307M, and 308L. The rectangular movable member 308L terminating in the vicinity of the outer peripheral edge on the bottom surface side of the flat plate 305 is bonded to the outer peripheral portion of the flat plate 305. The movable members 307L and 307M bonded to the movable member 308L and the central portions of the flat plates 303S and 303T are separated from each other and slidably and rotatably attached to the guide member 306 passing through a through-hole provided in the central portions of the flat plates 303S and 303T. The movable members 307L, 307M, and 308L are locked to the guide member 306 so as not to be rotatable and movable in the axial direction by lock mechanisms such as hexagon socket set screws provided in the movable members 307L, 307M, and 308L. As shown in the drawing, the flat plate 305 is supported with respect to the guide member 306. The flat plate 303S has exactly the same shape as the flat plate 203C having the three-dimensional shape shown in FIG. 12, and the flat plate 303T has exactly the same shape as the flat plate 203B having the three-dimensional shape shown in FIG. 11 of which the dimensions are reduced by 30% at a magnification of 1:1.

As shown in FIGS. 24(A) and 24(B), the stirring body 307 includes a flat plate 303U, a guide member 308, and a U-shaped movable member 309. The tips of the movable member 309 are provided around the central portion of the flat plate 303U at intervals of 180 degrees and bonded to the outer peripheral portion of the flat plate 303U. The guide member 308 passes through a lock mechanism 310 provided in the intermediate portion of the movable member 309 and is bonded to the central portion of the flat plate 303U. The lock mechanism 310 of the movable member 309 is slidably and rotatably attached to the guide member 308. The movable member 309 is locked to the guide member 308 so as not to be rotatable and movable in the axial direction by a hexagon socket set screw provided in the lock mechanism 310. As shown in the drawing, the flat plate 303U is supported with respect to the guide member 308. The flat plate 303U has exactly the same shape as the flat plate 203C having the three-dimensional shape shown in FIG. 12.

As shown in FIGS. 24(C) and 24(D), the stirring body 311 includes a flat plate 303V, a guide member 312 having three branch portions, and movable members 307N and 313A. Two movable members 313A are provided around the central portion of the flat plate 303V at intervals of 180 degrees and the movable member 307N is bonded to the central portion of the flat plate 303V. The movable members 307N and 313A are separated from each other and slidably and rotatably attached to the guide member 312. The movable members 307N and 313A are locked to the guide member 312 so as not to be rotatable and movable in the axial direction by lock mechanisms such as hexagon socket set screws provided in the movable members 307N and 313A. As shown in the drawing, the flat plate 303V is supported with respect to the guide member 312. The flat plate 303V has exactly the same shape as the flat plate 203C having the three-dimensional shape shown in FIG. 12.

As shown in FIGS. 24(E) and 24(F), the stirring body 314 includes flat plates 303W and 303X, a guide member 315 having a rectangular frame and having four branch portions, and movable members 307O, 307P, 313B, and 313C. The movable members 313B and 313C are provided around the central portions of the flat plates 303W and 303X at intervals of 180 degrees and bonded to the outer peripheral portions of the flat plates 303W and 303X. The movable members 307O and 307P are bonded to the central portions of the flat plates 303W and 303X. The movable members 307O, 307P, 313B, and 313C are separated from each other and slidably and rotatably attached to the guide member 315. The movable members 307O, 307P, 313B, and 313C are locked to the guide member 315 so as not to be rotatable and movable in the axial direction by lock mechanisms such as hexagon socket set screws provided respectively in the movable members 307O, 307P, 313B, and 313C. As shown in the drawing, the flat plates 303W and 303X are supported with respect to the guide member 315. The flat plates 303W and 303X have exactly the same shape as the flat plate 203C having the three-dimensional shape shown in FIG. 12.

In any of the aforementioned flat plates 303K, 303L, 303M, 303N, 303O, 303P, 303Q, 303R, 303S, 303T, 303U, 303V, 303W, and 303X, the cuts are formed so as not to contact the outer peripheral edges of the flat plates 303K, 303L, 303M, 303N, 303O, 303P, 303Q, 303R, 303S, 303T, 303U, 303V, 303W, and 303X.

According to the aforementioned stirring bodies 394, 396, 398, 301, 304, 307, 311, and 314, when the flat plates 303K, 303L, 303M, 303N, 303O, 303P, 303Q, 303R, 303S, 303T, 303U, 303V, 303W, and 303X made of an elastically deformable material are appropriately combined and are locked to the guide members 395, 397, 300, 303, 306, 308, 312, and 315 so as not to be rotatable and movable in the axial direction by the lock mechanisms provided in the movable members 307F, 307G, 308F, 308G, 307H, 3071, 308H, 3081, 308J, 308K, 307J, 307K, 307L, 307M, 308L, 309, 307N, 313A, 307O, 307P, 313B, and 313C, the flat plates 303K, 303L, 303M, 303N, 399, 302, 305, 303U, 303V, 303W, 303X can be elastically deformed to appropriately and reversibly form a plurality of deformed forms with different displacement amounts. Therefore, for example, when the stirring bodies 394, 396, 398, 301, 304, 307, 311, and 314 of the present embodiment are used in place of the stirring body 221 of the stirring device 218 shown in FIG. 14, the flat plates 303K, 303L, 303M, 303N, 399, 302, 305, 303U, 303V, 303W, and 303X are rotated. In this way, it is possible to generate a complex vertical reflux in the flow the liquid accompanied by a plurality of flows such as a swirling flow, a vertical convection flow, and a suction flow to satisfactorily stir a fluid medium without any mixing unevenness. Moreover, the arrangement positions of the movable members 307F, 307G, 308F, 308G, 307H, 3071, 308H, 3081, 308J, 308K, 307J, 307K, 307L, 307M, 308L, 309, 307N, 313A, 307O, 307P, 313B, and 313C in relation to the guide members 395, 397, 300, 303, 306, 308, 312, and 315 can be adjusted, and the flat plates 303K, 303L, 303M, 303N, 303O, 303P, 303Q, 303R, 303S, 303T, 303U, 303V, 303W, and 303X are elastically deformed to appropriately and reversibly form a plurality of deformed forms with different displacement amounts. In this way, it is possible to provide a stirring blade that can adjust the flow of a stirring flow and has a simple configuration and can be manufactured easily, and to provide a stirring device including the stirring blade.

### (Example of Forming Flat Plate in Second Embodiment of Stirring body)

As shown in FIG. 10, in the flat plate 203A of the stirring body 201A, the four cuts 202 are formed so as to extend in a spiral form about 540 degrees around the central portion of the flat plate 203A from the outer-peripheral-side ends 204 located inside the outer peripheral edge of the circular flat plate 203A, respectively and provided around the central portion of the flat plate 203A at intervals of 90 degrees toward the inner-peripheral-side ends 205A located near the central portion of the flat plate 203A and provided around the central portion at intervals of 90 degrees. However, the method of forming the flat plate of the stirring body according to the present invention is not limited to a method in which both ends of the cut 202 as shown in FIG. 10 are terminated near the inner side of the outer peripheral edge and the central portion of the flat plate 203A. For example, the flat plate and the flat plate auxiliary member in which the cut is penetrated to the outer peripheral edge may be appropriately combined to form a shape the same as or similar to that of the flat plates 203A, 203B, 203C, and 203D of the stirring bodies 201A, 201B, 201C, and 201D as shown in FIGS. 10 to 13 to obtain the same features and effects as the flat plates 203A, 203B, 203C, and 203D. The method of forming the flat plate of the stirring body according to the present invention is not particularly limited. Hereinafter, embodiments of various forming methods of the flat plate of the stirring body according to the present invention will be described with reference to FIG. 25. The flat plates 316 and 327 shown in FIG. 25 are made of an elastically deformable material.

In the three-dimensional flat plate 316 forming the neck portion as shown in FIG. 25(A), a circular through-hole is provided in the central portion 318 of the flat plate 317, and four cuts 319 are formed so as to extend in a spiral form about 540 degrees around the central portion 318 from the outer-peripheral-side ends 320 located in the outer peripheral edge of the flat plate 317 provided around the central portion 318 of the flat plate 317 at intervals of 90 degrees toward the inner-peripheral-side ends 321 located near the central portion 318 and provided around the central portion 318 at intervals of 90 degrees. Further, the flat plate auxiliary member 322 having a ring structure has an inner shape 323 the same as the outer shape of the flat plate 317 and has a circular outer shape 324. In this way, when the central portion 326 of the flat plate 325 in which the flat plate 317 and the flat plate auxiliary member 322 are bonded at the broken-line portion shown in the drawing is elastically deformed so as to appropriately extend in the direction of the arrow shown in the drawing, that is, in the normal direction of the flat plate 325, it is possible to form the three-dimensional flat plate 316 forming the neck portion from the two-dimensional flat plate 325. The flat plate 316 has exactly the same shape as the flat plate 203C of the stirring body 201C shown in FIG. 12(B), and has the same features and effects as the flat plate 203C of the stirring body 201C.

As shown in FIG. 25(B), in the three-dimensional flat plate 327 forming the neck portion, a circular through-hole is provided in the central portion 329 of the flat plate 328, and four cuts 330 are formed so as to extend in a spiral form about 540 degrees around the central portion 329 from the outer-peripheral-side ends 331 located in the outer peripheral edge of the flat plate 328 provided around the central portion 329 of the flat plate 328 at intervals of 90 degrees toward the inner-peripheral-side ends 332 located near the central portion 329 and provided around the central portion 329 at intervals of 90 degrees. Further, the flat plate auxiliary member 333 having a ring structure has a circular inner shape 334 and a circular outer shape 335. In this way, when the central portion 337 of the flat plate 336 in which the flat plate 328 and the flat plate auxiliary member 333 are bonded at the broken-line portion shown in the drawing is elastically deformed so as to appropriately extend in the direction of the arrow shown in the drawing, that is, in the normal direction of the flat plate 336, it is possible to form the three-dimensional flat plate 327 forming the neck portion from the two-dimensional flat plate 336. The flat plate 327 has a shape similar to that of the flat plate 203C of the stirring body 201C shown in FIG. 12(B), and has substantially the same features and effects as the flat plate 203C of the stirring body 201C.

According to the flat plate 316 and 327 of the aforementioned stirring body, when the flat plates 317 and 328 and the flat plate auxiliary members 322 and 333 are appropriately combined respectively and elastically deformed by a predetermined physical means to appropriately form a plurality of different deformed forms, it is possible to form a deformed form the same as or similar to that of the flat plates 203A, 203B, 203C, and 203D as shown in FIGS. 10 to 13. Thus, the flat plates 317 and 328 can provide features and effects the same as or similar to those of the flat plates 203A, 203B, 203C, and 203D of the stirring bodies 201A, 201B, 201C, and 201D shown in FIGS. 10 to 13.

### (Example of Cut in Flat Plate in the second Embodiment of Stirring body)

In the flat plate 203A as shown in FIG. 10, four spiral cuts 202 are formed in the flat plate 203A, but the number and shape of the cuts 202 are not particularly limited. FIG. 26 illustrates embodiments of various cuts of the flat plate of the stirring body according to the present invention. The flat plates 338, 343, 348, and 355 shown in FIG. 26 are made of an elastically deformable material.

In the flat plate 338 shown in FIG. 26(A), the spiral cut is modified to two slit-shaped cutouts 339. The two cutouts 339 are formed so as to extend in a spiral form about 360 degrees around the central portion 340 from two outer-peripheral-side ends 341 located inside the outer peripheral edges of a circular flat plate 338 having a wavy shape formed by connecting combinations of circular arcs and straight lines in series and provided around the central portion 340 at intervals of 180 degrees toward two inner-peripheral-side ends 342 located near the central portion 340 and provided around the central portion 340 at intervals of 180 degrees.

In the flat plate 343 shown in FIG. 26(B), four cuts 344 formed by connecting combinations of straight lines in series are formed so as to extend in a rectangular spiral form about 360 degrees around the central portion 345 from four outer-peripheral-side ends 346 located inside the vicinity of the corners of the outer peripheral edges of the square flat plate 343 and provided around the central portion 345 at intervals of 90 degrees toward four inner-peripheral-side ends 347 located near the central portion 345 and provided around the central portion 345 at intervals of 90 degrees.

In the flat plate 348 shown in FIG. 26(C), four cuts 349 formed by connecting combinations of circular arcs in series are formed so as to extend to the intermediate portion 353 in a spiral form about 360 degrees around the central portion 350 from four outer-peripheral-side ends 351 located inside the vicinity of the outer peripheral edges of the regular hexagonal flat plate 348 toward four inner-peripheral-side ends 352 located near the central portion 350 and provided around the central portion 350 at intervals of 90 degrees and are formed so as to extend in a spiral form about 360 degrees around the central portion 350 from the intermediate portion 353 toward the inner-peripheral-side ends 352 in the direction opposite to the spiral direction of the cuts 349 extending from the outer-peripheral-side ends 351 to the intermediate portion 353. Further, the outer peripheral edge of the flat plate 348 is bent toward the central portion 350 side with the broken-line portion shown in the drawing as the base point, whereby six flaps 354 are formed on the outer peripheral portion of the flat plate 348.

In the flat plate 355 shown in FIG. 26(D), two cuts 356 formed by connecting combinations of circular arcs in series are formed so as to extend in a spiral form about 360 degrees around the central portion 357 from two outer-peripheral-side ends 358 located inside the vicinity of the outer peripheral edges of an elliptical flat plate 355 and provided around the central portion 357 at intervals of 180 degrees toward two inner-peripheral-side ends 359 located near the central portion 357 and provided around the central portion 357 at intervals of 180 degrees, and a plurality of circular through-holes 360 are arranged.

According to the flat plates 338, 343, 348, and 355 of the aforementioned stirring body, when the spiral cuts formed in the flat plate 338 are modified as two slit-shaped cutouts 339, the cutouts function as a flat plate displacement amount adjustment structure that adjusts a blade width of the blade portion by elastic deformation, an expansion/contraction movement width, and a rotational movement width according to the shape of the cutout 339 to form a plurality of deformed forms with different displacement amounts reversibly. The flat plate 338 having a wavy shape can generate a plurality of flows accompanied by a plurality of small vortex flows. Since the cuts 344 formed in the square flat plate 343 are formed by connecting combinations of straight lines in series, it is possible to form a spiral cut shape that follows the shape of a polygonal flat plate. Due to the flaps 354 and the cuts 349 formed by being connected in series by a combination of right-handed winding and left-handed winding provided in the flat plate 348, it is possible to generate a plurality of flows accompanied by a vertical convection flow. Furthermore, due to the plurality of through-holes 360 arranged in the elliptical flat plate 355, it is possible to generate a plurality of flows accompanied by a plurality of large and small vortex flows and generate a circulating flow in a highly viscous liquid. In this way, for example, when the flat plates 338, 343, 348, and 355 are used in place of the flat plate 203E of the stirring device 218 shown in FIG. 14, since a plurality of different deformed forms can be formed reversibly by elastic deformation, it is possible to generate a circulating flow in a fluid medium having high and low viscosity. Moreover, it is possible to generate a complex vertical reflux in the liquid accompanied by various flows such as a swirling flow, a vertical convection flow, and a suction flow to stir a fluid medium. Thus, it is possible to provide a stirring blade that can adjust the displacement amounts of the flat plates 338, 343, 348, and 355 by elastic deformation to appropriately form deformed forms reversibly to adjust the flow of a stirring flow and that can be manufactured easily and to provide a stirring device including the stirring blade.

### (Third Embodiment of Stirring body)

FIG. 27 shows a third embodiment of the stirring body of the present invention. In the stirring bodies 401A, 401B, 401C, and 401D of the present embodiment, the central portion 404 of the flat plates 403A, 403B, 403C, 403D made of a material that can be plastically deformed by the shaft member 408 is supported, and, a plurality of Archimedean spiral or spiral cuts 402 extending outward from the vicinity of the central portion 404 so as not to touch the outer peripheral edge of the flat plates 403A, 403B, 403C, and 403D are formed around the central portion 404 at equal intervals. In this way, a plurality of spiral blade portions 407A, 407B, 407C, and 407D formed by the cut 402 are arranged at predetermined angular intervals in the circumferential direction around the central portion 404. The stirring bodies 401B, 401C, and 401D shown in FIGS. 28 to 30 show three different deformed forms having a multiple-spiral structure formed by plastically deforming the flat plate 403A of the stirring body 401A shown in FIG. 27. The stirring bodies 401B, 401C, and 401D shown in FIGS. 28 to 30 are the same as the stirring body 401A of the present embodiment shown in FIG. 27 except that the flat plate is plastically deformed to form different deformed forms.

As shown in FIG. 27(A), in the stirring body 401A, four cuts 402 are formed so as to extend in a spiral form about 540 degrees around the central portion 404 from the outer-peripheral-side ends 405 located inside the outer peripheral edges of the circular flat plate 403A and provided around the central portion 404 at intervals of 90 degrees toward the inner-peripheral-side ends 406A located near the central portion 404 and provided around the central portion 404 at intervals of 90 degrees. That is, the four blade portions 407A are arranged at positions rotated by 90 degrees in the circumferential direction around the central portion 404 with respect to the adjacent blade portions 407A, and are formed so as to extend in a spiral form about 540 degrees around the central portion 404. The outer-peripheral-side end 405 of the cut 402 is preferably formed so as to be separated from the outer peripheral edge toward the central portion 404 by a range of 5 to 10% of the diameter of the flat plate 403A. Moreover, the inner-peripheral-side end 406A of the cut 402 is preferably formed so as to be separated from the center of the flat plate 403A toward the outer peripheral edge by a range of 5 to 10% of the diameter of the flat plate 403A.

As shown in FIGS. 27(B) and 27(C), in the flat plate 403A of the stirring body 401A, the shaft member 408 is bonded to the central portion 404, so that the stirring body 401A is supported with respect to the shaft member 408. In this way, the blade portion 407A formed by the four cuts 402 is closed to form a two-dimensional flat plate 403A.

As shown in FIGS. 28(A) and 28(B), in the flat plate 403B of the stirring body 401B, the central portion 404 side of the flat plate 403A of the stirring body 401A shown in FIG. 27(B) is moved (that is, raised) to the extension position 410 shown in FIG. 28(A) in the direction of the arrow 409, that is, in the normal direction of the flat plate 403A, and the central portion 404 of the flat plate 403A shown in FIGS. 27(A) and 27(B) is moved (that is, raised) so that the blade portion 407A is plastically deformed so as to extend while being twisted. In this way, a three-dimensional flat plate 403B having the truncated cone-shaped blade portion 407B as shown in FIGS. 28(A) and 28(B) is formed.

Further, as shown in FIG. 28(C), in the flat plate 403B of the stirring body 401B, the central portion 404 side of the flat plate 403A of the stirring body 401A shown in FIG. 27(B) is raised to the extension position 410 shown in FIG. 28(A) in the direction of the arrow 409, and the blade portion 407A shown in FIG. 27(A) is plastically deformed so as to extend while being twisted so as to rotate in the direction of the cut 402 facing the central portion 404 of the flat plate 403A, that is, in the right-handed winding direction. In this way, the inner-peripheral-side end 406A of the flat plate 403A of the stirring body 401A shown in FIG. 27(A) rotates around the central portion 404 about 20 degrees in the right-handed winding direction and rotates to the position of the inner-peripheral-side end 406B shown in FIG. 28(C). In this way, a three-dimensional flat plate 403B having the truncated cone-shaped blade portion 407B is formed as shown in FIGS. 2(A) and 28(B).

As shown in FIGS. 29(A) and 29(B), in the flat plate 403C of the stirring body 401C, the central portion 404 side of the flat plate 403B of the stirring body 401B shown in FIG. 28(A) is further raised from the extension position 410 to the extension position 411 shown in FIG. 29(A), the central portion 404 of the flat plate 403B shown in FIGS. 28(A) and 28(B) is further raised, and the blade portion 206B is plastically deformed while being twisted accompanied by extension movement, outward spreading (hereinafter referred to as "spreading movement"), and convergence toward the center of the flat plate 403B (hereinafter referred to as "converging movement"). In this way, as shown in FIGS. 29(A) and 29(B), a three-dimensional flat plate 403C having a blade portion 407C that forms a neck portion 412A having a neck shape and creates a singular point 413 at which the plurality of blade portions 407C converges according to the strength of pressure is formed.

Further, as shown in FIG. 29(C), in the stirring body 201C, the central portion 404 side of the flat plate 403B of the stirring body 401B shown in FIG. 28(A) is further raised from the extension position 410 to the extension position 411 shown in FIG. 29(A), and the blade portion 407B shown in FIG. 28(C) is plastically deformed while being twisted so as to further rotate in the right-handed winding direction accompanied by extension movement, spreading movement, and converging movement. In this way, the inner-peripheral-side end 406A shown in FIG. 27(A) is rotated about 45 degrees in the right-handed winding direction around the central portion 404 as the base axis and is rotated to the position of the inner-peripheral-side end 406C of the flat plate 403A shown in FIG. 29(C). In this way, a three-dimensional flat plate 403C having the blade portion 407C forming the neck portion 412A is formed as shown in FIGS. 29(A) and 29(B).

Further, as shown in FIGS. 30(A) and 30(B), in the flat plate 403D of the stirring body 401D, the central portion 404 side of the flat plate 403C of the stirring body 401C shown in FIG. 29(A) is rotated about 180 degrees around the central portion 404 of the flat plate 403C as the base axis in the twisting direction 414 shown in FIG. 30(A) from the extension position 411, and the blade portion 407C is rotated while being twisted and the blade portion 407C around the neck portion 412A is plastically deformed accompanied by reverse movement. In this way, a complex three-dimensional flat plate 403D having a plurality of wavy blade portions 407D of which the front and back surfaces are exposed when seen from one direction is formed as shown in FIGS. 30(A) and 30(B).

As shown in FIG. 30(C), in the flat plate 403D of the stirring body 401D, the central portion 404 side of the flat plate 403C of the stirring body 401C shown in FIG. 29(A) is twisted (that is, rotated) about 180 degrees in the direction opposite to the direction of the cut 402 facing the central portion 404 of the flat plate 403C, that is, the left-handed twisting direction 414 shown in FIGS. 30(A) and 30(B). Moreover, the blade portion 407C shown in FIG. 29(C) is plastically deformed while being twisted in the left-handed winding direction accompanied by rotational movement and reverse movement. In this way, the inner-peripheral-side end 406C of the flat plate 403C of the stirring body 401C shown in FIG. 29(C) is rotated about 180 degrees around the central portion 404 as the base axis in the left-handed winding direction and is rotationally moved to the position of the inner-peripheral-side end 406D shown in FIG. 30(C). In this way, a three-dimensional flat plate 403D having a plurality of wavy blade portions 407D of which the front and back surfaces are exposed when seen from one direction is formed as shown in FIGS. 30(A) and 30(B).

According to the aforementioned stirring bodies 401A, 401B, 401C, and 401D, since the shaft member 408 is bonded to the central portions 4 of the flat plates 403A, 403B, 403C, and 403D, the stirring bodies 401A, 401B, 401C, and 401D are supported with respect to the shaft member 408. When the flat plates 403A, 403B, 403C, and 403D made of a plastically deformable material are plastically deformed by a predetermined physical means, it is possible to form a deformed form from the two-dimensional flat plate 403A having the blade portion 407A closed by the four spiral cuts 402 to the three-dimensional flat plate 403B having the truncated cone-shaped blade portion 740B in which the blade portion 407A is plastically deformed while being twisted accompanied by a plurality of operation movements including extension movement. Moreover, it is possible to form a deformed form from the state of the three-dimensional flat plate 403B to the three-dimensional flat plate 403C having the blade portion 407C that forms the neck portion 412A and creates the singular point 413 according to the strength of pressure in which the blade portion 407B is further plastically deformed while being twisted accompanied by a plurality of operation movements including extension movement, spreading movement, and converging movement toward the center. Furthermore, it is possible to form a deformed form from the state of the three-dimensional flat plate 403C to the three-dimensional flat plate 403D having the plurality of wavy blade portions 407D of which the front and back surfaces are exposed when seen from one direction in which the central portion 404 of the flat plate 403C is rotated about 180 degrees in the twisting direction 414 and the blade portion 407C around the neck portion 412A is plastically deformed while being twisted accompanied by a plurality of operation movements including rotational movement and reverse movement. In this way, the cut 402 functions as an expansion/contraction movement structure, a rotational movement structure, a convergence movement structure, and a twist/reverse movement structure of the blade portions 407A, 407B, 407C, and 407D. The cut 402 also functions as a displacement amount adjustment structure of the flat plates 407A, 407B, 407C, and 407D that adjusts the expansion/contraction movement width, the rotational movement width, and the spreading/converging movement width of the blade portions 407A, 407B, 407C, and 407D according to the length of the cut 402 extending around the central portion 404, that is, the angle extending around the central portion 404 (hereinafter referred to as a "circumferential angle") to form a plurality of deformed forms with different displacement amounts by plastic deformation. The cut 402 also functions as a shape deformation structure of the flat plates 403A, 403B, 403C, and 403D forming the deformed form. Further, since the flat plates 403A, 403B, 403C, and 403D have a circular outer shape without a tip wing, the flat plate functions as a container damage protection structure that prevents the inside of a container such as a stirring tank for stirring a stirring substance from being damaged. Therefore, for example, when a plastically deformable material is used as the material of the flat plates 403A, 403B, 403C, and 403D of the aforementioned stirring bodies 401A, 401B, 401C, and 401D of the present embodiment described above, the flat plates 403A, 403B, 403C, and 403D having different deformed forms are appropriately formed by adjusting the displacement amount by plastic deformation, and the shaft member 408 bonded to each of the central portions 404 of the stirring bodies 401A, 401B, 401C, and 401D is reciprocated by a predetermined physical means, it is possible to stir a fluid medium in an extremely complex and unsteady manner accompanied by a plurality of flows such as a vortex flow, a separation flow, a vertical convection flow, a circulating flow, and a turbulent flow. Moreover, when the shaft member 8 bonded to each of the central portions 404 of the stirring bodies 401A, 401B, 401C, 401D is rotated by a predetermined physical means, it is possible to generate a complex vertical reflux in the liquid accompanied by a plurality of flows such as a swirling flow, a vertical convection flow, and a suction flow and to satisfactorily stir a fluid medium without any mixing unevenness. In this way, it is possible to provide a low-cost stirring blade that has a simple configuration, and prevents damages in the stirring tank, and that can be manufactured easily, the stirring blade being capable of adjusting the displacement amounts of the flat plates 403A, 403B, 403C, and 403D of the stirring bodies 401A, 401B, 401C, 401D by plastic deformation to appropriately form a deformed form and adjust the flow of a stirring flow.

The position of the shaft member 408 that supports the aforementioned stirring bodies 401A, 401B, 401C, and 401D is not limited to the central portion 404 of each of the flat plates 403A, 403B, 403C, and 403D. Although not shown in the drawing, the position of the shaft member 408 may be bonded to the outer peripheral portion or the central portion and the outer peripheral portion of each of the flat plates 403A, 403B, 403C, and 403D. The position of the shaft member 408 that supports the aforementioned stirring bodies 401A, 401B, 401C, and 401D is not particularly limited as long as it can support the flat plate 403A, 403B, 403C, and 403D.

### (Tenth Embodiment of Stirring Device)

The stirring body according to the present embodiment includes a flat plate in which a plurality of spiral blade portions extending from a central portion is formed by a spiral cut that does not contact an outer peripheral portion. A flat plate made of a plastically deformable material is plastically deformed to appropriately form deformed forms with different displacement amounts and is reciprocated. In this way, the stirring body can be used as a stirring blade of a stirring device that stirs a fluid medium in an unsteady manner. Hereinafter, a tenth embodiment of the stirring device including the stirring body according to the present invention will be described with reference to FIG. 31. A flat plate 403E of a stirring body 417 provided in a stirring device 415 of the present embodiment shown in FIG. 31 is made of a plastically deformable material and has exactly the same shape as the flat plate 403C shown in FIG. 29, that is, the same one is used. The stirring body 417 shown in FIG. 31 is the same as the stirring body 401C shown in FIG. 29. Thus, the detailed description thereof will be omitted here.

As shown in FIG. 31, the stirring device 415 includes a stirring tank 416A containing a liquid and having a volume of 300 mL, a circular flat plate 403E of the stirring body 417, a shaft member 419A bonded to a central portion 418 of the flat plate 403E, a top plate portion 420A, a rubber seal 421A having a bellows structure, and a driving device 422A in which a brushless motor is provided and which reciprocates in the vertical direction. As shown in FIG. 31(A) and 31(B), in the stirring device 415, the shaft member 419A as the driving shaft of the driving device 422A vertically suspended in the stirring tank 416A passes through a center-side opening of the top plate portion 420A and is connected to the seal 421A so as to be able to follow reciprocation. Thus, the inside of the stirring tank 416A is airtightly provided.

As shown in FIG. 31(C), in the stirring device 415, the driving device 422A is driven by a predetermined physical means to cause the shaft member 419A to reciprocate in the direction of the arrow 423, and the flat plate 403E of the stirring body 417 vertically suspended in the stirring tank 416A moves vertically. In this way, a plurality of flows is generated around the neck portion 412B accompanied by a vortex flow based on a separation flow. That is, FIG. 31(C) schematically shows the movement of the stirring flow in the stirring tank 416A by the stirring device 415 shown in FIG. 31(A). The broken-line flat plate 403E shown in FIG. 31(C) shows the flat plate 403E at the substantially uppermost position of the movable range in which the shaft member 419A reciprocates.

According to the stirring device 415 including the aforementioned stirring body 17, when the driving device 422A provided with the brushless motor is driven by a predetermined physical means, and the shaft member 419A as the driving shaft of the driving device 422A is reciprocated in the direction of the arrow 423, since the shaft member 419A is bonded to the central portion 418 of the circular flat plate 403E, the flat plate 403E of the stirring body 417 of the present embodiment made of a plastically deformable material can be moved vertically in the stirring tank 416A. In this way, the spiral blade portions 407E reciprocate in the vertical direction in the liquid in the stirring tank 416A and generate a plurality of flows accompanied by a vortex flow around the adjacent blade portions 407E and the gaps therebetween. Moreover, the central portion 418 of the flat plate 403E moves vertically to generate a vertical convection flow. Further, the blade portion 407E having the neck portion 412B moves vertically to generate a vortex flow based on a separation flow around the neck portion 412B. Furthermore, the outer peripheral portion of the flat plate 403E moves vertically to generate a large vortex flow around the outer-peripheral-side end. In this way, the stirring device functions as a stirring device having a stirring blade capable of generating a plurality of flows such as a vortex flow, a separation flow, a vertical convection flow, a circulating flow, and a turbulent flow and stirring a fluid medium in an extremely complex and unsteady manner with low shear. Therefore, for example, when a physicochemical environment suitable for proliferation and metabolism of cultured cells, differentiation and maturation of megakaryocytes, polynuclearization of megakaryocytes, production of platelets, and maintenance of bioactivity of platelets is established in the aforementioned stirring device 415 and a culture solution is stirred, it is possible to provide a stirring blade that can stir a culture solution in a more complex and unsteady manner with low shear accompanied by a plurality of flows and that can be manufactured easily, as compared with conventional unsteady stirring required for stirring culture of culturing megakaryocyte cells derived from human iPS cells to produce a large amount of platelets and to provide a stirring culture device including the stirring blade.

### (Eleventh Embodiment of Stirring Device)

A third embodiment of the stirring body according to the present invention includes a flat plate in which a plurality of spiral blade portions extending from a central portion is formed by a spiral cut that does not contact an outer peripheral portion. A flat plate made of a plastically deformable material is plastically deformed to appropriately form deformed forms with different displacement amounts and is reciprocated. In this way, the stirring body can be used as a stirring blade of a stirring device capable of stirring a fluid medium in an unsteady manner and adjusting the flow of a stirring flow. Hereinafter, the eleventh embodiment of the stirring device including the stirring body according to the present invention will be described with reference to FIGS. 32 and 33. The flat plates 403F and 403G of the stirring bodies 425 and 429 of the present embodiment shown in FIGS. 32 and 33 are made of a plastically deformable material, and two different deformed forms other than the flat plate 403E of the stirring body 417 shown in FIG. 31 are formed by plastic deformation. The stirring bodies 425 and 429 shown in FIGS. 32 and 33 have exactly the same shape as the stirring bodies 401B and 401D shown in FIGS. 28 and 30, that is, the same ones are used. The stirring devices 424 and 428 of the present embodiment shown in FIGS. 32 and 33 are the same as the stirring device 415 shown in FIG. 31 except for the flat plates 403F and 403G of the stirring bodies 425 and 429 of the present embodiment. Thus, the detailed description thereof will be omitted here.

As shown in FIG. 32, the stirring device 424 includes a stirring tank 416B containing a liquid and having a volume of 300 mL, a flat plate 403F of the stirring body 425, a shaft member 419B bonded to a central portion 426 of the flat plate 403F, a top plate portion 420B, a rubber seal 421B having a bellows structure, and a driving device 422B in which a brushless motor is provided and which reciprocates. Since the flat plate 403F of the stirring body 425 is the same as the flat plate 403B of the stirring body 401B shown in FIG. 28, the detailed description thereof will be omitted here.

As shown in FIGS. 32(A) and 32(B), in the stirring device 424, the shaft member 419B as the driving shaft of the driving device 422B is bonded to the central portion 426 of the flat plate 403F of the stirring body 425 having the truncated cone-shaped blade portion 407F, passes through the center-side opening of the top plate portion 420B and is connected to the seal 421B so as to be able to follow reciprocation. Thus, the inside of the stirring tank 416B is airtightly provided. The driving device 422B is driven by a predetermined physical means to cause the shaft member 419B to reciprocate in the direction of the arrow 427, and the flat plate 403F of the stirring body 425 vertically suspended in the stirring tank 416B moves vertically. In this way, the blade portions 407 generate a plurality of flows accompanied by a plurality of vortex flows around the adjacent blade portions 407F and the gaps therebetween. That is, FIG. 32(A) schematically shows the movement of the stirring flow in the stirring tank 416B by the stirring device 424. The broken-line flat plate 403F shown in FIG. 32(A) shows the flat plate 403F at the substantially uppermost position of the movable range in which the shaft member 419B reciprocates.

As shown in FIG. 33, the stirring device 428 includes a stirring tank 416C containing a liquid and having a volume of 300 mL, a flat plate 403G of the stirring body 429, a shaft member 419C bonded to a central portion 430 of the flat plate 403G, a top plate portion 420C, a rubber seal 421C having a bellows structure, and a driving device 422C in which a brushless motor is provided and which reciprocates. Since the flat plate 403G of the stirring body 429 is the same as the flat plate 403D of the stirring body 401D shown in FIG. 30, the detailed description thereof will be omitted here.

As shown in FIGS. 33(A) and 33(B), in the stirring device 428, the shaft member 419C as the driving shaft of the driving device 422C is bonded to the central portion 430 of the flat plate 403G of the stirring body 429 having the plurality of wavy blade portions 407G of which the front and back surfaces are exposed when seen from one direction, passes through the center-side opening of the top plate portion 420C and is connected to the seal 421C so as to be able to follow reciprocation. Thus, the inside of the stirring tank 416B is airtightly provided. The driving device 422C is driven by a predetermined physical means to cause the shaft member 419C reciprocate in the direction of the arrow 431, and the flat plate 403G of the stirring body 429 vertically suspended in the stirring tank 416C moves vertically. In this way, the plurality of wavy blade portions 407G of which the front and back surfaces are exposed when seen from one direction moves vertically, and a plurality of flows accompanied by a large vortex flow around the curved portion of the blade portion 407G is generated. That is, FIG. 33(A) schematically shows the movement of the stirring flow in the stirring tank 416C by the stirring device 428. The broken-line flat plate 403G shown in FIG. 33(A) shows the flat plate 403G at the substantially uppermost position of the movable range in which the shaft member 419C reciprocates.

According to the stirring devices 424 and 428 including the aforementioned stirring bodies 425 and 428 described above, when the driving devices 422B and 422C provided with the brushless motor are driven by a predetermined physical means so that the shaft members 419B and 419C as the driving shafts of the driving devices 422B and 422C reciprocate in the directions of the arrows 427 and 431 shown in FIGS. 32(A) and 33(A), since the shaft members 419B and 419C are bonded to the central portions 426 and 430 of the circular flat plates 403F and 403G, the flat plates 403F and 403G of the stirring bodies 425 and 429 of the present embodiment made of a plastically deformable material can be moved vertically in the stirring tanks 416B and 416C. In this way, in the liquid in the stirring tank 416B, due to the three-dimensional flat plate 403F having the truncated cone-shaped blade portion 407F, the spiral blade portion 407F reciprocates in the vertical direction in the liquid in the stirring tank 416B. The blade portions 407F generate a plurality of vortex flows around the adjacent blade portions 407F and the gaps therebetween. The central portion of the flat plate 403F moves vertically to generate a vertical convection flow. The outer peripheral portion of the flat plate 403F moves vertically to generate a large vortex flow around the outer-peripheral-side end. Due to the complex three-dimensional flat plate 403G having the plurality of wavy blade portions 407G of which the front and back surfaces are exposed when seen from one direction, the spiral blade portions 407G reciprocate in the vertical direction, and the blade portions 407G generate a plurality of vortex flows around the adjacent blade portions 407G and the gaps therebetween. The central portion of the flat plate 403G moves vertically to generate a vertical convection flow. The plurality of wavy blade portions 407G of which the front and back surfaces are exposed when seen from one direction moves vertically to generate a large vortex flow around the curved portion of the blade portion 407G. In this way, the stirring device functions as a stirring device including a stirring blade capable of generating a plurality of flows in the liquid such as large and small vortex flows, a separation flow, a vertical convection flow, a circulating flow, and a turbulent flow to stir a fluid medium in an extremely complex and unsteady manner with low shear. Therefore, for example, when a physicochemical environment suitable for proliferation and metabolism of cultured cells, differentiation and maturation of megakaryocytes, polynuclearization of megakaryocytes, production of platelets, and maintenance of bioactivity of platelets is established in the aforementioned stirring devices 424 and 428 and a culture solution is stirred, it is possible to provide a stirring blade that can stir the culture solution in a complex and unsteady manner with low shear accompanied by a plurality of flows and that can be manufactured easily, as compared with conventional unsteady stirring required for stirring culture of culturing megakaryocyte cells derived from human iPS cells to produce a large amount of platelets and to provide a stirring device including the stirring blade.

Further, according to the stirring devices 415, 424, and 428 including the stirring bodies 417, 425, and 428 shown in FIGS. 31 to 33, the driving devices 422A, 422B, and 422C are driven by a predetermined physical means so that the shaft members 419A, 419B, and 419C as the driving shafts of the driving devices 422A, 422B, and 422C are reciprocated in the directions of the arrows 423, 427, and 431 shown in FIGS. 31 to 33, and the flat plates 403E, 403F, and 403G of the stirring bodies 417, 425, and 429 having the blade portions 407E, 407F, and 407G are reciprocated in the vertical direction. Thus, it is possible to generate a plurality of different flows in the liquid in the stirring tank 416A, 416B, and 416C to stir a fluid medium in an extremely complex and unsteady manner with low shear. That is, the stirring devices function as a stirring blade and a stirring device including the stirring blade that can be manufactured easily and that can adjust the flow of a stirring flow by plastically deforming the flat plates 403E, 403F, and 403G by a predetermined physical means to appropriately form deformed forms with different displacement amounts. Therefore, for example, when a physicochemical environment suitable for proliferation and metabolism of cultured cells, differentiation and maturation of megakaryocytes, polynuclearization of megakaryocytes, production of platelets, and maintenance of bioactivity of platelets is established in the aforementioned stirring devices 415, 424, and 428 and a culture solution is stirred, it is possible to provide a stirring blade that can be manufactured easily and a stirring culture device including the stirring blade capable of stirring a culture solution in a more complex and unsteady manner with low shear accompanied by a plurality of flows and adjusting the displacement amount of the flat plate by plastic deformation to appropriately form different deformed forms and adjust the flow of a stirring flow, as compared with conventional unsteady stirring required for stirring culture of culturing megakaryocyte cells derived from human iPS cells to produce a large amount of platelets.

### (Twelfth Embodiment of Stirring Device)

A third embodiment of the stirring body according to the present invention includes a flat plate in which a plurality of spiral blade portions extending from a central portion is formed by a spiral cut that does not contact an outer peripheral portion. A flat plate made of a plastically deformable material is plastically deformed to appropriately form deformed forms with different displacement amounts and is rotated. In this way, the stirring body can be used as a stirring device capable of generating a complex vertical reflux to satisfactorily stir a fluid medium without any mixing unevenness and adjusting the flow of a stirring flow. Hereinafter, a twelfth embodiment of the stirring device including the stirring body according to the present invention will be described with reference to FIG. 34. The flat plate 434 of the stirring body 433 provided in the stirring device 432 of the present embodiment shown in FIG. 34 is made of a plastically deformable material, and four cuts 435 are formed so as to extend in a spiral form about 540 degrees around the central portion 436 from the outer-peripheral-side ends 437 located inside the outer peripheral edge of the circular flat plate 434 and provided around the central portion 436 of the flat plate 434 at intervals of 90 degrees toward the inner-peripheral-side ends 438 located near the central portion 436 and provided around the central portion 436 at intervals of 90 degrees. The blade portion 439 of the flat plate 434 has exactly the same shape as the blade portion 407C of the flat plate 403C shown in FIG. 29, and the flat plate 434 shown in FIG. 34 is the same as the flat plate 403C shown in FIG. 29 except that a bar magnet 440 is provided on the outer peripheral portion.

As shown in FIGS. 34(A) to 34(C), the stirring device 432 includes a flat plate 434 of a stirring body 433, a shaft member 441 bonded to a central portion 436 of the flat plate 434, a center cap 443 having a bearing 442, two side arm caps 444, a glass stirring body tank 445 with three openings, containing liquid and having a volume of 500 mL, and a magnetic stirring driving device 447 having a motor 446. Two bar magnets 440 are provided around the central portion 436 of the flat plate 434 at intervals of 180 degrees and arranged in the outer peripheral portion of the flat plate 434. The shaft member 441 rotatably connected to the bearing 442 is bonded to the central portion 436 of the flat plate 434. Thus, the flat plate 434 of the stirring body 433 having the blade portion 439 forming such a neck portion as shown in FIG. 34(A) is formed. Further, the stirring tank 445 is disposed on the magnetic stirring driving device 447 such that the inside of the stirring tank 445 is airtightly provided by an O-ring provided in the center cap 443 and the two side arm caps 444, and the flat plate 434 of the stirring body 443 is sealed with polytetrafluoroethylene (registered trademark: Teflon). FIG. 34(A) shows a state in which the motor 446 provided in the magnetic stirring driving device 47 is driven by a predetermined physical means, and the flat plate 434 of the stirring body 433 vertically suspended in the stirring tank 445 is rotated in the liquid, whereby a plurality of flows is generated accompanied by large and small vortex flows, a separation flow, a vertical convection flow, a circulating flow, and a turbulent flow. That is, FIG. 34(A) schematically shows the movement of the stirring flow in the stirring tank 445 by the stirring device 432.

According to the stirring device 432 including the aforementioned stirring body 433 described above, when the shaft member 441 rotatably connected to the bearing 442 provided in the center cap 443 is bonded to the central portion 436 of the flat plate 434 including the bar magnet 440 sealed by polytetrafluoroethylene (registered trademark name: Teflon), and the motor 446 provided in the magnetic stirring driving device 447 is driven by a predetermined physical means, the flat plate 434 of the stirring body 433 made of a plastically deformable material can be rotated in the stirring tank 445 without contacting the driving unit of the magnetic stirring driving device 447 due to a magnetic force. In this way, it is possible to generate a complex vertical reflux in the liquid in the stirring tank 445 accompanied by a suction flow based on a suction force generated from the upper surface side near the outer peripheral portion of the flat plate 434 toward the central portion of the flat plate 434, a suction flow due to a suction force generated from the vicinity of the neck portion toward the central portion of the flat plate 34, a swirling flow, and a vertical convection flow to satisfactorily stir a fluid medium without any mixing unevenness. Thus, it is possible to provide a stirring blade and a stirring device including the stirring blade, the stirring blade being capable of plastically deforming the flat plate 434 by a predetermined physical means to appropriately form different deformed forms with different displacement amounts to adjust the flow of a stirring flow. Therefore, for example, when the flat plate 434 of the stirring body 433, the shaft member 441, the bearing 442, the center cap 443, the side arm cap 444, and the O-ring are processed with polytetrafluoroethylene (registered trademark: Teflon), and the motor 446 provided in the magnetic stirring driving device 447 (magnetic stirring body) is driven by a predetermined physical means, the flat plate 434 of the stirring body 433 including the bar magnet 440 rotates. In this way, it is possible to generate a complex vertical reflux in the liquid in the stirring tank 445 accompanied by a plurality of flows such as a suction flow based on a suction force generated from the upper surface side in the vicinity of the outer peripheral portion of the flat plate 434 toward the central portion of the flat plate 434, a suction flow based on a suction force generated from the vicinity of the neck portion toward the central portion of the flat plate 434, a swirling flow, and a vertical convection flow to satisfactorily stir a fluid medium without any mixing unevenness. Moreover, when the flat plate 434 is plastically deformed by a predetermined physical means to appropriately form different deformed forms with different displacement amounts and is sealed with polytetrafluoroethylene (registered trademark: Teflon), it is possible to adjust the flow of a stirring flow. Moreover, since such a sealing portion as in a direct drive-type stirring device is not required, the stirring device can be used as a stirring blade and a magnetic stirring device or a spinner flask including the stirring blade, which has no contamination (bacterial contamination) and is easy to clean. However, the flat plate 434 provided with the bar magnet 440 of the stirring body 433, the shaft member 441, the bearing 442, the center cap 443, the side arm cap 444, and the O-ring are not limited to being processed by polytetrafluoroethylene (registered trademark: Teflon). For example, they may be sealed with glass coating, polycarbonate, or the like. That is, the materials and processing methods of the flat plate 434 provided with the bar magnet 440 of the stirring body 433, the shaft member 441, the bearing 442, the center cap 443, the side arm cap 444, and the O-ring are not particularly limited. Further, the means for generating magnetization of the magnetic stirring driving device 447 provided in the stirring device 432 is not limited to magnets and electromagnets that generate magnetization by electricity may be used. That is, the means for generating the magnetization of the magnetic stirring driving device of the stirring device including the stirring body according to the present invention is not particularly limited.

### (Fourth Embodiment of Stirring body)

As shown in FIG. 29, in the flat plate 403C of the stirring body 401C, one flat plate is supported with respect to the shaft member 408. However, the number of flat plates of the stirring body according to the present invention is not limited to one, and for example, two or more flat plates may be combined. The number and configuration of the flat plates of the stirring body according to the present invention are not particularly limited. FIGS. 35 and 36 illustrate various embodiments of the stirring blade including the stirring body according to the present invention. The circular stirring bodies 401E, 401F, 452, 455, 458, and 461 included in the stirring blades 448, 450, 453, 456, and 459 shown in FIGS. 35 and 36 are made of a plastically deformable material and have exactly the same shape as any one of the stirring bodies 401A, 401B, 401C, and 401D shown in FIGS. 27 to 34 or a combination thereof. Thus, the detailed description thereof will be omitted here.

As shown in FIG. 35(A), the stirring blade 448 includes a shaft member 449 having U-shaped branch portions and stirring bodies 401E and 401F. The two branch portions of the shaft member 449 are provided around the central portions of the stirring bodies 401E and 401F at intervals of 90 degrees so that the outer peripheral portion sides of the stirring bodies 401E and 401F are fixed, and the stirring bodies 401E and 401F are arranged in a column as shown in the drawing. The stirring bodies 401E and 401F have exactly the same shape as the stirring body 401C shown in FIG. 29.

As shown in FIG. 35(B), the stirring blade 450 includes a shaft member 451 and a stirring body 452 in which the flat plates 403H and 4031 are bonded to each other at the outer peripheral portions thereof. The shaft member 451 is bonded to the central portion of the flat plate 403J so that the stirring body 452 is supported with respect to the shaft member 451 as shown in the drawing. The stirring body 452 has exactly the same shape as one in which the two stirring bodies 401C shown in FIG. 29 are bonded together at the outer peripheral portions thereof.

As shown in FIG. 36(A), the stirring blade 453 includes a shaft member 454 having U-shaped branch portions and a stirring body 455 in which the flat plates 403J and 403K are bonded to each other at the central portions thereof. The two branch portions of the shaft member 454 are provided around the central portions of the flat plates 403J and 403K at intervals of 180 degrees so that the outer peripheral portion sides of the flat plates 403J and 403K are fixed. Thus, the stirring body 455 is supported with respect to the shaft member 454 as shown in the drawing. The stirring body 455 has exactly the same shape as one in which the two stirring bodies 401C shown in FIG. 29 are bonded together at the central portions thereof.

As shown in FIG. 36(B), the stirring blade 456 includes a shaft member 457 and a stirring body 458 in which the flat plates 403L and 403M are bonded to each other at the outer peripheral portions thereof. The shaft member 457 is bonded to the central portion of the flat plate 403L so that the stirring body 458 is supported with respect to the shaft member 457 as shown in the drawing. The stirring body 458 has exactly the same shape as one in which the stirring body 401D shown in FIG. 30 and the stirring body 401B shown in FIG. 28 are bonded to each other at the outer peripheral portions thereof.

As shown in FIG. 36(C), the stirring blade 459 includes a shaft member 460 and a stirring body 461 in which the flat plate 403N is superimposed on the flat plate 403O so that they are bonded to each other at the outer peripheral portions thereof. The shaft member 460 is bonded to the central portion of the flat plate 403N, so that the stirring body 461 is supported with respect to the shaft member 460 as shown in the drawing. The flat plate 403N of the stirring body 461 has exactly the same shape as the flat plate 403C of the stirring body 401C shown in FIG. 29. The flat plate 403O of the stirring body 461 has exactly the same shape as the flat plate 403B of the stirring body 401B shown in FIG. 28 of which the dimensions are reduced by 30% at a magnification of 1:1.

In any of the aforementioned stirring bodies 401E, 401F, 452, 455, 458, and 461, the cut is formed so as not to contact the outer peripheral edges of the stirring bodies 401E, 401F, 452, 455, 458, and 461.

According to the aforementioned stirring bodies 401E, 401F, 452, 455, 458, and 461, a plurality of stirring bodies made of a plastically deformable material can be combined to form a plurality of different deformed forms by plastic deformation. Therefore, for example, when the stirring bodies 401E, 401F, 452, 455, 458, and 461 of the present embodiment are used in place of the stirring body 417 of the stirring device 415 shown in FIG. 33, since the stirring bodies 401E, 401F, 452, 455, 458, and 461 reciprocate, it is possible to generate a plurality of flows in the flow such as a circulating flow and a turbulent flow to stir a fluid medium in an extremely complex and unsteady manner with low shear. In this way, it is possible to provide a stirring blade that can be manufactured easily and a stirring device including the stirring blade, the stirring blade being capable of adjusting the displacement amounts of the stirring bodies 401E, 401F, 452, 455, 458, and 461 by plastic deformation to appropriately form a deformed form to adjust the flow of a stirring flow.

### (Example of Cut in Flat Plate in Third and Fourth Embodiments of Stirring body)

In the flat plate 403A of the stirring body as shown in FIG. 27, four spiral cuts 402 are formed in the flat plate 403A, but the number and shape of the cuts 402 are not particularly limited. FIG. 37 illustrates embodiments of various cuts of the flat plate of the stirring body according to the present invention. The flat plates 464, 470, 476, and 484 of the stirring bodies 462, 468, 474, and 482 shown in FIG. 37 are made of a plastically deformable material.

In the stirring body 462 shown in FIG. 37(A), the spiral cut is modified to two slit-shaped cutouts 463. The two cutouts 463 are formed so as to extend in a spiral form about 360 degrees around the central portion 465 from two outer-peripheral-side ends 466 located inside the outer peripheral edges of a circular flat plate 464 having a wavy shape formed by connecting combinations of circular arcs and straight lines in series and provided around the central portion 465 at intervals of 180 degrees toward two inner-peripheral-side ends 467 located near the central portion 465 and provided around the central portion 465 at intervals of 180 degrees.

In the flat plate 468 shown in FIG. 37(B), four cuts 469 formed by connecting combinations of straight lines in series are formed so as to extend in a rectangular spiral form about 360 degrees around the central portion 471 from four outer-peripheral-side ends 472 located inside the vicinity of the corners of the outer peripheral edges of the square flat plate 470 and provided around the central portion 471 at intervals of 90 degrees toward four inner-peripheral-side ends 473 located near the central portion 471 and provided around the central portion 471 at intervals of 90 degrees.

In the stirring body 474 shown in FIG. 37(C), four cuts 475 formed by connecting combinations of circular arcs in series are formed so as to extend to the intermediate portion 480 in a spiral form about 360 degrees around the central portion 477 from four outer-peripheral-side ends 478 located inside the vicinity of the outer peripheral edges of the regular hexagonal flat plate 476 toward four inner-peripheral-side ends 479 located near the central portion 477 and provided around the central portion 477 at intervals of 90 degrees and are formed so as to extend in a spiral form about 360 degrees around the central portion 477 from the intermediate portion 480 toward the inner-peripheral-side ends 479 in the direction opposite to the spiral direction of the cuts 475 extending from the outer-peripheral-side ends 478 to the intermediate portion 480. Further, the outer peripheral edge of the flat plate 476 is bent toward the central portion 477 side with the broken-line portion shown in the drawing as the base point, whereby six flaps 481 are formed on the outer peripheral portion of the flat plate 476.

In the flat plate 482 shown in FIG. 37(D), two cuts 483 formed by connecting combinations of circular arcs in series are formed so as to extend in a spiral form about 360 degrees around the central portion 485 from two outer-peripheral-side ends 486 located inside the vicinity of the outer peripheral edges of an elliptical flat plate 484 and provided around the central portion 485 at intervals of 180 degrees toward two inner-peripheral-side ends 487 located near the central portion 485 and provided around the central portion 485 at intervals of 180 degrees, and a plurality of circular through-holes 488 are arranged.

According to the aforementioned stirring bodies 462, 470, 474, and 482, when the spiral cuts formed in the flat plate 462 are modified as two slit-shaped cutouts 463, the cutouts function as a flat plate displacement amount adjustment structure that adjusts a blade width of the blade portion by plastic deformation, an expansion/contraction movement width, and a rotational movement width according to the shape of the cutout 463 to form a plurality of deformed forms with different displacement amounts. The flat plate 64 having a wavy shape can generate a plurality of flows accompanied by a plurality of small vortex flows. Since the cuts 469 formed in the square flat plate 470 are formed by connecting combinations of straight lines in series, it is possible to form a spiral cut shape that follows the shape of a polygonal flat plate. Due to the flaps 481 and the cuts 475 formed by being connected in series by a combination of right-handed winding and left-handed winding provided in the flat plate 476, it is possible to generate a plurality of flows accompanied by a plurality of large vortex flows. Furthermore, due to the plurality of through-holes 488 arranged in the elliptical flat plate 484, it is possible to generate a plurality of flows accompanied by a plurality of large and small vortex flows and generate a circulating flow in a highly viscous liquid. In this way, for example, when the stirring bodies 462, 470, 474, and 482 are used in place of the stirring body 417 of the stirring device 415 shown in FIG. 31, it is possible to generate a circulating flow in a fluid medium having high and low viscosity. Moreover, it is possible to stir a fluid medium in the liquid in an extremely complex and unsteady manner with low shear accompanied by various flows such as a vortex flow, a separation flow, a suction flow, an extrusion flow, a swirling flow, a vertical convection flow, a circulating flow, and a turbulent flow. Thus, it is possible to provide a stirring blade that can adjust the displacement amounts of the flat plates 464, 470, 476, and 484 of the stirring bodies 462, 470, 474, and 482 by plastic deformation to appropriately form deformed forms to adjust the flow of a stirring flow and that can be manufactured easily and to provide a stirring device including the stirring blade.

### (Thirteenth Embodiment of Stirring Device)

A fourth embodiment of the stirring body according to the present invention includes a flat plate in which a plurality of spiral blade portions extending from a central portion is formed by a spiral cut that does not contact an outer peripheral portion. A flat plate made of a plastically deformable material is plastically deformed to appropriately form deformed forms with different displacement amounts and is fixed in a pipeline. In this way, the stirring body can be used as a stirring blade of a pipe-type mixer that does not have a stirring tank (that is, a driving unit) capable of mixing and stirring a fluid medium. Hereinafter, the thirteenth embodiment of the stirring device including the stirring body according to the present invention will be described with reference to FIG. 38. The flat plates 403R and 403S of the stirring body 486 of the present embodiment shown in FIG. 38 are made of a plastically deformable material and have exactly the same shape as the flat plate 403C of the stirring body 401C shown in FIG. 29, that is, the same one is used. Thus, the detailed description thereof will be omitted here.

As shown in FIGS. 38(A) and 38(B), a mixer 489 includes a stirring body 490 in which three-dimensional flat plates 403P and 403Q having a neck portion are bonded to each other at the central portions thereof and a pipe 491 having a flange. The stirring body 490 is bonded in the pipe 491. In this way, the mixer 489 can be bonded to another pipe by screw-fastening using a gasket or the like and can be airtightly piped.

According to the mixer 489 provided with the aforementioned stirring body 490, when the stirring body 490 in which the flat plates 403P and 403Q made of a plastically deformable material are bonded to each other at the central portions thereof is bonded in the pipe 491 and a fluid medium is made to flow into the pipe 491 by a predetermined physical means, a swirling flow accompanied by a plurality of separation vortices is generated in the fluid medium in the pipe 491, and a mixing target fluid medium can be uniformly mixed. Therefore, for example, when the aforementioned mixer 489 is screw-fastened using a gasket and airtightly piped to an in-line fluid mixing device capable of mixing a plurality of fluid media, and the pump is driven by a predetermined physical means so that the fluid medium flows into the pipe 91, the stirring body 490 having a neck portion can generate a swirling flow accompanied by a plurality of separation vortices to uniformly mix a plurality of mixing target fluid media. Thus, it is possible to provide a stirring blade capable of atomizing air bubbles in a liquid and reducing labor, space, and energy and to provide a pipe-type mixer (that is, a static mixer) including the stirring blade, which does not have a driving unit. However, the fluid mixing by the mixer 489 of the present embodiment is not limited to liquid-liquid mixing, but may include gas-liquid mixing and various cases depending on whether the mixing target is soluble or insoluble, or has high viscosity or low viscosity. That is, the fluid mixing by the mixer 489 of the present embodiment is not particularly limited. Further, the method of bonding the mixer 489 of the present embodiment to another pipe is not limited to screw-fastening, and for example, welding, adhesion, fitting, and the like may be used. That is, the method of bonding the mixer 89 of the present embodiment to another pipe is not particularly limited.

### (Fourteenth Embodiment of Stirring Device)

A stirring body according to the present invention includes a flat plate in which a plurality of spiral blade portions extending from a central portion is formed by a spiral cut that does not contact an outer peripheral portion and which is made of an elastically deformable material, a support member that supports the outer peripheral portion of the flat plate, and a movable member that supports the outer peripheral portion and the central portion. The movable member is reciprocated in relation to the support member in a direction in which the outer peripheral portion and the central portion approach and separate from each other. Thus, a fluid medium can be stirred in an unsteady manner. Moreover, the support member is vibrated vertically so that a complex turbulent flow can be generated with extremely low shear and a fluid medium can be satisfactorily stirred without any mixing unevenness. Thus, the stirring body can be used as a stirring blade of a stirring device capable of adjusting the flow of a stirring flow. Hereinafter, a fourteenth embodiment of a stirring device including the stirring body according to the present invention will be described with reference to FIG. 39. The flat plate 03 of the stirring body 02 provided in the stirring device 01 of the present embodiment shown in FIG. 39 is the same as the flat plate 3D of the stirring body 19 shown in FIG. 4. Thus, the detailed description thereof will be omitted here.

As shown in FIG. 39, the stirring device 01 includes a stirring tank 04 containing a liquid and having a volume of 300 mL, a stirring body 02 including a circular flat plate 03 having a blade portion 016 made of an elastically deformable material, a movable member 05, and a support member 06, a top plate portion 07, a driving device 08 provided with a brushless motor and having a controller capable of adjusting a reciprocating distance in the vertical direction, and a low-frequency vibration device 09 that vibrates vertically. When the position of the movable member 05 is adjusted so that the low-frequency vibration device 09 vibrates vertically and the support member 06 vibrates vertically, the flat plate 03 vibrates vertically in a state in which a deformed form is appropriately formed by elastic deformation. The three-dimensional flat plate 03 forming the blade portion 010A having a neck portion has exactly the same shape as the flat plate 3C shown in FIG. 3. The three-dimensional flat plate 03 having the broken-line truncated cone-shaped blade portion 101B has exactly the same shape as the flat plate 3B shown in FIG. 2. The flat plate 03 having the blade portion 010A shows a deformed form at the substantially uppermost position 011 of the movable range in which the movable member 05 reciprocates. The flat plate 03 having the blade portion 010B show a deformed form at the substantially lowermost position 012 of the movable range in which the movable member 05 reciprocates.

As shown in FIGS. 39(A) and 39(B), in the stirring device 01, the top plate portion 07 has three through-holes through which the movable member 05 and the support member 06 pass. One set of ends of the two support members 06 pass through the through-holes of the top plate portion 07 and are bonded to the low-frequency vibration device 09. The other set of ends are provided around the central portion of the flat plate 03 at intervals of 180 degrees and connected to the position of the outer peripheral portion. Further, one end of the movable member 05 passes through the through-hole of the top plate portion 07 and is bonded to the driving device 08. The other end passes through the circular through-hole provided in the central portion 011 of the flat plate 03 to rotatably support the central portion 013 side of the flat plate 03 between two adjacent convex spherical portions provided at the tip of the movable member 05. In this way, the two support members 06, which are the driving shafts of the low-frequency vibration device 09, vibrate vertically in the direction of the arrow 014 so that the flat plate 03 vibrates vertically. Moreover, the movable member 05 which is the driving shaft of the driving device 08 is reciprocated in the direction of the arrow 015 so that the central portion side of the flat plate 03 reciprocates in the vertical direction. In this way, as shown in FIG. 39(A), the flat plate 03 is elastically deformed to the flat plate 03 forming the blade portion 010A having a neck portion and the flat plate 03 having the truncated cone-shaped blade portion 010B. In this way, the flat plate 03 of the stirring device 01 vibrates vertically in a state where the deformed form is appropriately formed by elastic deformation. Moreover, the flat plate 03 vibrates vertically accompanied by dynamic behavior so as to repeatedly form different deformed forms reversibly. The driving device 08 includes a controller for adjusting the relative moving distance between the movable member 05 and the support member 06, and can appropriately move and reciprocate within a distance of the movable range 017.

According to the stirring device 01 provided with the aforementioned stirring body 02, when the driving devices 08 and 09 are driven by predetermined physical means so that the movable member 05 appropriately moves and reciprocates vertically, the flat plate 03 made of an elastically deformable material is elastically deformed. In this way, the flat plate 03 can vibrate vertically while behaving dynamically so as to repeatedly form different deformed forms reversibly in the state where the flat plate 03 is elastically deformed to appropriately form a deformed form or from the three-dimensional flat plate 03 having the truncated cone-shaped blade portion 010B to the three-dimensional flat plate 03 forming the blade portion 010A having a neck portion. In this way, the movable member 05 reciprocates in the vertical direction accompanied by the vertical vibration of the flat plate 03 to generate a plurality of flows in the liquid in the stirring tank 04 accompanied by a vortex flow around the blade portions 016 and the gaps therebetween. Moreover, the central portion of the flat plate 03 moves vertically to generate a vertical convection flow. Further, the neck portion formed by the vertical movement of the central portion of the flat plate 03 generates a vortex flow based on a separation flow. The central portion of the flat plate 03 is raised to generate a suction flow rising due to a suction force generated from the bottom side of the stirring tank 04 toward the central portion of the flat plate 03. Furthermore, the suction flow hits the neck portion and the blade portions 016 that converge each other to generate a vortex flow and a vertical convection flow. Furthermore, the central portion of the flat plate 03 is lowered and the blade portion 016 and the neck portion spread toward the bottom side in the stirring tank 04 to generate an extrusion flow and a vertical convection flow that descend. In this way, it is possible to generate a plurality of flows such as a vortex flow, a separation flow, a suction flow, an extrusion flow, a vertical convection flow, a circulating flow, and a turbulent flow and stir a fluid medium in an extremely complex and unsteady manner with low shear. Therefore, for example, when an elastically deformable material is used for the material of the flat plate 03 of the stirring body 01 of the present embodiment described above and the driving devices 08 and 09 are driven by a predetermined physical means to cause the movable member 05 and the support member 06 so as to appropriately reciprocate and vibrate vertically, the movable member 05 reciprocates in relation to the support member 06 in a direction in which the outer peripheral portion and the central portion of the flat plate 03 approach and separate from each other. The blade portions 016 are elastically deformed while being twisted accompanied by an expansion/contraction movement in the vertical direction, a rotational movement in forward and reverse directions, a spreading movement, and a spreading/converging movement and the flat plate 03 behaves dynamically so as to repeatedly form different deformed forms reversibly. In this way, it is possible to stir a fluid medium in the liquid in an extremely complex and unsteady manner with low shear accompanied by a plurality of flows such as a vortex flow, a separation flow, a suction flow, an extrusion flow, a swirling flow, a vertical convection flow, a circulating flow, and a turbulent flow. Moreover, the controller provided in the driving device 08 controls the reciprocating distance of the movable member 05 and the flat plate 03 is elastically deformed to appropriately form deformed forms with different displacement amounts reversibly. In this way, it is possible to provide a stirring blade that can automatically adjust the flow of a stirring flow and that can be easily manufactured. However, the configuration of the stirring device 01 that is driven in the aforementioned plurality of operating directions described above is not limited to the configuration in which the driving device 08 is disposed on the central portion side of the flat plate 03 and the low-frequency vibration device 09 is disposed on the outer peripheral portion side. For example, the low-frequency vibration device 09 may be disposed on the central portion side of the flat plate 03, and the driving device 08 may be disposed on the outer peripheral portion side. That is, the configuration of the stirring device 01 that is driven in the aforementioned plurality of operating directions is not particularly limited.

The cut in the flat plate of the stirring body according to the present invention described above is not limited to an Archimedean spiral but may be configured by connecting circular arcs or straight lines or combinations thereof in series. The shape of the cut in the flat plate of the stirring body according to the present invention is not particularly limited.

Further, the cut angle (that is, the angle of the cut surface) of the flat plate of the stirring body according to the present invention described above is not limited to the form of cutting the flat plate at a right angle, but may be a form of cutting diagonally from the front surface side to the back surface side so as to approach the central portion, a form of cutting diagonally from the front surface side to the back surface side so as to approach the outer peripheral edge, and a curved surface form by curved surface processing or chamfering of corners.

Further, the direction in which the cut of the flat plate of the stirring body according to the present invention described above rotates and extends is not limited to a right-handed winding direction, but may be a left-handed winding direction, a direction which is a combination of the right-handed winding direction and the left-handed winding direction. That is, the direction in which the cut of the flat plate of the stirring body according to the present invention rotates and extends is not particularly limited.

The circumferential angle of the cut formed around the central portion of the flat plate of the stirring body according to the present invention described above can be freely adjusted. Preferably, the cut is formed so as to rotate and extend in the range of about 180 degrees to about 1440 degrees. Particularly preferably, the cut is formed so as to rotate and extend in the range of about 360 degrees to about 720 degrees.

Further, the number of cuts in the flat plate of the stirring body according to the present invention described above can be freely set according to the purpose. Preferably, the number is set in the range of 2 to 100, and particularly preferably in the range of 2 to 10.

The numbers of cut portions and singular points formed by the flat plate of the stirring body according to the present invention described above are not limited to one. For example, when the cuts and the cutouts of the flat plate of the stirring body according to the present invention are appropriately adjusted to form a deformed form, two or more cut portions and singular points may be formed. That is, the numbers of cut portions and singular points formed by the flat plate of the stirring body according to the present invention are not particularly limited.

Further, the displacement amount of the deformed form formed by the flat plate of the stirring body according to the present invention described above can be freely adjusted. The displacement amount is preferably adjusted in the range of 10% to 400%, particularly preferably in the range of 25% to 150%, with respect to the diameter of the circle passing through the outer peripheral ends of the spiral cut.

Further, the outer shape of the flat plate of the stirring body according to the present invention described above is not limited to a circular shape. For example, the outer shape may have a shape in which polygonal shapes, elliptical shapes, circular arcs, or straight lines, or combinations thereof are connected in series or a shape in which the flat plate is bent to form flaps, or a combination of the above-mentioned shaped. That is, the outer shape may be freely formed according to the purpose.

The material of the flat plate of the stirring body according to the present invention described above is not limited to an elastically deformable material. The material may have flexibility, springiness, conductivity, ferromagnetism, biocompatibility, biodegradability, chemical resistance, heat resistance, oil resistance, seawater resistance, rust resistance, corrosion resistance, shape memory, and any combination of these properties. That is, the properties of the material of the flat plate of the stirring body according to the present invention are not particularly limited.

The deformed form of the flat plate of the stirring body according to the present invention described above is not limited to a three-dimensional flat plate but may be any deformed form as long as it can be reversibly formed by elastic deformation.

The configuration of the flat plate and the movable member of the stirring body according to the present invention described above is not limited to the configuration in which the flat plate is rotatably supported with respect to the movable member. For example, the flat plate may be bonded and supported with respect to the movable member. That is, the configuration of the flat plate of the stirring body according to the present invention and the movable member described above is not particularly limited.

Further, the flat plate used for the stirring body and the stirring device including the stirring body according to the present invention described above can be freely replaced with other types of flat plates according to the purpose as long as they are the flat plates of the stirring bodies according to the present invention. That is, the flat plate used in the stirring body and the stirring device including the stirring body according to the present invention is not particularly limited.

The stirring body and the stirring device including the stirring body according to the present invention described above are not limited to a configuration in which a target object is stirred by a combination with a driving device. For example, a grasping portion may be provided in an operating member (for example, the movable member or the support member) of the stirring body as a manual whipping device to manually rotate the stirring body so as to rotate and reciprocate so that the target object is stirred. That is, the configuration of the stirring body and the stirring device including the stirring body according to the present invention is not particularly limited as long as it can stir a target object.

A method of connecting an operating member (for example, a movable member) to the driving device of the stirring body and the stirring device including the stirring body according to the present invention described above is not limited to bonding the operating member (the movement) to the driving shaft of the driving device. For example, the operating member may be the driving shaft of the driving device. That is, the method of connecting an operating member to the driving device of the stirring body and the stirring device including the stirring body according to the present invention is not particularly limited.

The driving device of the stirring body and the stirring device including the stirring body according to the present invention described above is not limited to a brushless motor, but, for example, may be a linear type, a sterling type, an air type, a magnetic type, or the like. That is, the driving device of the stirring body and the stirring device including the stirring body according to the present invention is not particularly limited.

Further, the driving operation of the stirring body and the stirring device including the stirring body according to the invention described above is not limited to a reciprocating operation in the vertical direction, but may be a reciprocating rotational movement in the forward and reverse directions, a rotational movement in one direction, and an operation of a combination thereof. That is, the driving operation of the stirring body and the stirring device including the stirring body according to the invention is not particularly limited.

Further, the configuration of the stirring body and the stirring device including the stirring body according to the present invention described above is not limited to a configuration in which the flat plate and the operating member (for example, the movable member) are connected to the driving device or the driving shaft of the driving device directly or via a connection member or the like. For example, the flat plate and the operating member (for example, the movable member) may be separated by a magnetic stirring device so as not to contact the driving device or the driving shaft of the driving device. That is, the configuration of the stirring body and the stirring device including the stirring body according to the present invention described above can be freely set.

The container shape of the stirring tank of the stirring body and the stirring device including the stirring body according to the present invention described above may be, for example, an open or closed bioreactor, a closed flask with screw caps, an open culture dish, a vertically or horizontally long pipe shape, or the like. That is, the container shape of the stirring tank of the stirring body and the stirring device including the stirring body according to the present invention described above is not particularly limited.

The volume of the stirring tank of the stirring body and the stirring device including the stirring body according to the present invention described above is not limited to 300 mL, but for example, may be 150 mL, 500 mL, 1 L, 50 L, 100 L, 500 L, 1000 L, 2000 L, 1 t, 10 t, 100 t, 200 t, or the like. That is, the volume of the stirring tank of the stirring body and the stirring device including the stirring body according to the present invention described above is not particularly limited.

The features of the stirring body and the stirring device including the stirring body according to the present invention described above can be appropriately combined.

The stirring body and the stirring device including the stirring body according to the present invention described above can be appropriately changed to various forms according to the purpose, and such changes are also included in the scope of the present invention, and the stirring body and the stirring device including the stirring body according to the present invention are not limited thereto.

Further, the stirring body and the stirring device including the stirring body according to the present invention can appropriately provide a physicochemical environment suitable for proliferation and metabolism of cultured cells, differentiation and maturation of megakaryocytes, polynuclearization of megakaryocytes, production of platelets, and maintenance of bioactivity of platelets. For example, the stirring body and the stirring device including the stirring body according to the present invention may be provided with a ventilation device, an exhaust device, a temperature controller, a pH controller, a dissolved oxygen pressure controller, a baffle, a sparger, a port, and the like and may be used as a stirring culture device for stirring culture solutions to culture cells. The physicochemical environment of the stirring body and the stirring device including the stirring body according to the present invention described above is not particularly limited.

Furthermore, the object to be cultured using the stirring body and the stirring culture device including the stirring body according to the present invention described above is not limited to megakaryocyte cells derived from human iPS cells. For example, the object to be cultured may be other cells derived from cells such as hematopoietic stem cells, stem cells, human iPS cells, human ES cells, and unicellular and multicellular organisms such as algae, seaweeds, euglena (commonly known as Euglena), and the like. That is, the object to be cultured using the stirring body, the stirring device and the stirring culture device including the stirring body according to the present invention is not particularly limited.

### Industrial Applicability

The stirring body and the stirring device including the stirring body according to the present invention are mainly used for stirring culture solutions in the pharmaceutical field and stirring, grinding and mixing of gases, liquids, powders, solids, gels and mixtures of combinations of any of them in the fields of food, beverages, feed, chemicals, cosmetics, paints, papermaking, petrochemicals, civil engineering, water treatment, energy, cell culture, regenerative medicine, and the like.

### Reference Signs List

- 1A: Stirring body
- 1B: Stirring body
- 1C: Stirring body
- 2: Cut
- 3A: Flat plate
- 3B: Flat plate
- 3C: Flat plate
- 3D: Flat plate
- 3E: Flat plate
- 3F: Flat plate
- 3G: Flat plate
- 3H: Flat plate
- 31: Flat plate
- 3J: Flat plate
- 3K: Flat plate
- 3L: Flat plate
- 4A: Central portion
- 4B: Central portion
- 4C: Central portion
- 4D: Central portion
- 4E: Central portion
- 4F: Central portion
- 4G: Central portion
- 4H: Central portion
- 41: Central portion
- 5: Outer-peripheral-side end
- 6A: Inner-peripheral-side end
- 6B: Inner-peripheral-side end
- 6C: Inner-peripheral-side end
- 7A: Blade portion
- 7B: Blade portion
- 7C: Blade portion
- 7D: Blade portion
- 7E: Blade portion
- 7F: Blade portion
- 7G: Blade portion
- 7H: Blade portion
- 71: Blade portion
- 7J: Blade portion
- 7K: Blade portion
- 7L: Blade portion
- 7M: Blade portion
- 8: Movable member
- 9: Support member
- 11: Extension position
- 12: Extension position
- 13A: Neck portion
- 13B: Neck portion
- 15: Stirring device
- 16: Stirring tank
- 17: Movable member
- 18: Support member
- 19: Expansion/contraction stirring body
- 20: Top plate portion
- 21: Seal
- 22: Driving device
- 24: Stirring device
- 25: Stirring tank
- 26: Flat plate
- 27: Movable member
- 28: Support member
- 29: Contraction stirring body
- 30: Top plate portion
- 31: Bearing
- 32: Bearing
- 33: Gear
- 34: Gear
- 35: Protective portion
- 36: Branch portion
- 37: Driving device
- 38: Driving device
- 39A: Blade portion
- 39B: Blade portion
- 43: Movable range
- 44: Expansion/contraction stirring body
- 45: Support member
- 46: Connecting flat plate
- 47: Movable member
- 48: Expansion/contraction stirring body
- 49: Support member
- 50: Movement auxiliary member
- 51: Movable member
- 52: Expansion/contraction stirring body
- 53: Support member
- 54: Movable member
- 55: Expansion/contraction stirring body
- 56: Support member
- 57: Connecting flat plate
- 58: Movable member
- 59: Expansion/contraction stirring body
- 60: Support member
- 61: Connecting flat plate
- 62: Movement auxiliary member
- 63: Movable member
- 64: Flat plate
- 65: Flat plate
- 66: Central portion
- 67: Cut
- 68: Outer-peripheral-side end
- 69: Inner-peripheral-side end
- 70: Central portion
- 71A: Flat plate auxiliary member
- 71B: Flat plate auxiliary member
- 72: Flat plate
- 73: Central portion
- 74: Flat plate
- 75: Flat plate
- 76: Central portion
- 77: Cut
- 78: Outer-peripheral-side end
- 79: Inner-peripheral-side end
- 80: Flat plate auxiliary member
- 81: Inner shape
- 82: Outer shape
- 83: Flat plate
- 84: Central portion
- 85: Flat plate
- 86: Flat plate
- 87: Central portion
- 88: Cut
- 89: Outer-peripheral-side end
- 90: Inner-peripheral-side end
- 91: Flat plate auxiliary member
- 92: Inner shape
- 93: Outer shape
- 94: Flat plate
- 95: Central portion
- 96: Flat plate
- 97: Cutout
- 98: Central portion
- 99: Outer-peripheral-side end
- 100: Inner-peripheral-side end
- 101: Flat plate
- 102: Cut
- 103: Central portion
- 104: Outer-peripheral-side end
- 105: Inner-peripheral-side end
- 106: Flat plate
- 107: Cut
- 108: Central portion
- 109: Outer-peripheral-side end
- 110: Inner-peripheral-side end
- 111: Intermediate portion
- 112: Flap
- 113: Flat plate
- 114: Cut
- 115: Central portion
- 116: Outer-peripheral-side end
- 117: Inner-peripheral-side end
- 118: Through-hole

## Claims

1. A stirring body comprising
a flat plate in which a plurality of spiral blade portions extending from a central portion is formed by a spiral cut that does not contact an outer peripheral portion; and
an operating member that supports said flat plate,
either one of said operating member or an outer peripheral portion of said flat plate being movable in a direction of separating and approaching in relation to the other, and
said blade portion rising while being separated from an adjacent blade portion in conjunction with the movement in a direction of separating in relation to each other, thereby said flat plate being extended to form a three-dimensional shape.

2. The stirring body according to claim 1, wherein
said operating member supports said flat plate in a state of being fixed.

3. The stirring body according to claim 1, wherein
said operating member supports said flat plate in a state of being rotatable.

4. The stirring body according to claim 1, wherein
said operating member or said flat plate is rotatable in forward and reverse directions during said movement.

5. The stirring body according to any one of claims 1 to 4, wherein
one or more neck portions are formed in said three-dimensional shape in which said flat plate is extended.

6. The stirring body according to any one of claims 1 to 5, wherein
said blade portion is formed in a plurality of wavy shapes of which said front and back surfaces are exposed on condition that seen from one direction in said three-dimensional shape in which said flat plate is extended.

7. The stirring body according to claim 1, wherein
said cut is formed by connecting circular arcs, straight lines, or combinations thereof in series.

8. A stirring device comprising
said stirring body according to any one of claims 1 to 7; and
a driving device that moves said operating member or said flat version.

9. The stirring device according to claim 8, wherein
said driving device rotates said operating member or said flat plate in addition to said movement.
